# EUROPEAN PATENT SPECIFICATION

(11) **EP 4 577 243 B1**
(45) Date of publication and mention of the grant of the patent: **30.09.2026**
(21) Application number: 23764254.1
(22) Date of filing: 25.08.2023
(51) Int. Cl.: A61K 47/10, A61K 47/26, C12N 15/88, A61K 47/22, A61K 47/44, A61K 9/51, C12N 15/00

(54) **STABLE LIPID OR LIPIDOID NANOPARTICLE SUSPENSIONS**
STABILISIERUNG VON LIPID- ODER LIPIDOIDNANOPARTIKELSUSPENSIONEN
STABILISATION DE SUSPENSIONS DE NANOPARTICULES LIPIDIQUES OU LIPIDOÏDES

(30) Priority: 26.08.2022 EP 22192299; 16.05.2023 EP 23173773
(43) Date of publication of application: 02.07.2025
(73) Proprietor: Ethris GmbH, 82152 Planegg (DE)
(72) Inventor: DOHMEN, Christian, 82152 Planegg (DE); RUDOLPH, Carsten, 82152 Planegg (DE); SOHR, Katharina, 82152 Planegg (DE); WEICHSELBAUM, Andrea, 82152 Planegg (DE); DAUBERT, Daniela, 82152 Planegg (DE)
(74) Representative: Vossius & Partner Patentanwälte Rechtsanwälte mbB
(86) International application number: PCT/EP2023/073435
(87) International publication number: WO 2024/042236

(56) References cited:
- WO-A1-2017/218704
- WO-A1-2018/089540
- WO-A1-2022/036170
- WO-A1-2022/180213
- MOEIN MOGHIMI S.: "Allergic Reactions and Anaphylaxis to LNP-Based COVID-19 Vaccines", MOLECULAR THERAPY, vol. 29, no. 3, 1 March 2021 (2021-03-01), US, pages 898 - 900, XP093081422, ISSN: 1525-0016, Retrieved from the Internet <URL:https://www.ncbi.nlm.nih.gov/pmc/articles/PMC7862013/pdf/main.pdf> DOI: 10.1016/j.ymthe.2021.01.030

## Description

The present invention relates to the stabilization of suspension formulations comprising lipid nanoparticles or lipidoid nanoparticles for the delivery of nucleic acids.

Lipid or lipidoid nanoparticles (LNPs or LiNPs) are frequently used for the delivery of active pharmaceutical ingredients in patients. For example, lipid or lipidoid formulations of nucleic acids are extremely useful and efficient for introducing nucleic acids into cells. This advantageous property of lipid or lipidoid formulations of nucleic acids has been used for decades in biological and medical research and in therapeutic approaches to i) overexpress genes or to complement genetic defects in target cells, or ii) to downregulate or upregulate endogenous gene expression in cells, or iii) to repair genetic defects (mutations). mRNA formulations relying on nanoparticles are now also established as vaccines against COVID-19.

However, it has been found that conditions of physical stress to which suspension formulations comprising lipid nanoparticles or lipidoid nanoparticles may be subjected during handling or transport may impact the formulations' efficiency for the delivery of the nucleic acid to a patient. In particular, exposure of the formulation to vibrational stress or shaking the formulation may have a destabilizing effect or reduce its efficacy (Kudsiova L, Lansley A, Scutt G, et al. Stability testing of the Pfizer- BioNTech BNT162b2 COVID-19 vaccine: a translational study in UK vaccination centres. BMJ Open Science 2021;5:e100203. doi:10.1136/bmjos-2021-100203; S. Grau et al., Clinical Microbiology and Infection 27 (2021) 1698.e1e1698.e4).

Thus, reliable strategies for the stabilization of suspension formulations comprising lipid nanoparticles or lipidoid nanoparticles for the delivery of nucleic acids would be desirable.

### SUMMARY OF THE INVENTION

In the context of the present invention, it was found that the presence of a surfactant stabilizes a suspension of lipid nanoparticles or of lipidoid nanoparticles in an aqueous vehicle solution against particle aggregation under physical stress conditions.

To that extent, the following aspects provided by the invention are indicated in a non-exclusive manner.

In one aspect, the invention provides a suspension of lipid nanoparticles or of lipidoid nanoparticles in an aqueous vehicle solution, wherein the aqueous vehicle solution comprises a surfactant, and wherein the lipid nanoparticles or lipidoid nanoparticles comprise the following components (a) and (b):
(a) a therapeutic agent, which is preferably a nucleic acid, and
(b) at least one selected from a permanently cationic lipid, an ionizable lipid and an ionizable lipidoid. Methods for the preparation of the suspension are also provided.

In accordance with another aspect, the invention provides the use of a surfactant for stabilizing a suspension of lipid nanoparticles or of lipidoid nanoparticles in an aqueous vehicle solution against particle aggregation under a physical stress condition, wherein the lipid nanoparticles or lipidoid nanoparticles comprise the following components (a) and (b):
(a) a therapeutic agent, which is preferably a nucleic acid, and
(b) at least one selected from a permanently cationic lipid, an ionizable lipid and an ionizable lipidoid.

In a related aspect, the invention provides a method for stabilizing a suspension of lipid nanoparticles or of lipidoid nanoparticles in an aqueous vehicle solution against particle aggregation under a physical stress condition, wherein the lipid nanoparticles or lipidoid nanoparticles comprise the following components (a) and (b):
(a) a therapeutic agent, which is preferably a nucleic acid, and
(b) at least one selected from a permanently cationic lipid, an ionizable lipid and an ionizable lipidoid,
and wherein the method comprises incorporating a surfactant into the suspension of lipid nanoparticles or of lipidoid nanoparticles.

Furthermore, the invention provides the suspensions in accordance with the invention, including the suspensions obtained by the methods in accordance with the invention, for use as a medicament. In a related context, the formulations in accordance with the invention, including the suspensions obtained by the methods in accordance with the invention, are suitable for reducing side effects in therapy with lipid or lipidoid nanoparticles.

Without wanting to be bound by theory, the present invention provides stabilized LNP/LiNP formulations and LNP/LiNP suspensions, uses thereof and uses in method of treatment based on the findings that the addition of the surfactant to the formulations or suspensions avoid aggregation, allowing e.g. a surprisingly long shelf life and extended stability to shaking. Said reduction of aggregation according to the invention results in a reduction of side effects of the formulations and suspension of the invention, such as a reduction of side effects caused by vaccines formulations or anticancer formulations comprising LNPs or LiNPs.

A summary of various aspects of the invention is provided in the following first set of items.
1. A suspension of lipid nanoparticles or of lipidoid nanoparticles in an aqueous vehicle solution, wherein the aqueous vehicle solution comprises a surfactant, and wherein the lipid nanoparticles or lipidoid nanoparticles comprise the following components (a) and (b):
   (a) a therapeutic agent, and
   (b) at least one selected from a permanently cationic lipid, an ionizable lipid and an ionizable lipidoid.
2. The suspension in accordance with item 1, wherein the therapeutic agent is a nucleic acid.
3. The suspension in accordance with item 2, wherein the nucleic acid is selected from RNA and plasmid DNA.
4. The suspension in accordance with item 2 or 3, wherein the nucleic acid is selected from mRNA, siRNA, miRNA, antisense RNA, tRNA, and noncoding RNA.
5. The suspension in accordance with item 4, wherein the nucleic acid is mRNA.
6. The suspension in accordance with any of items 2 to 5, wherein the concentration of the nucleic acid in the suspension ranges from 0.01 to 10 mg/mL, more preferably from 0.02 to 10 mg/mL, still more preferably from 0.05 to 5 mg/mL, and most preferably from 0.05 to 2.5 mg/ml based on the total volume of the suspension.
7. The suspension in accordance with any of items 1 to 6, wherein the weight-to-volume ratio of the nanoparticles in the aqueous vehicle solution in gram per liter is in the range of 0.1 g/L to 300 g/L, more preferably 0.2 g/L to 300 g/L, still more preferably 0.5 g/L to 250 g/L and most preferably 0.5 g/L to 125 g/L.
8. The suspension in accordance with any of items 1 to 7, wherein the suspended nanoparticles have a Z-average diameter, as determined by dynamic light scattering, in the range of 10 to 500 nm, more preferably in the range of 10 to 250 nm, still more preferably 20 to 200 nm.
9. The suspension in accordance with any of items 1 to 8, wherein the suspended nanoparticles have a polydispersity index, as determined by dynamic light scattering, in the range of 0.02 to 0.4, more preferably in the range of 0.03 to 0.2.
10. The suspension in accordance with any of items 1 to 9, wherein the nanoparticles further comprise one or more of the following components (c1) to (c6):
   (c1) a non-ionizable lipid having a sterol structure;
   (c2) a phosphoglyceride lipid;
   (c3) a PEG-conjugated lipid;
   (c4) a polysarcosine-conjugated lipid
   (c5) a PASylated lipid; and
   (c6) a cationic polymer.
11. The suspension in accordance with any of items 1 to 10, wherein the nanoparticles comprise:
   30 to 65 mol% of at least one of the permanently cationic lipid, ionizable lipid and ionizable lipidoid (b), preferably the ionizable lipid or ionizable lipidoid (b), and one or more of the following components:
   10 to 50 mol% of the lipid having a sterol structure (c1),
   4 to 50 mol% of the phosphoglyceride lipid (c2),
   0.5 to 10 mol% of one of the PEG-conjugated lipid (c3), the polysarcosine-conjugated lipid (c4) and the PASylated lipid (c5), or of any combination thereof,
   0.5 to 10 mol% of the cationic polymer (c6),
   such that the sum of (b) and (c1) to (c6) amounts to 100 mol%.
12. The suspension in accordance with any of items 1 to 11, wherein the nanoparticles further comprise the following components (c1) to (c3):
   (c1) a non-ionizable lipid having a sterol structure;
   (c2) a phosphoglyceride lipid; and
   (c3) a PEG-conjugated lipid.
13. The suspension in accordance with item 12, wherein the nanoparticles comprise:
   30 to 65 mol% of at least one of the permanently cationic lipid, ionizable lipid and ionizable lipidoid (b), preferably the ionizable lipid or ionizable lipidoid (b),
   10 to 50 mol% of the lipid having a sterol structure (c1),
   4 to 50 mol% of the phosphoglyceride lipid (c2), and
   0.5 to 10 mol% of the PEG-conjugated lipid (c3),
   such that the sum of (b) and (c1) to (c3) amounts to 100 mol%.
14. The suspension in accordance with any of items 2 to 13, wherein the nanoparticles further comprise a polyanionic component which is different from the nucleic acid.
15. The suspension in accordance with any of items 2 to 14, wherein the composition of the nanoparticles is such that the weight ratio in the nanoparticles of the sum of the weights of components other than the nucleic acid to the weight of the nucleic acid is in the range of 50:1 to 1:1, more preferably 40:1 to 2:1 and most preferably 30:1 to 3:1.
16. The suspension in accordance with any of items 1 to 15, wherein the nanoparticles comprise an ionizable lipidoid (b) of the following formula (b-1), 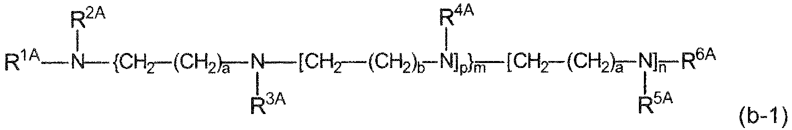 wherein:
   a is 1 and b is an integer of 2 to 4; or a is an integer of 2 to 4 and b is 1,
   p is 1 or 2,
   m is 1 or 2; n is 0 or 1 and m+n is ≥ 2; and
   R^{1A} to R^{6A} are independently of each other selected from: hydrogen; -CH₂-CH(OH)-R^{7A}, -CH(R^{7A})-CH₂-OH, -CH₂-CH₂-(C=O)-O-R^{7A},
   -CH₂-CH₂-(C=O)-NH-R^{7A}; -CH₂-R^{7A}; -C(NH)-NH₂; a poly(ethylene glycol) chain; and a receptor ligand; wherein R^{7A} is selected from C3-C18 alkyl and C3-C18 alkenyl having one C-C double bond;
   provided that at least two residues among R^{1A} to R^{6A} are selected from -CH₂-CH(OH)-R^{7A}, -CH(R^{7A})-CH₂-OH, -CH₂-CH₂-(C=O)-O-R^{7A},
   -CH₂-CH₂-(C=O)-NH-R^{7A} and -CH₂-R^{7A}, wherein R^{7A} is selected from C3-C18 alkyl or C3-C18 alkenyl having one C-C double bond;
   or a protonated form thereof, wherein one or more of the nitrogen atoms contained in the compound of formula (b-1) are protonated to provide a compound carrying a positive charge.
17. The suspension in accordance with any of items 1 to 16, wherein the nanoparticles comprise an ionizable lipidoid (b-1) of the following formula (b-1b), 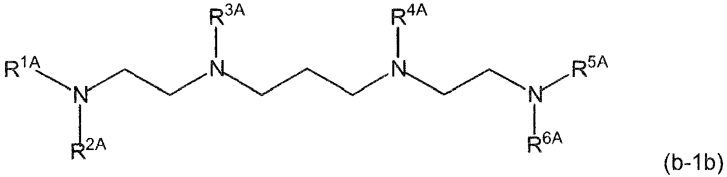 wherein R^{1A} to R^{6A} are defined as in item 16,
   or a protonated form thereof, wherein one or more of the nitrogen atoms contained in the compound of formula (b-1b) are protonated to provide a compound carrying a positive charge.
18. The suspension in accordance with item 16 or 17, wherein R^{1A} to R^{6A} are independently selected from hydrogen and -CH₂-CH(OH)-R^{7A}, wherein R^{7A} is selected from C8-C18 alkyl and C8-C18 alkenyl having one C-C double bond, provided that at least two residues, preferably at least three residues, and more preferably at least four residues, among R^{1A} to R^{6A} are -CH₂-CH(OH)-R^{7A} wherein R^{7A} is selected from C8-C18 alkyl and C8-C18 alkenyl having one C-C double bond.
19. The suspension in accordance with any of items 1 to 18, wherein the nanoparticles comprise an ionizable lipidoid dL_05(R) having the formula: 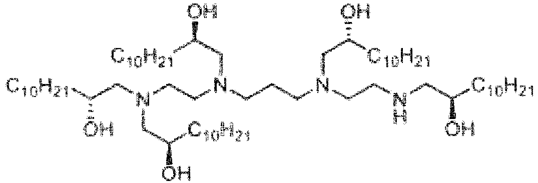
20. The suspension in accordance with any of items 1 to 15, wherein the nanoparticles comprise an ionizable lipid (b) of the following formula (a-III): 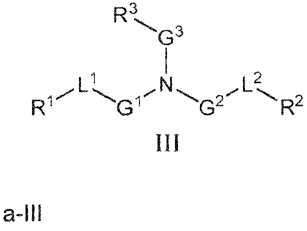 or a pharmaceutically acceptable salt, prodrug or stereoisomer thereof, wherein:
   one of L¹ or L² is -O(C=O)-, -(C=O)O-, -C(=O)-, -O-, -S(O)ₓ-, -S-S-, - C(=O)S-, SC(=O)-, - NR^{a}C(=O)-, -C(=O)NR^{a}-, NR^{a}C(=O)NR^{a}-, -OC(=O)NR^{a}- or - NR^{a}C(=O)O-, and the other of L¹ or L² is-O(C=O)-, -(C=O)O-, -C(=O)-, -O-, -S(O)ₓ-, -S- S-, -C(=O)S-, SC(=O)-, -NR^{a}C(=O)-, -C(=O)NR^{a}-, ,NR^{a}C(=O)NR^{a}-, -OC(=O)NR^{a}- or -NR^{a}C(=O)O- or a direct bond;
   G¹ and G² are each independently C₁-C₁₂ alkylene or C₁-C₁₂ alkenylene;
   G³ is C₁-C₂₄ alkylene, C₁-C₂₄ alkenylene, C₃-C₈ cycloalkylene, C₃-C₈ cycloalkenylene, wherein each of alkylene, alkenylene, cycloalkylene, and cyloalkenylene is optionally substituted;
   R^{a} is H or C₁-C₁₂ alkyl wherein alkyl is optionally substituted;
   R¹ and R² are each independently C₆-C₂₄ alkyl or C₆-C₂₄ alkenyl, wherein each of alkyl and alkenyl is optionally substituted ;
   R³ is H, OR⁵, CN, -C(=O)OR⁴, -OC(=O)R⁴ or-NR⁵C(=O)R⁴; R⁴ is C₁-C₁₂ alkyl, wherein alkyl is optionally substituted;
   R⁵ is H or C₁-C₆ alkyl, wherein alkyl is optionally substituted; and
   x is 0, 1 or 2.
21. The suspension in accordance with any of items 1 to 15, wherein the nanoparticles comprise, as an ionizable lipid (b), (6Z,9Z,28Z,31Z)-heptatriaconta-6,9,28,31-tetraene-19-yl 4-(dimethylamino)butanoate or a protonated form thereof wherein the nitrogen atom of the compound is protonated to provide a compound carrying a positive charge.
22. The suspension in accordance with any of items 1 to 15, wherein the nanoparticles comprise, as an ionizable lipid (b), ((4-hydroxybutyl)azanediyl)bis(hexan-6,1-diyl)bis(2-hexyldecanoate) or a protonated form thereof wherein the nitrogen atom of the compound is protonated to provide a compound carrying a positive charge.
23. The suspension in accordance with any of items 1 to 15 or 22, wherein the nanoparticles comprise, as an ionizable lipid (b), heptadecan-9-yl 8-((2-hydroxyethyl)(6-oxo-6-(undecyloxy)hexyl)amino)octanoate (SM-102) or a protonated form thereof wherein the nitrogen atom of the compound is protonated to provide a compound carrying a positive charge.
24. The suspension in accordance with any of items 10 to 23, wherein the non-ionizable lipid having a sterol structure (c1) comprises a non-ionizable lipid of formula (c1-1): 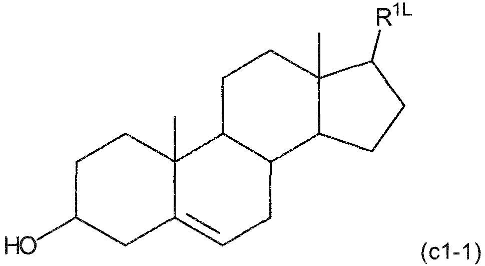 wherein R^{1L} is a C3-C12 alkyl group.
25. The suspension in accordance with any of items 10 to 24, wherein the non-ionizable lipid having a sterol structure (c1) comprises cholesterol.
26. The suspension in accordance with any of items 10 to 25, wherein the phosphoglyceride lipid (c2) comprises a phosphoglyceride lipid of formula (c2-1) 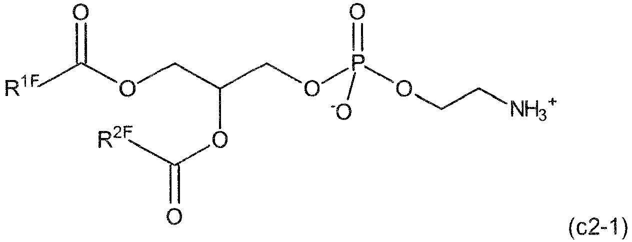 wherein
   R^{1F} and R^{2F} are independently a C8-C18 alkyl group or a C8-C18 alkenyl group, preferably a C12-C18 alkyl group or a C12-C18 alkenyl group,
   or a pharmaceutically acceptable salt thereof;
   or a phosphoglyceride lipid of formula (c2-2) 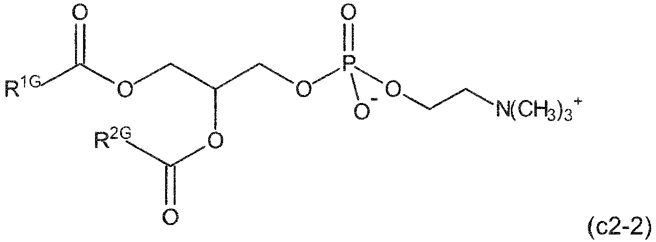 wherein
      R^{1G} and R^{2G} are independently a C8-C18 alkyl group or a C8-C18 alkenyl group, preferably a C12-C18 alkyl group or a C12-C18 alkenyl group,
   or a pharmaceutically acceptable salt thereof.
27. The suspension in accordance with any of items 10 to 26, wherein the phosphoglyceride lipid (c2) comprises 1,2-dipalmitoyl-sn-glycero-3-phosphocholine (DPPC) or a pharmaceutically acceptable salt thereof or 1,2-distearoyl-sn-glycero-3-phosphocholine (DSPC) or a pharmaceutically acceptable salt thereof.
28. The suspension in accordance with any of items 10 to 27, wherein the PEG-conjugated lipid (c3) comprises a PEG-conjugated lipid of formula (c3-1) 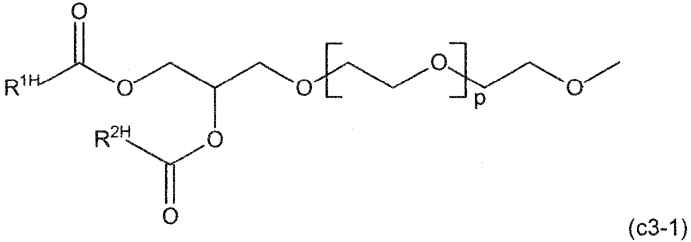 wherein
   R^{1H} and R^{2H} are independently a C8-C18 alkyl group or a C8-C18 alkenyl group, preferably a C12-C18 alkyl group or a C12-C18 alkenyl group, and p is an integer of 5 to 200, preferably 10 to 100, and more preferably 20 to 60;
   or a PEG-conjugated lipid of formula (c3-2) 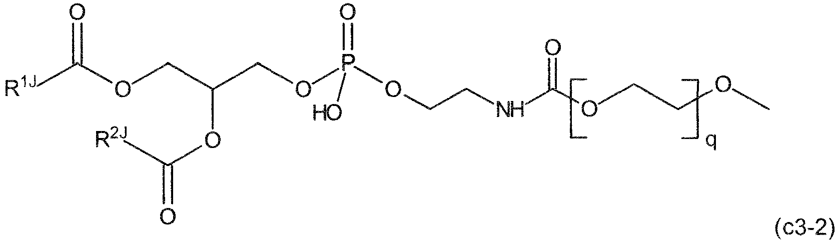 wherein
      R^{1J} and R^{2J} are independently a C8-C18 alkyl group or a C8-C18 alkenyl group, preferably a C12-C18 alkyl group or a C12-C18 alkenyl group, and q is an integer of 5 to 200, preferably 10 to 100, and more preferably 20 to 60,
      or a pharmaceutically acceptable salt thereof,
   or a PEG-conjugated lipid of formula (c3-3) 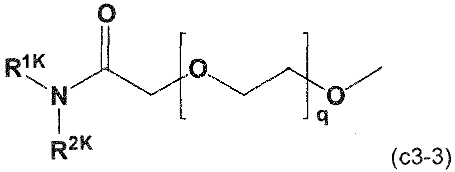 wherein
      R^{1K} and R^{2K} are independently a C8-C18 alkyl group or a C8-C18 alkenyl group, preferably a C12-C18 alkyl group or a C12-C18 alkenyl group, and q is an integer of 5 to 200, preferably 10 to 100, and more preferably 20 to 60.
29. The suspension in accordance with item 10 to 28, wherein the PEG conjugated lipid (c3) comprises 1,2-dimyristoyl-sn-glycerolmethoxy(polyethylene glycol)-2000 (DMG-PEG2k) or 2-[(polyethylenglycol)-2000]-N,N-ditetradecylacetamid (ALC-0159).
30. The suspension in accordance with item 22, wherein the nanoparticles comprise, as an ionizable lipid (b), the ((4-hydroxybutyl)azanediyl)bis(hexan-6,1-diyl)bis(2-hexyldecanoate) or a protonated form thereof wherein the nitrogen atom of the compound is protonated to provide a compound carrying a positive charge, and further comprise one or more of the following components (d1) to (d8):
   (d1) 2-[(polyethylene glycol)-2000]-N,N-ditetradecylacetamide (ALC-0159)
   (d2) 1,2-distearoyl-sn-glycero-3-phosphocholine (DSPC)
   (d3) cholesterol
   (d4) potassium chloride
   (d5) potassium dihydrogen phosphate
   (d6) sodium chloride
   (d7) disodium phosphate dihydrate
   (d8) sucrose.
31. The suspension in accordance with item 23, wherein the nanoparticles comprise, as an ionizable lipid (b), heptadecan-9-yl 8-((2-hydroxyethyl)(6-oxo-6-(undecyloxy)hexyl)amino)octanoate (SM-102) or a protonated form thereof wherein the nitrogen atom of the compound is protonated to provide a compound carrying a positive charge, and further comprise one or more of the following components (e1) to (e7):
   (e1) 1,2-distearoyl-sn-glycero-3-phosphocholine (DSPC),
   (e2) cholesterol,
   (e3) 1,2-dimyristoyl-rac-glycero-3-methoxypolyethylene glycol-2000 (PEG2000 DMG),
   (e4) tromethamol hydrochloride
   (e5) sodium acetate trihydrate
   (e6) acetic acid
   (e7) sucrose.
32. The suspension in accordance with any of items 1 to 31, wherein the N/P ratio in the nanoparticles is in the range of 0.5 to 20, more preferably in the range of 0.5 to 10.
33. The suspension in accordance with any of items 1 to 32, wherein the surfactant is essentially not attached to the nanoparticles.
34. The suspension in accordance with any of items 1 to 33, wherein the surfactant is a nonionic surfactant.
35. The suspension in accordance with item 34, wherein the nonionic surfactant is at least one selected from the group consisting of fatty alcohol ethoxylates, fatty acid ethoxylates, block copolymers of ethylene oxide and propylene oxide, alkylphenol ethoxylates or oligomers of alkylphenol ethoxylates, fatty acid esters of sorbitol, ethoxylated fatty acid esters of sorbitol, fatty acid esters of glycerol, ethoxylated castor oil and ethoxylated vitamin E
36. The suspension in accordance with item 35, wherein the block copolymer of ethylene oxide and propylene oxide is a poloxamer.
37. The suspension in accordance with item 36, wherein the poloxamer contains one poly(propylene oxide) block B of formula (p-1): 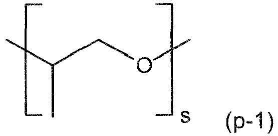
   wherein s is an integer of 15 to 60, and
   two poly(ethylene oxides) blocks A of formula (p-2): 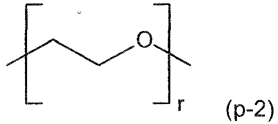
   wherein r is, independently for each block, an integer of 8 to 150, preferably 10 to 150.
38. The suspension in accordance with item 35, wherein the nonionic surfactant is at least one selected from the group consisting of fatty alcohol ethoxylates, fatty acid ethoxylates, alkylphenol ethoxylates or oligomers of alkylphenol ethoxylates, fatty acid esters of sorbitol, ethoxylated fatty acid esters of sorbitol, fatty acid esters of glycerol, ethoxylated castor oil and ethoxylated vitamin E.
39. The suspension in accordance with any of items 35 to 37, wherein the nonionic surfactant is at least one selected from the group of poloxamer 124, poloxamer 188, poloxamer 338, poloxamer 407, polysorbate 20, polysorbate 80, polyoxyethylenelaurylether, poyloxyethylene-35 castor oil, D-α-tocopherol polyethylene glycol 1000 succinate, and Tyloxapol.
40. The suspension in accordance with 39, wherein the nonionic surfactant is at least one selected from the group consisting of polysorbate 20, polysorbate 80, polyoxyethylenelaurylether, poyloxyethylene-35 castor oil, D-α-tocopherol polyethylene glycol 1000 succinate, and Tyloxapol.
41. The suspension in accordance with any of items 1 to 40, wherein the surfactant does not comprise Poloxamer 188.
42. The suspension in accordance with any of items 1 to 41, wherein the surfactant does not comprise Poloxamer 407.
43. The suspension in accordance with any of items 1 to 42, which comprises the surfactant at a concentration of 0.01 to 10 % (w/v), preferably 0.1 to 10 % (w/v), more preferably 0.25 to 5 % (w/v), still more preferably 0.33 to 2.5 % (w/v), even more preferably 0.45 to 10 % (w/v) and most preferably 0.5 to 1.5 % (w/v), with regard to the total volume of the suspension of the nanoparticles in the aqueous vehicle solution.
44. The suspension in accordance with item 43, which comprises the surfactant at a concentration 0.5 to 1.5 % (w/v), with regard to the total volume of the suspension of the nanoparticles in the aqueous vehicle solution.
45. The suspension in accordance with any of items 1 to 44, wherein the nanoparticles have not been lyophilized.
46. A method for the preparation of a suspension in accordance with any of items 1 to 45, which comprises:
   generating a preparation of lipid nanoparticles or lipidoid nanoparticles by mixing of at least one selected from a permanently cationic lipid, an ionizable lipid and an ionizable lipidoid dissolved in an organic phase with a therapeutic agent dissolved in an aqueous solution, and combining the nanoparticles with a surfactant to obtain a suspension of the nanoparticles in an aqueous vehicle solution.
47. The method in accordance with item 46, wherein the method comprises the following steps:
   i) generating a preparation of lipid nanoparticles or lipidoid nanoparticles by mixing of at least one selected from a permanently cationic lipid, an ionizable lipid and an ionizable lipidoid dissolved in an organic phase with a therapeutic agent dissolved in an aqueous solution,
   ii) diluting the preparation of lipid nanoparticles or lipidoid nanoparticles by dilution with a first solution,
   iii) concentrating the diluted preparation of lipid nanoparticles or lipidoid nanoparticles by buffer exchange using ultra/diafiltration by TFF, wherein a second solution is used for the ultra/diafiltration,
   iv) obtaining a suspension of lipid nanoparticles or lipidoid nanoparticles in an aqueous vehicle solution comprising a surfactant,

   wherein the first solution comprises between 0.01% w/v and 10% w/v surfactant, preferably 0.1% w/v and 10% w/v surfactant, more preferably between 0.25% w/v and 5% w/v surfactant, still more preferably between 0.33% w/v and 2.5% w/v surfactant, even more preferably between 0.45% w/v and 1.5% w/v surfactant, most preferably between 0.5% w/v and 1.5% w/v surfactant, and/or
   wherein the second solution comprises between 0.01% w/v and 10% w/v surfactant, preferably 0.1% w/v and 10% w/v surfactant, more preferably between 0.25% w/v and 5% w/v surfactant, still more preferably between 0.33% w/v and 2.5% w/v surfactant, even more preferably between 0.45% w/v and 1.5% w/v surfactant, most preferably between 0.5% w/v and 1.5% w/v surfactant,
   and wherein the final concentration of surfactant from combined first and second solution is between 0.01% w/v and 10% w/v surfactant, preferably between 0.1% w/v and 10% w/v surfactant, more preferably between 0.25% w/v and 5% w/v surfactant, still more preferably between 0.33% w/v and 2.5% w/v surfactant, even more preferably between 0.45% w/v and 1.5% w/v surfactant, most preferably between 0.5% w/v and 1.5% w/v surfactant with regard to the total volume of the suspension of the nanoparticles in the aqueous vehicle solution.
48. The method according to item 47, wherein an incorporation of a surfactant into the suspension does not occur before or during step i).
49. The method according to item 47 or 48, wherein the surfactant is added both with the first and the second solution.
50. The method according to any of items 47 to 49, wherein 30 to 70 wt% of the surfactant, preferably 40 to 60 wt%, and more preferably 45 to 55 wt% of the surfactant, based on the total weight of the surfactant in the suspension obtained in step iv), are added with the first solution, and 70 to 30 wt% of the surfactant, preferably 60 to 40 wt%, and more preferably 55 to 45 wt% of the surfactant, based on the total weight of the surfactant in the suspension obtained in step iv), are added with the second solution, such that the sum of the amount of surfactant added with the first and the second solution is 100 wt%.
51. The method according to any of items 47 to 50, wherein approximately half of the surfactant is added with the first solution and approximately half of the surfactant is added with the second solution.
52. A suspension of lipid nanoparticles or lipidoid nanoparticles (LNP or LiNP suspension) obtained by the method according to any of items 46 to 51.
53. A formulation of lipid nanoparticles or lipidoid nanoparticles comprising a suspension of lipid nanoparticles or lipidoid nanoparticles in accordance with any of items 1 to 45 or 52.
58. The suspension or formulation of lipid nanoparticles or lipidoid nanoparticles according to any of items 1 to 45, 52 or 53 for use in the treatment or prevention of a disease.
59. The suspension or formulation of lipid nanoparticles or lipidoid nanoparticles according to any of items 1 to 45, 52 or 53 for use as a medicament.
60. The suspension or formulation of lipid nanoparticles or lipidoid nanoparticles according to any of items 1 to 45, 52 or 53, for use in vaccination or immunization.
61. A method of inducing an immune response against a target pathogen in a subject in need thereof, the method comprising administering a formulation comprising the suspension of lipid nanoparticles or lipidoid nanoparticles as defined in any of items 1 to 45 or 52 to the subject.
62. A method of reducing the occurrence or severity of one or more side effects associated with a a LNP/LiNP based vaccine in a subject, the method comprising administering a vaccine formulation or an anticancer formulation comprising the suspension of lipid nanoparticles or lipidoid nanoparticles as defined in any of items 1 to 45 or 52 to the subject.
63. The method of item 62, wherein the reduction of the occurrence or severity of one or more side effects are caused by a reduction of LNP/LiNP aggregation.
64. The method of item 63, wherein the reduction of aggregation is measured by determining the hydrodynamic diameter of the nanoparticle, for example by via dynamic light scattering or photon correlation spectroscopy.

Further aspects of the invention are summarized in the following second set of items.
1b. Use of a surfactant for stabilizing a suspension of lipid nanoparticles or of lipidoid nanoparticles in an aqueous vehicle solution against particle aggregation under a physical stress condition, wherein the lipid nanoparticles or lipidoid nanoparticles comprise the following components (a) and (b):
   (a) a therapeutic agent, and
   (b) at least one selected from a permanently cationic lipid, an ionizable lipid and an ionizable lipidoid.
2b. A method for stabilizing a suspension of lipid nanoparticles or of lipidoid nanoparticles in an aqueous vehicle solution against particle aggregation under a physical stress condition, wherein the lipid nanoparticles or lipidoid nanoparticles comprise the following components (a) and (b):
   (a) a therapeutic agent and
   (b) at least one selected from a permanently cationic lipid, an ionizable lipid and an ionizable lipidoid,
   and wherein the method comprises incorporating a surfactant into the suspension of lipid nanoparticles or of lipidoid nanoparticles.
3b. The use or method in accordance with item 1b or 2b, wherein the therapeutic agent is a nucleic acid.
4b. The use or method in accordance with item 3b, wherein the nucleic acid is selected from RNA and plasmid DNA.
5b. The use or method in accordance with item 3b, wherein the nucleic acid is selected from mRNA, siRNA, miRNA, antisense RNA, tRNA, and noncoding RNA and is more preferably mRNA.
6b. The use or method in accordance with any of items 3b to 5b, wherein the concentration of the nucleic acid in the suspension ranges from 0.01 to 10 mg/mL, more preferably from 0.02 to 10 mg/mL, still more preferably from 0.05 to 5 mg/mL, and most preferably from 0.05 to 2.5 mg/ml based on the total volume of the suspension.
7b. The use or method in accordance with any of items 1b to 6b, wherein the weight-to-volume ratio of the nanoparticles in the aqueous vehicle solution in gram per liter is in the range of 0.1 g/L to 300 g/L, more preferably 0.2 g/L to 300 g/L, still more preferably 0.5 g/L to 250 g/L and most preferably 0.5 g/L to 125 g/L.
8b. The use or method in accordance with any of items 1b to 7b, wherein the suspended nanoparticles have a Z-average diameter, as determined by dynamic light scattering, in the range of 10 to 500 nm, more preferably in the range of 10 to 250 nm, still more preferably 20 to 200 nm.
9b. The use or method in accordance with any of items 1b to 8b, wherein the suspended nanoparticles have a polydispersity index, as determined by dynamic light scattering, in the range of 0.02 to 0.4, more preferably in the range of 0.03 to 0.2.
10b. The use or method in accordance with any of items 1b to 9b, wherein the nanoparticles further comprise one or more of the following components (c1) to (c6):
   (c1) a non-ionizable lipid having a sterol structure;
   (c2) a phosphoglyceride lipid;
   (c3) a PEG-conjugated lipid;
   (c4) a polysarcosine-conjugated lipid
   (c5) a PASylated lipid; and
   (c6) a cationic polymer.
11b. The use or method in accordance with any of items 1b to 10b, wherein the nanoparticles comprise:
   30 to 65 mol% of at least one of the permanently cationic lipid, ionizable lipid and ionizable lipidoid (b), preferably the ionizable lipid or ionizable lipidoid (b), and one or more of the following components:
   10 to 50 mol% of the lipid having a sterol structure (c1),
   4 to 50 mol% of the phosphoglyceride lipid (c2),
   0.5 to 10 mol% of one of the PEG-conjugated lipid (c3), the polysarcosine-conjugated lipid (c4) and the PASylated lipid (c5), or of any combination thereof,
   0.5 to 10 mol% of the cationic polymer (c6),
   such that the sum of (b) and (c1) to (c6) amounts to 100 mol%.
12b. The use or method in accordance with any of items 1b to 11b, wherein the nanoparticles further comprise the following components (c1) to (c3):
   (c1) a non-ionizable lipid having a sterol structure;
   (c2) a phosphoglyceride lipid; and
   (c3) a PEG-conjugated lipid.
13b. The use or method in accordance with item 12b, wherein the nanoparticles comprise:
   30 to 65 mol% of at least one of the permanently cationic lipid, ionizable lipid and ionizable lipidoid (b), preferably the ionizable lipid or ionizable lipidoid (b),
   10 to 50 mol% of the lipid having a sterol structure (c1),
   4 to 50 mol% of the phosphoglyceride lipid (c2), and
   0.5 to 10 mol% of the PEG-conjugated lipid (c3),
   such that the sum of (b) and (c1) to (c3) amounts to 100 mol%.
14b. The use or method in accordance with any of items 3b to 13b, wherein the nanoparticles further comprise a polyanionic component which is different from the nucleic acid.
15b. The use or method in accordance with any of items 3b to 14b, wherein the composition of the nanoparticles is such that the weight ratio in the nanoparticles of the sum of the weights of components other than the nucleic acid to the weight of the nucleic acid is in the range of 50:1 to 1:1, more preferably 40:1 to 2:1 and most preferably 30:1 to 3:1.
16b. The use or method in accordance with any of items 1b to 15b, wherein the nanoparticles comprise an ionizable lipidoid (b) of the following formula (b-1), 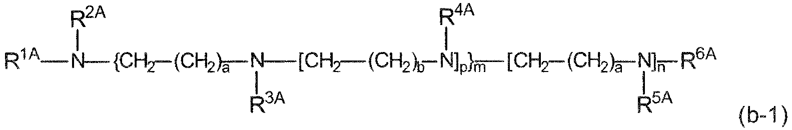 wherein:
   a is 1 and b is an integer of 2 to 4; or a is an integer of 2 to 4 and b is 1,
   p is 1 or 2,
   m is 1 or 2; n is 0 or 1 and m+n is ≥ 2; and
   R^{1A} to R^{6A} are independently of each other selected from: hydrogen; -CH₂-CH(OH)-R^{7A}, -CH(R^{7A})-CH₂-OH, -CH₂-CH₂-(C=O)-O-R^{7A},
   -CH₂-CH₂-(C=O)-NH-R^{7A}; -CH₂-R^{7A}; -C(NH)-NH₂; a poly(ethylene glycol) chain; and a receptor ligand; wherein R^{7A} is selected from C3-C18 alkyl and C3-C18 alkenyl having one C-C double bond;
   provided that at least two residues among R^{1A} to R^{6A} are selected from -CH₂-CH(OH)-R^{7A}, -CH(R^{7A})-CH₂-OH, -CH₂-CH₂-(C=O)-O-R^{7A},
   -CH₂-CH₂-(C=O)-NH-R^{7A} and -CH₂-R^{7A}, wherein R^{7A} is selected from C3-C18 alkyl or C3-C18 alkenyl having one C-C double bond;
   or a protonated form thereof, wherein one or more of the nitrogen atoms contained in the compound of formula (b-1) are protonated to provide a compound carrying a positive charge.
17b. The use or method in accordance with any of items 1b to 16b, wherein the nanoparticles comprise an ionizable lipidoid (b-1) of the following formula (b-1b), 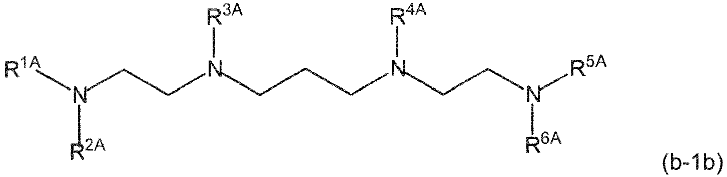 wherein R^{1A} to R^{6A} are defined as in item 16b,
   or a protonated form thereof, wherein one or more of the nitrogen atoms contained in the compound of formula (b-1b) are protonated to provide a compound carrying a positive charge.
18b. The use or method in accordance with item 16b or 17b, wherein R^{1A} to R^{6A} are independently selected from hydrogen and -CH₂-CH(OH)-R^{7A}, wherein R^{7A} is selected from C8-C18 alkyl and C8-C18 alkenyl having one C-C double bond, provided that at least two residues, preferably at least three residues, and more preferably at least four residues, among R^{1A} to R^{6A} are -CH₂-CH(OH)-R^{7A} wherein R^{7A} is selected from C8-C18 alkyl and C8-C18 alkenyl having one C-C double bond.
19b. The use or method in accordance with any of items 1b to 18b, wherein the nanoparticles comprise an ionizable lipidoid dL_05(R) having the formula: 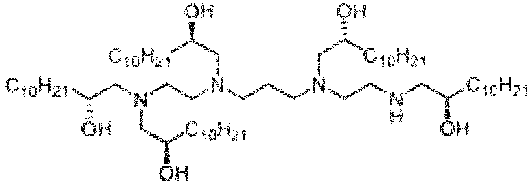
20b. The use or method in accordance with any of items 1b to 15b, wherein the nanoparticles comprise an ionizable lipid (b) of the following formula (a-III): 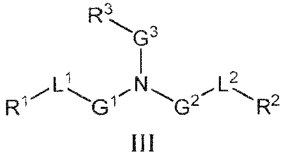 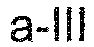 or a pharmaceutically acceptable salt, prodrug or stereoisomer thereof, wherein:
   one of L¹ or L² is -O(C=O)-, -(C=O)O-, -C(=O)-, -O-, -S(O)ₓ-, -S-S-, - C(=O)S-, SC(=O)-, - NR^{a}C(=O)-, -C(=O)NR^{a}-, NR^{a}C(=O)NR^{a}-, -OC(=O)NR^{a}- or - NR^{a}C(=O)O-, and the other of L¹ or L² is-O(C=O)-, -(C=O)O-, -C(=O)-, -O-, -S(O)ₓ-, -S- S-, -C(=O)S-, SC(=O)-, -NR^{a}C(=O)-, -C(=O)NR^{a}-, ,NR^{a}C(=O)NR^{a}-, -OC(=O)NR^{a}- or -NR^{a}C(=O)O- or a direct bond;
   G¹ and G² are each independently C₁-C₁₂ alkylene or C₁-C₁₂ alkenylene;
   G³ is C₁-C₂₄ alkylene, C₁-C₂₄ alkenylene, C₃-C₈ cycloalkylene, C₃-C₈ cycloalkenylene,
   wherein each of alkylene, alkenylene, cycloalkylene, and cyloalkenylene is optionally substituted;
   R^{a} is H or C₁-C₁₂ alkyl wherein alkyl is optionally substituted;
   R¹ and R² are each independently C₆-C₂₄ alkyl or C₆-C₂₄ alkenyl, wherein each of alkyl and alkenyl is optionally substituted ;
   R³ is H, OR⁵, CN, -C(=O)OR⁴, -OC(=O)R⁴ or-NR⁵C(=O)R⁴; R⁴ is C₁-C₁₂ alkyl, wherein alkyl is optionally substituted;
   R⁵ is H or C₁-C₆ alkyl, wherein alkyl is optionally substituted; and
   x is 0, 1 or 2.
21b. The use or method in accordance with any of items 1b to 15b, wherein the nanoparticles comprise, as an ionizable lipid (b), (6Z,9Z,28Z,31Z)-heptatriaconta-6,9,28,31-tetraene-19-yl 4-(dimethylamino)butanoate or a protonated form thereof wherein the nitrogen atom of the compound is protonated to provide a compound carrying a positive charge.
22b. The use or method in accordance with any of items 1b to 15b, wherein the nanoparticles comprise, as an ionizable lipid (b), ((4-hydroxybutyl)azanediyl)bis(hexan-6,1-diyl)bis(2-hexyldecanoate) or a protonated form thereof wherein the nitrogen atom of the compound is protonated to provide a compound carrying a positive charge.
23b. The use or method in accordance with any of items 1b to 15b or 22b, wherein the nanoparticles comprise, as an ionizable lipid (b), heptadecan-9-yl 8-((2-hydroxyethyl)(6-oxo-6-(undecyloxy)hexyl)amino)octanoate (SM-102) or a protonated form thereof wherein the nitrogen atom of the compound is protonated to provide a compound carrying a positive charge.
24b. The use or method in accordance with any of items 10b to 23b, wherein the non-ionizable lipid having a sterol structure (c1) comprises a non-ionizable lipid of formula (c1-1): 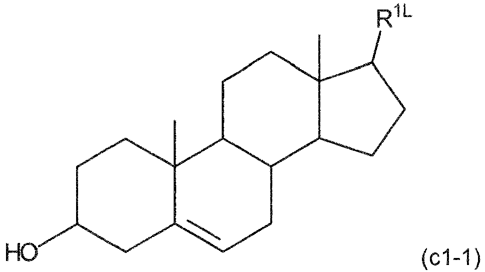 wherein R^{1L} is a C3-C12 alkyl group.
25b. The use or method in accordance with any of items 10b to 24b, wherein the non-ionizable lipid having a sterol structure (c1) comprises cholesterol.
26b. The use or method in accordance with any of items 10b to 25b, wherein the phosphoglyceride lipid (c2) comprises a phosphoglyceride lipid of formula (c2-1) 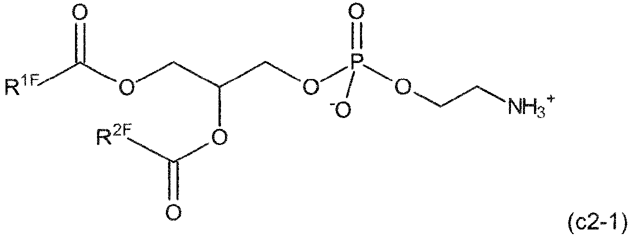 wherein
   R^{1F} and R^{2F} are independently a C8-C18 alkyl group or a C8-C18 alkenyl group, preferably a C12-C18 alkyl group or a C12-C18 alkenyl group,
   or a pharmaceutically acceptable salt thereof;
   or a phosphoglyceride lipid of formula (c2-2) 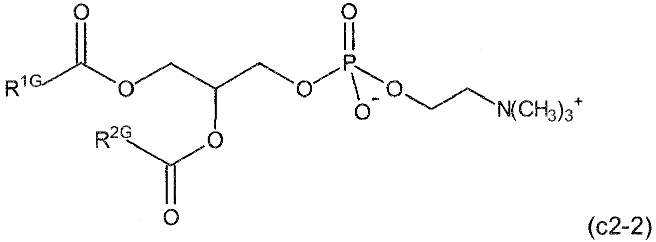 wherein
      R^{1G} and R^{2G} are independently a C8-C18 alkyl group or a C8-C18 alkenyl group, preferably a C12-C18 alkyl group or a C12-C18 alkenyl group,
   or a pharmaceutically acceptable salt thereof.
27b. The use or method in accordance with any of items 10b to 26b, wherein the phosphoglyceride lipid (c2) comprises 1,2-dipalmitoyl-sn-glycero-3-phosphocholine (DPPC) or a pharmaceutically acceptable salt thereof or 1,2-distearoyl-sn-glycero-3-phosphocholine (DSPC) or a pharmaceutically acceptable salt thereof.
28b. The use or method in accordance with any of items 10b to 27b, wherein the PEG-conjugated lipid (c3) comprises a PEG-conjugated lipid of formula (c3-1) 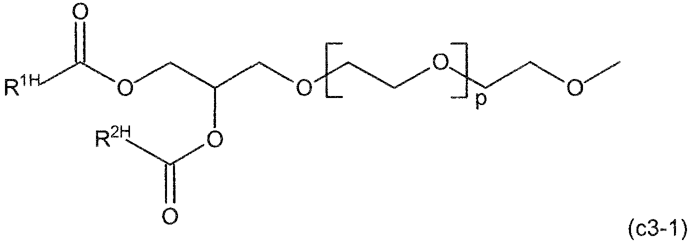 wherein
   R^{1H} and R^{2H} are independently a C8-C18 alkyl group or a C8-C18 alkenyl group, preferably a C12-C18 alkyl group or a C12-C18 alkenyl group, and p is an integer of 5 to 200, preferably 10 to 100, and more preferably 20 to 60;
   or a PEG-conjugated lipid of formula (c3-2) 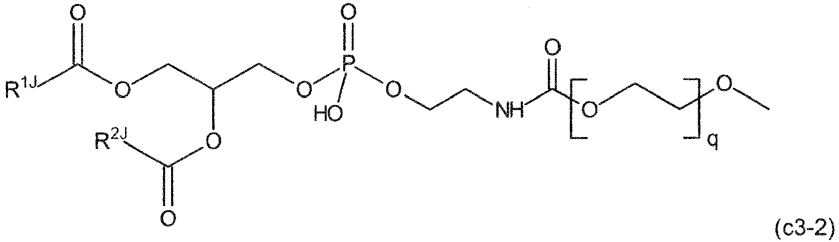 wherein
      R^{1J} and R^{2J} are independently a C8-C18 alkyl group or a C8-C18 alkenyl group, preferably a C12-C18 alkyl group or a C12-C18 alkenyl group, and q is an integer of 5 to 200, preferably 10 to 100, and more preferably 20 to 60,
      or a pharmaceutically acceptable salt thereof,
   or a PEG-conjugated lipid of formula (c3-3) 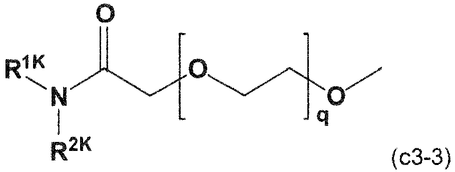 wherein
      R^{1K} and R^{2K} are independently a C8-C18 alkyl group or a C8-C18 alkenyl group, preferably a C12-C18 alkyl group or a C12-C18 alkenyl group, and q is an integer of 5 to 200, preferably 10 to 100, and more preferably 20 to 60.
29b. The use or method in accordance with item 10b to 28b, wherein the PEG conjugated lipid (c3) comprises 1,2-dimyristoyl-sn-glycerolmethoxy(polyethylene glycol)-2000 (DMG-PEG2k) or 2-[(polyethylenglycol)-2000]-N,N-ditetradecylacetamid (ALC-0159).
30b. The use or method in accordance with item 22b, wherein the nanoparticles comprise, as an ionizable lipid (b), the ((4-hydroxybutyl)azanediyl)bis(hexan-6,1-diyl)bis(2-hexyldecanoate) or a protonated form thereof wherein the nitrogen atom of the compound is protonated to provide a compound carrying a positive charge, and further comprise one or more of the following components (d1) to (d8):
   (d1) 2-[(polyethylene glycol)-2000]-N,N-ditetradecylacetamide (ALC-0159)
   (d2) 1,2-distearoyl-sn-glycero-3-phosphocholine (DSPC)
   (d3) cholesterol
   (d4) potassium chloride
   (d5) potassium dihydrogen phosphate
   (d6) sodium chloride
   (d7) disodium phosphate dihydrate
   (d8) sucrose.
31b. The use or method in accordance with item 23b, wherein the nanoparticles comprise, as an ionizable lipid (b), heptadecan-9-yl 8-((2-hydroxyethyl)(6-oxo-6-(undecyloxy)hexyl)amino)octanoate (SM-102) or a protonated form thereof wherein the nitrogen atom of the compound is protonated to provide a compound carrying a positive charge, and further comprise one or more of the following components (e1) to (e7):
   (e1) 1,2-distearoyl-sn-glycero-3-phosphocholine (DSPC),
   (e2) cholesterol,
   (e3) 1,2-dimyristoyl-rac-glycero-3-methoxypolyethylene glycol-2000 (PEG2000 DMG),
   (e4) tromethamol hydrochloride
   (e5) sodium acetate trihydrate
   (e6) acetic acid
   (e7) sucrose.
32b. The use or method in accordance with any of items 1b to 31b, wherein the N/P ratio in the nanoparticles is in the range of 0.5 to 20, more preferably in the range of 0.5 to 10.
33b. The use or method in accordance with any of items 1b to 32b, wherein the physical stress condition is selected from shaking, stirring, vibrating, mixing, inverting, tapping or dropping of the nanoparticle suspension, or any combination thereof, or from a physical stress condition caused by pumping the nanoparticle suspension or by its withdrawal into a syringe.
34b. The use or method in accordance with any of items 1b to 33b, wherein the surfactant is incorporated as an excipient into the aqueous vehicle solution.
35b. The use or method in accordance with any of items 1b to 34b, wherein the surfactant is essentially not attached to the nanoparticles.
36b. The use or method in accordance with any of items 1b to 35b, wherein the surfactant is a nonionic surfactant.
37b. The use or method in accordance with item 36b, wherein the nonionic surfactant is at least one selected from the group consisting of fatty alcohol ethoxylates, fatty acid ethoxylates, block copolymers of ethylene oxide and propylene oxide, alkylphenol ethoxylates or oligomers of alkylphenol ethoxylates, fatty acid esters of sorbitol, ethoxylated fatty acid esters of sorbitol, fatty acid esters of glycerol, ethoxylated castor oil and ethoxylated vitamin E.
38b. The use or method in accordance with item 37b, wherein the block copolymer of ethylene oxide and propylene oxide is a poloxamer.
39b. The use or method in accordance with item 38b, wherein the poloxamer contains one poly(propylene oxide) block B of formula (p-1): 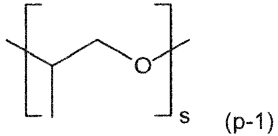
   wherein s is an integer of 15 to 60, and
   two poly(ethylene oxides) blocks A of formula (p-2): 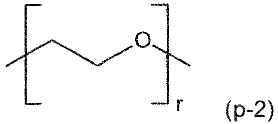
   wherein r is, independently for each block, an integer of 8 to 150, preferably 10 to 150.
40b. The use or method in accordance with item 36b, wherein the nonionic surfactant is at least one selected from the group consisting of fatty alcohol ethoxylates, fatty acid ethoxylates, alkylphenol ethoxylates or oligomers of alkylphenol ethoxylates, fatty acid esters of sorbitol, ethoxylated fatty acid esters of sorbitol, fatty acid esters of glycerol, ethoxylated castor oil and ethoxylated vitamin E.
41b. The use or method in accordance with item 36b or 37b, wherein the nonionic surfactant is at least one selected from the group consisting of poloxamer 124, poloxamer 188, poloxamer 338, poloxamer 407, polysorbate 20, polysorbate 80, polyoxyethylenelaurylether, poyloxyethylene-35 castor oil, D-α-tocopherol polyethylene glycol 1000 succinate, and Tyloxapol.
42b. The use or method in accordance with item 36b or 37b, wherein the nonionic surfactant is at least one selected from the group consisting of polyoxyethylenelaurylether, poyloxyethylene-35 castor oil, D-α-tocopherol polyethylene glycol 1000 succinate, and Tyloxapol.
43b. The use or method in accordance with any of items 1b to 41b, wherein the surfactant does not comprise Poloxamer 188.
44b. The use or method in accordance with any of items 1 to 43b, wherein the surfactant does not comprise Poloxamer 407.
45b. The use in accordance with any of items 1b to 44b, wherein the suspension of lipid nanoparticles or lipidoid nanoparticles in an aqueous vehicle solution comprises the surfactant at a concentration of 0.01 to 10 % (w/v), preferably 0.1 to 10 % (w/v), more preferably 0.25 to 5 % (w/v), still more preferably 0.33 to 2.5 % (w/v), even more preferably 0.45 to 1.5 % (w/v)and most preferably 0.5 to 1.5 % (w/v), with regard to the total volume of the suspension of the nanoparticles in the aqueous vehicle solution.
46b. The use in accordance with item 45b, wherein the suspension of nanoparticles comprises the surfactant at a concentration of 0.5 to 1.5 % (w/v), with regard to the total volume of the suspension of the nanoparticles in the aqueous vehicle solution.
47b. The use in accordance with any of items 1b to 46b, wherein the nanoparticles have not been lyophilized.
48b. The use in accordance with any of items 1b to 46b, wherein the surfactant is not present in the vehicle solution during a lyophilization process.
49b. The use in accordance with any of items 1b to 46b, wherein the surfactant is added before a lyophilization process.
50b. The method in accordance with any of items 2b to 44b, wherein the surfactant is incorporated into the suspension of lipid nanoparticles or of lipidoid nanoparticles to achieve a concentration of the surfactant of 0.01 to 10 % (w/v), preferably 0.1 to 10 % (w/v), more preferably 0.25 to 5 % (w/v), still more preferably 0.33 to 2.5 % (w/v), even more preferably 0.45 to 1.5 % (w/v) and most preferably 0.5 to 1.5 % (w/v), with regard to the total volume of the suspension of the nanoparticles in the aqueous vehicle solution.
51b. The method in accordance with item 50b, wherein the surfactant is incorporated into the suspension of nanoparticles to achieve a concentration of the surfactant of 0.5 to 1.5 % (w/v), with regard to the total volume of the suspension of the nanoparticles in the aqueous vehicle solution.
52b. The method in accordance with any of items 2b to 44b, 50b or 51b, which is free of a lyophilization step.
53b. The method in accordance with any of items 2b to 44b, 50b or 51b, wherein the surfactant is not present in the vehicle solution during a lyophilization step.
54b. The method in accordance with any of items 2b to 44b, 50b or 51b, wherein the surfactant is added before a lyophilization step.
55b. The method in accordance with any of items 2b to 44b, 50b or 51b, wherein the method comprises the following steps:
   i) generating a preparation of lipid nanoparticles or lipidoid nanoparticles by mixing of at least one selected from a permanently cationic lipid, an ionizable lipid and an ionizable lipidoid dissolved in an organic phase with a therapeutic agent dissolved in an aqueous solution,
   ii) diluting the preparation of lipid nanoparticles or lipidoid nanoparticles by dilution with a first solution,
   iii) concentrating the diluted preparation of lipid nanoparticles or lipidoid nanoparticles by buffer exchange using ultra/diafiltration by TFF, wherein a second solution is used for the ultra/diafiltration,
   iv) obtaining a suspension of lipid nanoparticles or lipidoid nanoparticles in an aqueous vehicle solution,

   wherein the first solution comprises between 0.01% w/v and 10% w/v surfactant, preferably between 0.1% w/v and 10% surfactant, more preferably between 0.25% w/v surfactant and 5% w/v surfactant, still more preferably between 0.33% w/v surfactant and 2.5% w/v surfactant, even more preferably between 0.45% w/v and 1.5% w/v surfactant, most preferably between 0.5% w/v and 1.5% w/v surfactant, and/or
   wherein the second solution comprises 0.01% w/v and 10% w/v surfactant, preferably between 0.1% w/v and 10% surfactant, more preferably between 0.25% w/v surfactant and 5% w/v surfactant, still more preferably between 0.33% w/v surfactant and 2.5% w/v surfactant, even more preferably between 0.45% w/v and 1.5% w/v surfactant, most preferably between 0.5% w/v and 1.5% w/v surfactant,
   and wherein the final concentration of surfactant from combined first and second solution is 0.01% w/v and 10% w/v surfactant, preferably between 0.1% w/v and 10% surfactant, more preferably between 0.25% w/v surfactant and 5% w/v surfactant, still more preferably between 0.33% w/v surfactant and 2.5% w/v surfactant, even more preferably between 0.45% w/v and 1.5% w/v surfactant, most preferably between 0.5% w/v and 1.5% w/v surfactant with regard to the total volume of the suspension of the nanoparticles in the aqueous vehicle solution.
56b. The method according to item 55b, wherein the incorporation of the surfactant into the suspension does not occur before or during step i).
57b. The method according to item 55b or 56b, wherein the surfactant is added both with the first and the second solution.
58b. The method according to any of items 55b to 57b, wherein 30 to 70 wt% of the surfactant, preferably 40 to 60 wt%, and more preferably 45 to 55 wt% of the surfactant, based on the total weight of the surfactant in the suspension obtained in step iv), are added with the first solution, and 70 to 30 wt% of the surfactant, preferably 60 to 40 wt%, and more preferably 55 to 45 wt% of the surfactant, based on the total weight of the surfactant in the suspension obtained in step iv), are added with the second solution, such that the sum of the amount of surfactant added with the first and the second solution is 100 wt%.
59b. The method according to any of items 55b to 58b, wherein approximately half of the surfactant is added with the first solution and approximately half of the surfactant is added with the second solution.
60b. A suspension of lipid nanoparticles or lipidoid nanoparticles (LNP or LiNP suspension) obtained by the method according to any of items 55b to 59b.
61b. The suspension of lipid nanoparticles or lipidoid nanoparticles according to of item 60b for use in the treatment or prevention of a disease.
62b. The suspension of lipid nanoparticles or lipidoid nanoparticles according to of item 60b for use as a medicament.
63b. The suspension of lipid nanoparticles or lipidoid nanoparticles according to of item 60b, for use in vaccination or immunization.
64b. A method of avoiding side effects in a therapy with lipid nanoparticles or lipidoid nanoparticles carrying at least one therapeutic agent, wherein the method comprises the steps:
   i) determine whether lipid nanoparticles or lipidoid nanoparticles in a pharmaceutical composition comprising lipid nanoparticles or lipidoid nanoparticles aggregates when subjected to a mechanical stress or a temperature stress, by determining their aggregation level before and after subjecting said pharmaceutical composition is subjected to said mechanical or temperature stress
   ii) if the lipid nanoparticles or lipidoid nanoparticles show aggregation after the test of step (i), then add to the lipid nanoparticles or lipidoid nanoparticles formulation a surfactant to obtain a LNP or LiNP suspension with a final surfactant concentration of between 0.01% w/v and 10% w/v, preferably between 0.1% w/v 10% w/v, more preferably between 0.25% w/w and 5%, still more preferably between 0.33% and 2,5%, even more preferably between 0.45% and 1,5%, most preferably between 0.5% and 1.5%,
   iii) reconstitute with mixing to generate a stable suspension of lipid nanoparticles or lipidoid nanoparticles.
65b. A method of reducing one or more side effects associated with a vaccine formulation or an anticancer formulation comprising lipid nanoparticles or lipidoid nanoparticles carrying a nucleic acid, the method comprising modifying the vaccine formulation or an anticancer formulation by adding a surfactant to a vaccine formulation or anticancer formulation comprising a suspension of lipid nanoparticles or lipidoid nanoparticles.
66b. The method of item 65b, wherein the surfactant represents between between 0.01% w/v and 10% w/v, preferably between 0.1% w/v 10% w/v, more preferably between 0.25% w/w and 5%, still more preferably between 0.33% and 2,5%, even more preferably between 0.45% and 1,5%, most preferably between 0.5% and 1.5%.
67b. The method of item 66b, wherein the surfactant is at least one nonionic surfactant selected from the group of fatty alcohol ethoxylates, fatty acid ethoxylates, block copolymers of ethylene oxide and propylene oxide, alkylphenol ethoxylates or oligomers of alkylphenol ethoxylates, fatty acid esters of sorbitol, ethoxylated fatty acid esters of sorbitol, fatty acid esters of glycerol, ethoxylated castor oil and ethoxylated vitamin E, preferably one selected from the group of poloxamer 124, poloxamer 188, poloxamer 338, poloxamer 407, polysorbate 20, polysorbate 80, polyoxyethylenelaurylether, poyloxyethylene-35 castor oil, D-α-tocopherol polyethylene glycol 1000 succinate, and Tyloxapol.
68b. The method of item 67b, wherein the surfactant is a block copolymer of ethylene oxide and propylene oxide, preferably a poloxamer selected from the group of poloxamer 124, poloxamer 188, poloxamer 338, poloxamer 407.
69b. The method of item 67b, wherein the poloxamer is not poloxamer 188 or poloxamer 407.
70b. A method of inducing an immune response against a target pathogen in a subject in need thereof, the method comprising administering a formulation comprising the suspension of lipid nanoparticles or lipidoid nanoparticles as defined in any of items 1b to 49b or 60b to the subject.
71b. A method of reducing the occurrence or severity of one or more side effects associated with a a LNP/LiNP based vaccine in a subject, the method comprising administering a vaccine formulation or an anticancer formulation comprising the suspension of lipid nanoparticles or lipidoid nanoparticles as defined in any of items 1b to 49b or 60b to the subject.
72b. The method of item 71b, wherein the reduction of the occurrence or severity of one or more side effects are caused by a reduction of LNP/LiNP aggregation.
73b. The method of item 72b, wherein the reduction of aggregation is measured by determining the hydrodynamic diameter of the nanoparticle, for example by via dynamic light scattering or photon correlation spectroscopy.

It will be understood that the summary in the above items forms a part of the general disclosure of the present invention, such that the information provided in the following detailed description, e.g. with regard to further preferred embodiments or optional features, also applies for the above items and *vice versa.*

### DETAILED DESCRIPTION

Unless indicated to the contrary in any specific context, the following explanation, e.g. with respect to therapeutic agents, lipid nanoparticles or lipidoid nanoparticles or surfactants, applies for all aspects of the invention.

In order to facilitate the discussion, the lipid nanoparticles ("LNP") or lipidoid nanoparticles ("LiNP") may be collectively referred to as "nanoparticles" herein. Likewise, the suspension of lipid nanoparticles or of lipidoid nanoparticles in an aqueous vehicle solution, wherein the lipid nanoparticles or lipidoid nanoparticles comprise (a) a nucleic acid and (b) at least one of a permanently cationic lipid, an ionizable lipid and an ionizable lipidoid may be briefly referred to as "nanoparticle suspension" herein. It will be understood that the "or" used when making reference to lipid nanoparticles or lipidoid nanoparticles nanoparticles, unless indicated to the contrary, does not have an exclusive meaning. Thus, a suspension of lipid nanoparticles or lipidoid nanoparticles may comprise lipid nanoparticles but no lipidoid nanoparticles, lipidoid nanoparticles but no lipid nanoparticles, or lipid nanoparticles and lipidoid nanoparticles. The same is expressed, e.g., by the abbreviation LNP/LiNP.

In the following, the nanoparticles of the suspension and their components will be explained. Unless specifically indicated to the contrary, a reference to "nanoparticles" herein encompasses the lipid nanoparticles (also referred to as LNP) as well as the lipidoid nanoparticles (also referred to as LiNP). In accordance with the various aspects of the invention, the nanoparticles of the nanoparticle suspension comprise: (a) a therapeutic agent and (b) at least one selected from a permanently cationic lipid, an ionizable lipid and an ionizable lipidoid. Thus, if a suspension in the context of the invention comprises only LNPs but no LiNPs, components (a) and (b) are contained in the LNPs. If a suspension comprises only LiNPs but no LNPs, components (a) and (b) are contained in the LiNPs. And if the suspension in the context of the invention comprises LNPs and LiNPs, both the LNPs and the LiNPs typically comprise a component (a) and a component (b).

As component (a), the nanoparticles comprise a therapeutic agent. Preferably, the therapeutic agent is a nucleic acid, which thus generally provides a pharmaceutically active ingredient of the nanoparticles.

The nature of the nucleic acid is not particularly limited. In principle any type of nucleic acid can be employed in the context of the present invention. Nucleic acids are known to the skilled person and refer to biopolymers or small biomolecules composed of nucleotides which are the monomers made of three components: a 5-carbon sugar, a phosphate group and a nitrogenous base.

The term nucleic acid is the overall name for DNA (deoxyribonucleic acid) and RNA (ribonucleic acid), i.e., the members of the above family of biopolymers. If the sugar is a compound ribose, the polymer is RNA if the sugar is derived from ribose as deoxyribose, the polymer is DNA. The term "nucleic acid" encompasses oligonucleotides or polynucleotides. As a nucleic acid is a biopolymer composed of nucleotides, the term "nucleic acid" is also often referred to as a "sequence of nucleotides" and, accordingly, as will be understood by the skilled person, the terms "nucleic acid" and "nucleic acid sequence" are often used interchangeably.

In a preferred embodiment, the nanoparticles comprise ribonucleic acid (RNA) as nucleic acid, more preferably single stranded RNA, and most preferred is mRNA.

The term "nucleic acid" encompasses all forms of naturally occurring types of nucleic acids as well as chemically and/or enzymatically synthesized nucleic acids and also encompasses nucleic acid analogues and nucleic acid derivatives. The term in particular includes any backbone-modified, sugar-modified or base-modified single-stranded or double-stranded nucleic acid, such as e.g. locked nucleic acids (LNA), peptide nucleic acids (PNA), oligonucleoside thiophosphates and phosphotriesters, morpholino oligonucleotides, cationic oligonucleotides (US6017700 A, WO/2007/069092), substituted ribo-oligonucleotides or phosphorothioates. Furthermore, the term "nucleic acid" also refers to any molecule that comprises nucleotides or nucleotide analogues. There are no limitations concerning sequence or size of a nucleic acid comprised in the nanoparticles of the present invention. The nucleic acid is predominantly defined by the biological effect that is to be achieved at the biological target the nanoparticles of the present invention are delivered to. For instance, as will be outlined in more detail further below, in the case of an application in gene or nucleic acid therapy, the nucleic acid or nucleic acid sequence can be defined by the gene or gene fragment that is to be expressed or by the intended substitution or repair of a defective gene or any gene target sequence or by the target sequence of a gene to be inhibited, knocked-down, downregulated or up-regulated.

The nanoparticles of the suspension may comprise a nucleic acid being a DNA molecule. A preferred embodiment of such a DNA molecule is a DNA molecule which can be transcribed into an mRNA molecule. Transcription is the first step of gene expression, in which a particular segment of a DNA molecule is copied into an mRNA molecule by the enzyme RNA polymerase. During transcription, a DNA sequence is read by an RNA polymerase, which produces a complementary, anti-parallel RNA strand called a primary transcript.

A DNA molecule may be introduced in a vector, preferably an expression vector, by standard molecular biology techniques (see, e.g. Sambrook et al., Molecular Cloning, A laboratory manual, 2nd Ed, 1989). The term "vector" such as "expression vector" or "cloning vector" in the sense of the present invention is understood as a circular, double-stranded unit of DNA that is preferably able to replicate within a cell independently of the chromosomal DNA and which is used as a vehicle to carry genetic material into a cell, where it can be (replicated and/or) expressed (i.e., transcribed into RNA and translated into a amino acid sequence). A vector containing foreign DNA is termed recombinant DNA. The vector itself is generally a DNA sequence that typically consists of an insert (e.g., a nucleic acid molecule/DNA molecule of the present invention) and a larger sequence that serves as the "backbone" of the vector. Plasmids in the sense of the present invention are most often found in bacteria and are used in recombinant DNA research to transfer genes between cells and are as such a subpopulation of "vectors" as used in the sense of the present invention.

It is evident to the person skilled in the art that further regulatory sequences may be added to the DNA molecule of the invention. For example, transcriptional enhancers and/or sequences which allow for induced expression may be employed. A suitable inducible system is for example tetracycline-regulated gene expression as described, e.g., by Gossen and Bujard, Proc. Natl. Acad. Sci. USA 89 (1992), 5547-5551) and Gossen, Trends Biotech. 12 (1994), 58-62, or a dexamethasone-inducible gene expression system as described, e.g. by Crook, EMBO J. 8 (1989), 513-519. The present invention may also use a vector, preferably an expression vector, comprising the DNA molecule. The vector may be, e.g., a plasmid, cosmid, virus, bacteriophage or another vector used e.g. conventionally in genetic engineering, and may comprise further genes such as marker genes which allow for the selection of said vector in a suitable host cell and under suitable conditions.

If the nucleic acid used in the context of the present invention is a DNA molecule, it can be a plasmid DNA (pDNA) molecule.

As noted above, the nanoparticles preferably comprise ribonucleic acid (RNA) as nucleic acid, more preferably single stranded RNA, and most preferred is mRNA.

As regards RNA, in principle any type of RNA can be employed in the context of the present invention. In a preferred embodiment the RNA is a single-stranded RNA. The term "single-stranded RNA" means a single consecutive chain of ribonucleotides in contrast to RNA molecules in which two or more separate chains form a double-stranded molecule due to hybridization of the separate chains. The term "single-stranded RNA" does not exclude that the single-stranded molecule forms in itself double-stranded structures such as secondary (e.g., loops and stem-loops) or tertiary structures. Examples are tRNA and mRNA but also any other type of single-stranded RNA like antisense-RNA, siRNA, miRNA and the like.

The term "RNA" covers RNA which codes for an amino acid sequence as well as RNA which does not code for an amino acid sequence. It has been suggested that more than 80 % of the genome contains functional DNA elements that do not code for proteins. These noncoding sequences include regulatory DNA elements (binding sites for transcription factors, regulators and coregulators etc.) and sequences that code for transcripts that are never translated into proteins. These transcripts, which are encoded by the genome and transcribed into RNA but do not get translated into proteins, are called noncoding RNAs (ncRNAs). Thus, in one embodiment the RNA is a noncoding RNA. Preferably, the noncoding RNA is a single-stranded molecule. Studies demonstrate that ncRNAs are critical players in gene regulation, maintenance of genomic integrity, cell differentiation, and development, and they are misregulated in various human diseases. There are different types of ncRNAs: short (20-50 nt), medium (50-200 nt), and long (>200 nt) ncRNAs. Short ncRNA includes microRNA (miRNA), small interfering RNA (siRNA), piwi-interacting RNA (piRNA), and transcription initiating RNA (tiRNA). Examples of medium ncRNAs are small nuclear RNAs (snRNAs), small nucleolar RNAs (snoRNAs), transfer RNAs (tRNAs), transcription start-site-associated RNAs (TSSaRNAs), promoter-associated small RNAs (PASRs), and promoter upstream transcripts (PROMPTs). Long noncoding RNAs (IncRNA) include long-intergenic noncoding RNA (lincRNA), antisense-IncRNA, intronic IncRNA, transcribed ultra-conserved RNAs (T-UCRs), and others (Bhan A, Mandal SS, ChemMedChem. 2014 Mar 26. doi: 10.1002/cmdc.201300534). Of the above-mentioned non-coding RNAs only siRNA is double-stranded. Thus, since in a preferred embodiment the noncoding RNA is single-stranded, it is preferred that the noncoding RNA is not siRNA. In another embodiment the RNA is a coding RNA, i.e. an RNA which codes for an amino acid sequence. Such RNA molecules are also referred to as mRNA (messenger RNA) and are single-stranded RNA molecules. The RNA may be made by synthetic chemical and enzymatic methodology known to one of ordinary skill in the art, or by the use of recombinant technology, or may be isolated from natural sources, or by a combination thereof.

Messenger RNAs (mRNA) are copolymers which are built up of nucleoside phosphate building blocks mainly with adenosine, cytidine, uridine and guanosine as nucleosides, which as intermediate carriers bring the genetic information from the DNA in the cell nucleus into the cytoplasm, where it is translated into proteins. They are thus suitable as alternatives for gene expression.

In the context of the present invention, mRNA should be understood to mean any polyribonucleotide molecule which, if it comes into the cell, is suitable for the expression of a protein or fragment thereof or is translatable to a protein or fragment thereof. The term "protein" here encompasses any kind of amino acid sequence, i.e. chains of two or more amino acids which are each linked via peptide bonds and also includes peptides and fusion proteins.

The mRNA contains a ribonucleotide sequence which encodes a protein or fragment thereof whose function in the cell or in the vicinity of the cell is needed or beneficial, e.g. a protein the lack or defective form of which is a trigger for a disease or an illness, the provision of which can moderate or prevent a disease or disorder, or a protein which can promote a process which is beneficial for the body, in a cell or its vicinity. The mRNA may contain the sequence for the complete protein or a functional variant thereof. Further, the ribonucleotide sequence can encode a protein which acts as a factor, inducer, regulator, stimulator or enzyme, or a functional fragment thereof, where this protein is one whose function is necessary in order to remedy a disorder, in particular a metabolic disorder or in order to initiate processes in vivo such as the formation of new blood vessels, tissues, etc. Examples for proteins which can be encoded by mRNA include antibodies, cytokines or chemokines. Here, functional variant is understood to mean a fragment which in the cell can undertake the function of the protein whose function in the cell is needed or the lack or defective form whereof is pathogenic. In addition, the mRNA may also have further functional regions and/or 3' or 5' noncoding regions, in particular 3' and/or 5' UTRs. The 3' and/or 5' noncoding regions can be the regions naturally flanking the protein-encoding sequence or artificial sequences, e.g. sequences which contribute to the stabilization of the RNA. Those skilled in the art can determine the sequences suitable for this in each case by routine experiments.

In a preferred embodiment, the mRNA contains a 5'-cap (five-prime-cap; cap-0) consisting of a m7GpppG connected to the mRNA via a 5' to 5' triphosphate linkage, an additional methyl group onto the penultimate nucleotide from the 5'-end of the mRNA (Cap-1, Anti-Reverse Cap Analog (ARCA)) and/or an internal ribosome entry site (IRES) and/or a polyA-tail at the 3'-end, in particular, in order to improve translation. The mRNA can have further regions promoting translation such as, for example, cap-2 structures or histone stem-loop structures.

The RNA which may be present in the nanoparticles may contain unmodified and modified nucleotides. The term "unmodified nucleotide" used herein refers to A, C, G and U nucleotides. The term "modified nucleotide" used herein refers to any naturally occurring or non-naturally occurring isomers of A, C, G and U nucleotides as well as to any naturally occurring or naturally occurring analogues, alternative or modified nucleotide or isomer thereof having for example chemical modifications or substituted residues. Modified nucleotides can have a base modification and/or a sugar modification. Modified nucleotides can also have phosphate group modifications, e.g., with respect to the 5'- prime cap of an mRNA molecule. Modified nucleotides also include nucleotides that are synthesized post-transcriptionally by covalent modification of the nucleotides. Further, any suitable mixture of non-modified and modified nucleotides is possible. A non-limiting number of examples of modified nucleotides can be found in the literature (e.g. US 2013/0123481 A1; Cantara et al., Nucleic Acids Res, 2011, 39(Issue suppl_1):D195-D201; Helm and Alfonzo, Chem Biol, 2014, 21(2):174-185; or Carell et al., Angew Chem Int Ed Engl, 2012, 51(29):7110-31) and some preferable modified nucleotides are mentioned exemplarily in the following based on their respective nucleoside residue:
1-methyladenosine, 2-methylthio-N6-hydroxynorvalyl carbamoyladenosine, 2-methyladenosine, 2'-O-ribosylphosphate adenosine, N6-methyl-N6-threonylcarbamoyladenosine, N6-acetyladenosine, N6-glycinylcarbamoyladenosine, N6-isopentenyladenosine, N6-methyladenosine, N6-threonylcarbamoyladenosine, N6,N6-dimethyladenosine, N6-(cis-hydroxyisopentenyl)adenosine, N6-hydroxynorvalylcarbamoyladenosine, 1,2'-O-dimethyladenosine, N6,2'-O-dimethyladenosine, 2'-O-methyladenosine, N6,N6,2'-O-trimethyladenosine, 2-methylthio-N6-(cis-hydroxyisopentenyl)adenosine, 2-methylthio-N6-methyladenosine, 2-methylthio-N6-isopentenyladenosine, 2-methylthio-N6-threonyl carbamoyladenosine, N6-2-methylthio-N6-threonyl carbamoyladenosine, 2-methylthio-N6-(cis-hydroxyisopentenyl)adenosine, 7-methyladenosine, 2-methylthio-adenosine, 2-methoxy-adenosine, 2'-amino-2'-deoxyadenosine, 2'-azido-2'-deoxyadenosine, 2'-fluoro-2'-deoxyadenosine, 2-aminopurine, 2,6-diaminopurine, 7-deaza-adenosine, 7-deaza-8-aza-adenosine, 7-deaza-2-aminopurine, 7-deaza-8-aza-2-aminopurine, 7-deaza-2,6-diaminopurine, 7-deaza-8-aza-2,6-diaminopurine; 2-thiocytidine, 3-methylcytidine, N4-acetylcytidine, 5-formylcytidine, N4-methylcytidine, 5-methylcytidine, 5-hydroxymethylcytidine, 5-hydroxycytidine, lysidine, N4-acetyl-2'-O-methylcytidine, 5-formyl-2'-O-methylcytidine, 5,2'-O-dimethylcytidine, 2-O-methylcytidine, N4,2'-O-dimethylcytidine, N4,N4,2'-O-trimethylcytidine, isocytidine, pseudocytidine, pseudoisocytidine, 2-thio-cytidine, 2'-methyl-2'-deoxycytidine, 2'-amino-2'-deoxycytidine, 2'-fluoro-2'-deoxycytidine, 5-iodocytidine, 5-bromocytidine, 2'-azido-2'-deoxycytidine, 2'-amino-2'-deoxycytidine, 2'-fluor-2'-deoxycytidine, 5-aza-cytidine, 3-methyl-cytidine, 1-methyl-pseudoisocytidine, pyrrolo-cytidine, pyrrolo-pseudoisocytidine, 2-thio-5-methyl-cytidine, 4-thio-pseudoisocytidine, 4-thio-l-methyl-pseudoisocytidine, 4-thio-l-methyl-1-deaza-pseudoisocytidine, 1-methyl-l-deaza-pseudoisocytidine, 2-methoxy-cytidine, 2-methoxy-5-methyl-cytidine, 4-methoxy-pseudoisocytidine, 4-methoxy-l-methyl-pseudoisocytidine, zebularine,5-aza-zebularine, 5-methyl-zebularine, 5-aza-2-thio-zebularine, 2-thio-zebularine; 1-methylguanosine, N2,7-dimethylguanosine, N2-methylguanosine, 2'-O-ribosylphosphate guanosine, 7-methylguanosine, hydroxywybutosine, 7-aminomethyl-7-deazaguanosine, 7-cyano-7-deazaguanosine, N2,N2-dimethylguanosine, N2,7,2'-O-trimethylguanosine, N2,2'-O-dimethylguanosine, 1,2'-O-dimethylguanosine, 2'-O-methylguanosine, N2,N2,2'-O-trimethylguanosine, N2,N2J-trimethylguanosine, Isoguanosine, 4-demethylwyosine, epoxyqueuosine, undermodified hydroxywybutosine, methylated undermodified hydroxywybutosine, isowyosine, peroxywybutosine, galactosyl-queuosine, mannosyl-queuosine, queuosine, archaeosine, wybutosine, methylwyosine, wyosine, 7-aminocarboxypropyldemethylwyosine, 7-aminocarboxypropylwyosine, 7-aminocarboxypropylwyosinemethylester, 7-deaza-guanosine, 7-deaza-8-aza-guanosine, 6-thio-guanosine, 6-thio-7-deaza-guanosine, 6-thio-7-deaza-8-aza-guanosine, 7-methylguanosine, 6-thio-7-methyl-guanosine, 7-methylinosine, 6-methoxy-guanosine, 1-methylguanosine, 8-oxo-guanosine, 7-methyl-8-oxo-guanosine, 1-methyl-6-thio-guanosine, N2-methyl-6-thio-guanosine, N2,N2-dimethyl-6-thio-guanosine, N1-methylguanosine, 2'-amino-3'-deoxyguanosine, 2'-azido-2'-deoxyguanosine, 2'-fluoro-2'-deoxyguanosine, 2-thiouridine, 3-(3-amino-3-carboxypropyl)uridine, 3-methyluridine, 4-thiouridine, 5-methyl-2-thiouridine, 5-methylaminomethyluridine, 5-carboxymethyluridine, 5-carboxymethylaminomethyluridine, 5-hydroxyuridine, 5-methyluridine, 5-taurinomethyluridine, 5-carbamoylmethyluridine, 5-(carboxyhydroxymethyl)uridine methyl ester, dihydrouridine, 5-methyldihydrouridine, 5-methylaminomethyl-2-thiouridine, 5-(carboxyhydroxymethyl)uridine, 5-(carboxyhydroxymethyl)-2'-O-methyluridine methyl ester, 5-(isopentenylaminomethyl)uridine, 5-(isopentenylaminomethyl)-2-thiouridine, 3,2'-O-dimethyluridine, 5-carboxymethylaminomethyl-2'-O-methyluridine, 5-carbamoylhydroxymethyluridine, 5-carbamoylmethyl-2'-O-methyluridine, 5-carbamoylmethyl-2-thiouridine,5-methoxycarbonylmethyl-2'-O-methyluridine,5-(isopentenylaminomethyl)-2'-O-methyluridine, 5,2'-O-dimethyluridine, 2'-O-methyluridine, 2'-O-methyl-2-thiorudine, 2-thio-2'-O-methyluridine, uridine 5-oxyacetic acid, 5-methoxycarbonylmethyluridine, uridine 5-oxyacetic acid methyl ester, 5-methoxyuridine, 5-aminomethyl-2-thiouridine, 5-carboxymethylaminomethyl-2-thiouridine, 5-methylaminomethyl-2-selenouridine, 5-methoxycarbonylmethyl-2-thiouridine, 5-taurinomethyl-2-thiouridine, pseudouridine, 1-methyl-3-(3-amino-3-carboxypropyl)pseudouridine, 1-methylpseudouridine, 3-methylpseudouridine, 2'-O-methylpseudouridine, 5-formyluridine, 5-aminomethyl-2-geranyluridine, 5-taurinomethyluridine, 5-iodouridine, 5-bromouridine, 2'-methyl-2'-deoxyuridine, 2'-amino-2'-deoxyuridine, 2'-azido-2'-deoxyuridine, 2'-fluoro-2'-deoxyuridine, inosine, 1-methylinosine, 1,2'-O-dimethylinosine, 2'-O-methylinosine, 5-aza-uridine, 2-thio-5-aza-uridine, 4-thio-pseudouridine, 2-thio-pseudouridine, 5-carboxymethyl-uridine, 1-carboxymethyl-pseudouridine, 5-propynyl-uridine, 1-propynyl-pseudouridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-l-methyl-pseudouridine, 2-thio-l-methyl-pseudouridine, 1-methyl-l-deaza-pseudouridine, 2-thio-1-methyl-l-deaza-pseudouridine, dihydropseudouridine, 2-thio-dihydrouridine, 2-thio-dihydropseudouridine, 2-methoxyuridine, 2-methoxy-4-thio-uridine, 4-methoxy-pseudouridine, 4-methoxy-2-thio-pseudouridine, 1,2'-O-dimethyladenosine, 1,2'-O-dimethylguanosine, 1,2'-O-dimethylinosine, 2,8-dimethyladenosine, 2-methylthiomethylenethio-N6-isopentenyl-adenosine, 2-geranylthiouridine, 2-lysidine, 2-methylthio cyclic N6-threonylcarbamoyladenosine, 2-methylthio-N6-(cis-hydroxyisopentenyl) adenosine, 2-methylthio-N6-hydroxynorvalylcarbamoyladenosine, 2-methylthio-N6-threonylcarbamoyladenosine, 2-selenouridine, 2-thio-2'-O-methyluridine, 2'-O-methyladenosine, 2'-O-methylcytidine, 2'-O-methylguanosine, 2'-O-methylinosine, 2'-O-methylpseudouridine, 2'-O-methyluridine, 2'-O-methyluridine 5-oxyacetic acid methyl ester, 2'-O-ribosyladenosinephosphate, 2'-O-ribosylguanosinephosphate, 3,2'-O-dimethyluridine, 3-(3-amino-3-carboxypropyl)-5,6-dihydrouridine, 3-(3-amino-3-carboxypropyl)pseudouridine, 5,2'-O-dimethylcytidine, 5,2'-O-dimethyluridine, 5-(carboxyhydroxymethyl)-2'-O-methyluridine methyl ester, 55-(isopentenylaminomethyl)-2'-O-methyluridine, 5-aminomethyl-2-geranylthiouridine, 5-aminomethyl-2-selenouridine, 5-aminomethyluridine, 5-carbamoylmethyl-2'-O-methyluridine, 5-carboxyhydroxymethyluridine, 5-carboxymethyl-2-thiouridine, 5-carboxymethylaminomethyl-2-geranylthiouridine, 5-carboxymethylaminomethyl-2-selenouridine, 5-carboxymethylaminomethyl-2'-O-methyluridine, 5-cyanomethyluridine, 5-formyl-2'-O-methylcytidine, 5-methoxycarbonylmethyl-2'-O-methyluridine, 5-methylaminomethyl-2-geranylthiouridine, 7-aminocarboxypropyl-demethylwyosine, 7-methylguanosine, 8-methyladenosine, N2,2'-O-dimethylguanosine, N2,7,2'-O-trimethylguanosine, N2,7-dimethylguanosine, N2,N2,2'-O-trimethylguanosine, N2,N2,7-trimethylguanosine, N2,N2,7-trimethylguanosine , N4,2'-O-dimethylcytidine, N4,N4,2'-O-trimethylcytidine, N4,N4-dimethylcytidine, N4-acetyl-2'-O-methylcytidine, N6,2'-O-dimethyladenosine, N6,N6,2'-O-trimethyladenosine, N6-formyladenosine, N6-hydroxymethyladenosine, agmatidine, 2-methylthio cyclic N6-threonylcarbamoyladenosine, glutamyl-queuosine, guanosine added to any nucleotide, guanylylated 5' end , hydroxy-N6-threonylcarbamoyladenosine; most preferably pseudo-uridine, N1-methyl-pseudo-uridine, 2'-fluoro-2'-deoxycytidine, 5-iodocytidine, 5-methylcytidine, 2-thiouridine, 5-iodouridine and/or 5-methyl-uridine.

Furthermore, the term "modified nucleotide" comprises nucleotides containing isotopes such as deuterium. The term "isotope" refers to an element having the same number of protons but different number of neutrons resulting in different mass numbers. Thus, isotopes of hydrogen for example are not limited to deuterium, but include also tritium. Furthermore, the polyribonucleotide can also contain isotopes of other elements including for example carbon, oxygen, nitrogen and phosphor. It is also possible that modified nucleotides are deuterated or contain another isotope of hydrogen or of oxygen, carbon, nitrogen or phosphorus.

Among the U, C, A and G nucleotides either none, one, two, three or all of them can be modified. Hence, in some embodiments, at least one nucleotide of one nucleotide type, e.g. at least one U nucleotide, can be a modified nucleotide. In some embodiments, at least one nucleotide of in total two nucleotide types, e.g., at least one U nucleotide and at least one C nucleotide, can be a modified nucleotide. In some embodiments, at least one nucleotide of in total three nucleotide types, e.g., at least one G nucleotide, at least one U nucleotide and at least one C nucleotide, can be a modified nucleotide. In some embodiments, at least one nucleotide of all four nucleotide types can be a modified nucleotide. In all these embodiments one or more nucleotides per nucleotide type can be modified, the percentage of said modified nucleotides of per nucleotide type being 0%, 2.5%, 5%, 7.5%, 10%, 15%, 20%, 25%, 30%, 35%, 40%, 45%, 50%, 55%, 60%, 65%, 70%, 75%, 80%, 85%, 90% or 100%.

In some embodiments, the total percentage of modified nucleotides comprised in the mRNA molecules is 0%, 2.5%, 5 %, 7.5%, 10%, 15%, 20%, 25%, 30%, 35%, 40%, 45%, 50%, 55%, 60%, 65%, 70%, 75%, 80%, 85%, 90% or 100%.

In a preferred embodiment the mRNA is an mRNA which contains a combination of modified and unmodified nucleotides. Preferably, it is an mRNA containing a combination of modified and unmodified nucleotides as described in WO2011/012316. The mRNA described therein is reported to show an increased stability and diminished immunogenicity. In a preferred embodiment, in such a modified mRNA 5 to 50% of the cytidine nucleotides and 5 to 50% of the uridine nucleotides are modified. In another preferred embodiment, 5 to 50% of the uridine nucleotides are replaced by N1-methyl-pseudo-uridine. The adenosine- and guanosine-containing nucleotides can be unmodified. The adenosine and guanosine nucleotides can be unmodified or partially modified, and they are preferably present in unmodified form.

In certain embodiments of any of the foregoing, the percentage of analogues of a given nucleotide refers to input percentage (e.g., the percentage of analogues in a starting reaction, such as a starting in vitro transcription reaction). In certain embodiments of any of the foregoing, the percentage of analogues of a given nucleotide refers to output (e.g., the percentage in a synthesized or transcribed compound). Both options are equally contemplated.

The RNA, preferably the mRNA, molecules may be produced recombinantly in *in vivo* systems by methods known to a person skilled in the art.

Alternatively, the modified RNA, preferably the mRNA molecules may be produced in an *in vitro* system using, for example, an *in vitro* transcription system which is known to the person skilled in the art. An *in vitro* transcription system capable of producing RNA, preferably mRNA requires an input mixture of modified and unmodified nucleoside triphosphates to produce modified RNA. In certain embodiments, 5 to 50% of the cytidines are analogues of cytidine in such an input mixture and 5 to 50% of the uridines are analogues of uridine in such an input mixture. In certain embodiments, 5 to 40% of the cytidines are analogues of cytidine in such an input mixture and 5 to 40% of the uridines are analogues of uridine in such an input mixture. In certain embodiments, 5 to 30% of the cytidines are analogues of cytidine in such a mixture and 5 to 30% of the uridines are analogues of uridine in such an input mixture. In certain embodiments, 5 to 30% of the cytidines are analogues of cytidine in such mixture and 10 to 30% of the uridines are analogues of uridine in such mixture. In certain embodiments, 5 to 20% of the cytidines are analogues of cytidine in such an input mixture and 5 to 20% of the uridines are analogues of uridine in such an input mixture. In certain embodiments, 5 to 10% of the cytidines are analogues of cytidine in such an input mixture and 5 to 10% of the uridines are analogues of uridine in such an input mixture. In certain embodiments, 25% of the cytidines are analogues of cytidine in such an input mixture and 25% of the uridines are analogues of uridine in such an input mixture. In certain embodiments, the input mixture does not comprise analogues of adenosine and/or guanosine. In other embodiments, optionally, the input mixture comprises one or more analogues of adenosine and/or guanosine (or none of either or both).

In certain embodiments, the percentage of cytidines in an input mixture that are analogues of cytidine is not the same as the percentage of uridines in an input mixture that are analogues of uridine. In certain embodiments, the percentage of analogues of cytidine in an input mixture is lower than the percentage of analogues of uridine in an input mixture. As noted above, this may be in the presence or the absence of analogues of adenosine and guanosine in the input mixture but, in certain embodiments, is in the absence of analogues of adenosine and analogues of guanosine in the input mixture.

In certain embodiments, an input mixture of nucleotides for an *in vitro* transcription system that produces a RNA, preferably mRNA of the present invention comprises analogues of cytidine and analogues of uridine, and 5 to 20% of the cytidines of the input mixture are analogues of cytidine and 25 to 45% of the uridines of the input mixture are analogues of uridine. In other words, the input mixture comprises modified and unmodified cytidines and modified and unmodified uridines, and 5 to 20% of the cytidines of the input mixture comprise analogues of cytidine while 25 to 45% of the uridines of the input mixture comprise analogues of uridine. In other embodiments, the input mixture comprises 5 to 10% analogues of cytidine and 30 to 40% analogues of uridine, such as 7-9% analogues of cytidine, such as 7, 7.5 or 8% and, such as 32-38% analogues of uridine, such as 33, 34, 35, 36%.

In certain embodiments, any of the analogues of uridine and analogues of cytidine described herein may be used, optionally excluding pseudouridine. In certain embodiments, the analogue of cytidine comprises or consists of (e.g., it is the single C analogue type used) 5-iodocytidine and the analogue of uridine comprises or consists of (e.g., it is the single U analogue type used) 5-iodouridine.

Exemplary analogues are described above. It should be understood that for modified polyribonucleotides encoding the desired polypeptide, the analogues and level of modification is, unless indicated otherwise, considered across the entire polyribonucleotide encoding the desired polypeptide, including 5' and 3' untranslated regions (e.g., the level of modification is based on input ratios of analogues in an in vitro transcription reaction such that analogues may be incorporated at positions that are transcribed).

Furthermore, the modified RNA, preferably mRNA molecules may be chemically synthesized, e.g., by conventional chemical synthesis on an automated nucleotide sequence synthesizer using a solid-phase support and standard techniques or by chemical synthesis of the respective DNA sequences and subsequent *in vitro* or *in vivo* transcription of the same.

In another preferred embodiment, the mRNA may be combined with target binding sites, targeting sequences and/or with micro-RNA binding sites, in order to allow activity of the desired mRNA only in the relevant cells. In a further preferred embodiment, the RNA can be combined with micro-RNAs or shRNAs in the untranslated regions.

In general, therapeutic effects can be achieved by the interaction of the ribonucleic acid with cellular molecules and organelles. Such interaction alone may for example activate the innate immune system, as is the case for certain CpG oligonucleotides and sequences designed to specifically interact with toll-like and other extra- or intracellular receptors. Furthermore, the uptake or introduction of nucleic acids (preferably ribonucleic acids, more preferably mRNAs) in cells can be intended to lead to the expression of nucleotide sequences such as genes comprised in the nucleic acid (preferably ribonucleic acids, more preferably the mRNA), can be intended for the downregulation, silencing or knockdown of endogenous gene expression as a consequence of the intracellular presence of an introduced exogenous nucleic acid, or can be intended for the modification of endogenous nucleic acid sequences such as repair, excision, insertion or exchange of selected bases or of whole stretches of endogenous nucleic acid sequences, or can be intended for interference with virtually any cellular process as a consequence of the intracellular presence and interaction of an introduced exogenous ribonucleic acid (preferably an mRNA). Overexpression of introduced exogenous nucleic acids (preferably ribonucleic acids, more preferably mRNAs) may be intended to compensate or complement endogenous gene expression, in particular in cases where an endogenous gene is defective or silent, leading to no, insufficient or a defective or a dysfunctional product of gene expression such as is the case with many metabolic and hereditary diseases like cystic fibrosis, hemophilia or muscular dystrophy to name a few. Overexpression of introduced exogenous nucleic acids (preferably ribonucleic acids, more preferably mRNAs) may also be intended to have the product of the expression interact or interfere with any endogenous cellular process such as the regulation of gene expression, signal transduction and other cellular processes. The overexpression of introduced exogenous nucleic acids (preferably ribonucleic acids, more preferably mRNAs) may also be intended to give rise to an immune response in context of the organism in which a transfected or transduced cell resides or is made to reside. Examples are the genetic modification of antigen-presenting cells such as dendritic cells in order to have them present an antigen for vaccination purposes. Other examples are the overexpression of cytokines in tumors in order to elicit a tumor-specific immune response. Furthermore, the overexpression of introduced exogenous ribonucleic acids (preferably mRNAs) may also be intended to generate in vivo or ex vivo transiently genetically modified cells for cellular therapies such as modified T-cells, NK cells and other lymphocytes or precursor or stem or other cells for regenerative medicine.

Downregulation, silencing or knockdown of endogenous gene expression for therapeutic purposes can for example be achieved by RNA interference (RNAi), with ribozymes, antisense oligonucleotides, tRNAs, long double-stranded RNA where such downregulation can be sequence-specific or unspecific and can also lead to cell death as is the case when long double-stranded RNAs are introduced into cells. Downregulation, silencing or knockdown of endogenous or pre-existing gene expression can be useful in the treatment of acquired, hereditary or spontaneously incurring diseases including viral infections and cancer. It can also be envisaged that the introduction of nucleic acids into cells can be practiced as a preventive measure in order to prevent, for example, viral infection or neoplasias. Downregulation, silencing or knockdown of endogenous gene expression can be exerted on the transcriptional level and on the translational level. Multiple mechanisms are known to the one skilled in the art and include for example epigenetic modifications, changes in chromatin structure, selective binding of transcription factors by the introduced nucleic acid, hybridization of the introduced nucleic acid to complementary sequences in genomic DNA, mRNA or other RNA species by base pairing including unconventional base pairing mechanisms such as triple helix formation. Similarly, gene repair, base or sequence changes can be achieved at the genomic level and at the mRNA level including exon skipping. Base or sequence changes can for example be achieved by RNA-guided site-specific DNA cleavage, by cut and paste mechanisms exploiting trans-splicing, trans-splicing ribozymes, chimeraplasts, splicosome-mediated RNA trans-splicing, or by exploiting group II or retargeted introns, or by exploiting insertional mutagenesis mediated by viruses or exploiting targeted genomic insertion using prokaryotic, eukaryotic or viral integrase systems. As nucleic acids are the carriers of the building plans of living systems and as they participate in many cellular processes in a direct and indirect manner, in theory any cellular process can be influenced by the introduction of nucleic acids into cells from outside. Notably, this introduction can be carried out directly in vivo and ex vivo in cell or organ culture followed by transplantation of thus modified organs or cells into a recipient. The particles for use in the context of the present invention with nucleic acids as therapeutically active agent may be useful for all purposes described above.

As mentioned above, the RNA, preferably the mRNA, may contain a ribonucleotide sequence which encodes a protein or fragment thereof whose function in the cell or in the vicinity of the cell is needed or beneficial, e.g. a protein the lack or defective form of which is a trigger for a disease or an illness, the provision of which can moderate or prevent a disease or an illness, or a protein which can promote a process which is beneficial for the body, in a cell or its vicinity.

Indeed, in recent years, RNA (in particular, mRNA) has become increasingly relevant as a new drug entity. As opposed to DNA-based gene therapeutics, mRNA does not need to be transported into the nucleus but is directly translated into protein in the cytoplasm (J Control Release, 2011, 150:238-247, and Eur J Pharm Biopharm, 2009, 71:484-489).

Moreover, numerous genetic disorders, caused by the mutation of a single gene are known and candidates for RNA, preferably mRNA, therapeutic approaches. Disorders caused by single-gene mutations, like cystic fibrosis, hemophilia and many others, can be dominant or recessive with respect to the likelihood that a certain trait will appear in the offspring. While a dominant allele manifests a phenotype in individuals who have only one copy of the allele, for a recessive allele the individual must have two copies, one from each parent to become manifest. In contrast, polygenic disorders are caused by two or more genes and the manifestation of the respective disease is often fluent and associated to environmental factors. Examples for polygenic disorders are hypertension, elevated cholesterol level, cancer, neurodegenerative disorders, mental illness and others. Also in these cases therapeutic RNA, preferably the mRNA, representing one or more of these genes may be beneficial to those subjects. Furthermore, a genetic disorder must not have been passed down from the parents' genes, but can also be caused by new mutations. Also in these cases therapeutic RNA, preferably the mRNA, representing the correct gene sequence may be beneficial to the subjects.

An online catalog with presently 22,993 entries of Human Genes and Genetic Disorders together with their respective genes and a description of their phenotypes are available at the ONIM (Online Mendelian Inheritance in Man) webpage (http://onim.org); sequences of each are available from the Uniprot database (http://www.uniprot.org). As non-limiting examples, the following **Table A** lists some congenital diseases and disorders, and the corresponding gene(s). Due to the high degree of interaction of cellular signaling pathways, the mutation of a certain gene causes a multiply of pathogenic symptoms, of which only a characteristic one is listed in **Table A.**

In some embodiments of the present invention, the therapeutic protein which is encoded by the RNA, preferably the mRNA, which may be present in the suspension formulation and the aerosol of the present invention is chosen from the cellular proteins listed in **Table A.** Thus, the RNA, preferably the mRNA, molecule may encode a therapeutic cellular protein, wherein the encoded therapeutic protein is one listed in **Table A** or a homolog thereof.

In another embodiment of the present invention, the therapeutic protein which is encoded by the RNA, preferably the mRNA, is chosen from the secreted proteins listed in **Table A.** Thus, the RNA, preferably the mRNA, may encode a therapeutic fusion protein, wherein the encoded therapeutic protein or a homolog thereof is one listed in **Table A** and the second protein is a signal peptide that allows the secretion of the therapeutic protein. A signal peptide is a short, typically 5-30 amino acids long sequence present at the N-terminus of said therapeutic protein and that leads the fusion protein towards the cell's secretory pathway via certain organelles (i.e. the endoplasmic reticulum, the golgi-apparatus or the endosomes). Thus, such fusion protein is secreted from the cell or from a cellular organelle or inserted into a cellular membrane (e.g. multi-spanning trans- membrane proteins) at a cellular compartment or at the cell's surface.

Thus, in preferred embodiments of the present invention the RNA, preferably the mRNA, may encode one or more, but is not limited to, the following proteins of the genes that cause, predispose or protect from diseases. Non-limiting examples of such diseases or disorders that may be treated (or prevented) include those wherein said polypeptide, protein or peptide is selected from the group consisting of the ones as outlined in the following **Table A.**

In some embodiments, the encoding sequence of the RNA, preferably the mRNA, may be transcribed and translated into a partial or full-length protein comprising cellular activity at a level equal to or greater than that of the native protein. In some embodiments, the RNA, preferably the mRNA, encodes a therapeutically or pharmaceutically active polypeptide, protein or peptide having a therapeutic or preventive effect, wherein said polypeptide, protein or peptide is selected from the group consisting of the ones as outlined in the following **Table A.** The RNA, preferably the mRNA, more specifically the encoding sequence thereof, may be used to express a partial or full-length protein with cellular activity at a level equal to or less than that of the native protein. This may allow the treatment of diseases for which the administration of an RNA molecule can be indicated.

**Table A: Non-limiting examples of human genes and genetic diseases or disorders**

| **Disease** | | **Pathology** | **Gene, heredity** |
|---|---|---|---|
| | **Blood diseases** | | |
| Fanconi Anemia | | Anemia and neutropenia, evidence that a DNA repair mechanism is affected | FANCA, autosomal recessive |
| Hemophilia-A | | Abnormal bleeding | Coagulation Factor VIII, X-chromosomal recessive |
| Hemophilia-B | | Abnormal bleeding | Coagulation Factor IX, X-chromosomal recessive |
| Hereditary Spherocytosis (various types) | | spherical-shaped erythrocytes (spherocytes) | Ankyrin (ANK1) |
| Paroxysmal nocturnal hemoglobinuria | | Anemia and presence of blood in the urine | PIG-A, X-chromosomal |
| Porphyria cutanea tarda | | Overproduction of heme, iron overload | Uroporphyrinogen decarboxylase (UROD), autosomal recessive |
| Severe combined immune deficiency (SCID) | | Due to impaired DNA synthesis severe immune deficiency in humoral and cellular immunity | Adenosine deaminase, autosomal recessive, IL-2R-γ, JAK3, (IL-7R-α, RAG1/2, Artemis, CD3δ, CD3ε |
| Sickle-cell anemia | | Abnormal hemoglobin (HbS) | β-Hemoglobin (HB), autosomal recessive |
| Thalassemia (α- and β form) | | Lack of α- or β hemoglobin resulting in anemia | Deletion of HBA1 and/or HBA2, |
| Von Willebrand disease (three types known, Type-III is most severe) | | Abnormal bleeding, hemorrhage similar to hemophilia A and B | Autosomal dominant and recessive forms |
| | | | |

| | **Cancer** | | |
|---|---|---|---|
| Malignant melanoma | | P16 mutation leads to uncontrolled proliferation of fibroblasts | Cyclie dependant kinase inhibitor 2 (CDKN2) |
| Neurofibromatosis (2 types) | | Benign tumors on auditory nerves leads to deafness | NF1, NF2, autosomal dominant |
| | | | |

| | **Deafness (Ear)** | | |
|---|---|---|---|
| Deafness | | Hearing loss | Deafness-1A (DFNB1), autosomal recessive |
| Pendred syndrome | | Hearing loss | Pendrin (PDS), autosomal recessive |
| | | | |

| | **Heart** | | |
|---|---|---|---|
| Ataxia telangiectasia | | DNA damage repair disturbed, | ATM, |
| Atherosclerosis | | Increase of blood cholesterol | apoE, |
| LQT Syndrome (Long QT) | | Potassium channel defect | LQT1 and other genes |
| Von-Hippel Lindau Syndrome | | Abnormal growth of blood vessels, can lead to cancer | VHL, autosomal dominant |
| William's Beuren Syndrome | | Deletion of elastin results in vascular defects, **supravalvular aortic stenosis** | Deletion of elastin and LIM kinase genes |
| | | | |

| | **Metabolic disorders and glycogen storage diseases** | | |
|---|---|---|---|
| Adrenoleukodystrophy | | Disturbed fatty acid transport and metabolism | ABCD1, X-chromosomal |
| Alkaptonuria | | Nitrogen metabolism defect, Urine turns dark when exposed to oxygen | Homogentisic Oxidase, autosomal recessive |
| Diabetes type I | | Disturbed insulin production | IDDM1, IDDM2, GCK, ... |
| Galactosemia | | disorder of galactose metabolism | Galactose-1-phosphate uridyltransferase gene (GALT), autosomal recessive |
| Gauche disease | | Disturbance of fat metabolism | Glucocerebrosidase |
| Glucose Galactosidase Malabsorption | | Disturbed glucose and galactose transport out of the intestinal lumen resulting in diarrhea | SGLT1, autosomal recessive |
| Glycogen storage disease Type I, Von-Gierke's disease | | Accumulation of glucose in liver and kidney | Glucose-6-Phosphatase, autosomal recessive |
| Glycogen storage disease Type II, Pompe's disease | | Accumulation of glycogen in liver, heart, skeletal muscle, cardiomegaly | α-1-Glucosidase, autosomal recessive |
| Glycogen storage disease Type III, Cori's disease | | Accumulation of glycogen in liver, heart, skeletal muscle, hepatoomeqaly | Debranching enzyme, autosomal recessive |
| Glycogen storage disease Type V, McArdle's disease | | Cannot untilize glycogen in muscle cells | Muscle phosphorylase, autosomal recessive |
| Glucose-6-Phosphate Dehydrogenase | | Inability to maintain glutathione leads to hemolytic anemia | G6PD, X-chromosomal recessive |
| Hereditary Hemochromatosis (4 types) | | Excess of iron in the body (esp. liver) due to excessive iron absorption in the qut | Hemochromatosis (HFE) |
| Homocystinuria | | Nitrogen metabolism defect | Cystathione synthetase defect, autosomal recessive |
| Lesh Nyhan Syndrome | | Accumulation of uric acid leading to gout, ureate stones and muscle loss | HPRT1, X-chromosomal |
| Maple Syrup Urine Disease | | Amino acid metabolism defect leads to the accumulation of α-Ketoacides and death in the first months if untreated | Branched-chain-alpha-dehydrogenase (BCKDH) |
| Menkes' Syndrome | | Reduced ability to absorb copper, leads to death in infancy if untreated | ATP7A, X-chromosomal recessive |
| Obesity | | Elevated body weight | Polygenic, elevated leptin levels may play a role |
| Phenylketonuria | | Inability to break down Phenylalanine into tyrosine leads to mental retardation | Phenylalanine hydroxylase (PAH), autosomal recessive |
| Tangier disease | | reduced levels of plasma high density lipoproteins | ATP-binding cassette-1 gene (ABCA1) |
| Zellweger Syndrome (leads to death in infants) | | High levels of iron and copper in the blood | PXR1 (receptor on the surface of peroxisomes) |
| Wilsons Disease | | Copper accumulation in brain and liver | ATP7B (P-type ATPase), autosomal recessive |
| | | | |

| | **Musculoskeletal system** | | |
|---|---|---|---|
| Achondroplasis | | Short stature with a large head due to slow proliferation of chondrocytes | Fibroblast growth factor receptor 3 (FGF3R), |
| Charcot-Marie-Tooth Syndrome and its more severe form Dejerine-Sottas Syndrome | | Degeneration of the muscles in limbs | Different forms caused by different gene mutations, autosomal recessive and X-chromosomal |
| Cockayne syndrome (2 types) | | Premature aging and short stature, loss of "on the fly" DNA repair | group 8 excision repair cross-complementing protein (ERCC8) |
| Chondroectodermal dysplasia | | Malformation of bones and polydactyly | EVC, autosomal recessive |
| Diastrophic dysplasia (DTD) | | Malformed hands, sulfate transporter defect | DTDST gene |
| Duchenne muscular dystrophy | | Enlargement of muscle tissue with subsequent loss of function | DMD, X-chromosomal recessive |
| Fibrodysplasia Ossificans Progressiva | | Heterotopic bone formation | NOG, BMP, Autosomal dominant |
| Friedreich's ataxia | | Heart enlargement and progressive loss of muscular coordination | Frataxin, autosomal recessive |
| Hypophosphatasia | | Production of an abnormal version of alkaline phosphatase | ALPL, autosomal recessive |
| | | affecting the mineralization process | |
| Marfan Syndrome | | Connective tissue disorder due fibrillin deficiency | Fibrillin 1 (FBN), autosomal dominant |
| Myotonic dystrophy (onset during young adulthood) | | Protein kinase defect in skeletal muscle cells | Dystrophia myotonica protein kinase (DMPK), autosomal dominant |
| Osteogenesis imperfect (various types) | | Defect in type-I collagen formation leads to multiple fractures after birth | COL1A1, COL1A2 |
| Prader-Willi Syndrome | | Decreased muscle tone and mental retardation | SNRPN (small ribinucleoprotein N) deleted due to a deletion on chromosome 15 |
| | | | |

| | **Neurons and Brain** | | |
|---|---|---|---|
| Alzheimer disease | | Increased amyloid production, progressive inability to remember facts | Polygenic, PS1, PS2, ... |
| Amyotrophic lateral sclerosis (ALS) (various forms) | | Progressive degeneration of motor neuron cells (defect in elimination superoxide radicals) | Superoxide dismutase 1 (SOD1), various genes involved |
| Angelman syndrome | | Mental retardation with inadequate laughing | Genomic imprinting on chromosome 15 |
| Pyruvat dehydrogenase | | Neurological defects if untreated | Pyruvat dehydrogenase, autosomal recessive |
| Refsum disease | | Accumulation of phytanic acid leads to peripheral neuropathy | Phytanoyl-CoA hydroxylase (PHYH), autosomal recessive |
| Rett's syndrome | | Mental retardation with arrested development between 6 and 18 months of age | Methyl-CpG-binding protein-2 (MECP2), X-chromosomal dominant |
| Tay-Sachs disease (various forms of severity) | | Disturbed break down of GM2 ganglioside leads to neurological damage | HEXA (β-hexosaminidas A), autosomal recessive |
| LaFora Disease | | Aggressive form of epilepsy | EPM2A, autosomal recessive |
| Essential tremor (variable forms) | | Uncontrollable shaking | ETM1, ETM2, autosomal dominant |
| Fragile X syndrome | | Lack of FMR1 RNA binding protein, mental retardation | FMR1 gene is not expressed due to an CGG amplification in the 5'UTR region |
| Huntington's disease | | Progressive dementia with onset in adulthood | HTT (huntingtin), autosomal dominant |

| | **Intestine** | | |
|---|---|---|---|
| Bartter's syndrome (3 types) | | Renal disease | Kidney chloride channel B gene (CLCNKB), autosomal recessive |
| Polycystic kidney disease (2 types) | | renal disease | PDK1, PDK2, autosomal dominant, there is also a autosomal recessive form known (ARPKD) |
| | | | |

| | **Lung** | | |
|---|---|---|---|
| Alpha-1-antitrypsin | | Defect alveoli due to uncontrolled release of elastase | SERPINA1, autosomal codominant |
| Asthma | | Chronic inflammatory disorder of the airways | Polygenic |
| Cystic fibrosis | | Excessively viscous mucous due to defective Cl⁻ ion transport | CFTR (cystic fibrosis conductance transmembrane regulator), autosomal recessive |
| Surfactant metabolism dysfunction (various types) | | Newborns are of normal body weight, but all fail to inflate | ATP-binding cassette transporter (ABCA3) |
| Primary cliliary dyskinesia | | Excessively viscous mucous due to defective/missing cilia function | DNAI1, CCNO, CCDC40 among others |
| | | | |

| | **Lysosomal storage diseases** | | |
|---|---|---|---|
| Fabry's disease | | Beyond others, skin lesions due to the accumulation of ceramide trihexoside | α-Galactosidase A, X-chromosomal recessive |
| Gaucher's Disease | | Accumulation of glucocerebrosides (gangliosides, sphingolipids) | Glucocerebrosidase, autosomal recessive, |
| Type-I: adult form (normal lifespan under treatment) | | | |
| Type-II: infantile form (death before age 1) | | | |
| Type-III: juvenile form (onset in early childhood, less severe than Type-II) | | | |
| Hunter's Syndrome | | Accumulation of mucopolysaccharides | L-iduronosulfat sulfatase, X-chromosomal recessive |
| Hurler's Syndrome (death by age of 10) | | Accumulation of mucopolysaccharides | α-L-iduronidase, autosomal recessive |
| Niemann-Pick Disease (three distinct forms A, B, C) | | Defect in releasing Cholesterol from lysosomes, accumulation of Sphingomyelin | Sphingomyelinase, autosomal recessive |
| Tay-Sachs disease (death by age of 4) | | Accumulation of G_{M2} ganglioside in neuronal cells | Hexosaminidase A, autosomal recessive |
| | | | |

| | **Skin** | | |
|---|---|---|---|
| Albinism | | Nitrogen metabolism defect | Tyrosinase deficiency, autosomal recessive |
| Albinism, oculocutaneous, type II | | Reduced biosynthesis of melanin pigment | OCA2, autosomal recessive |
| Ehlers-Danlos Syndrome (various types) | | Diaphragmatic hernia. common, retinal detachment | Various defects in collagen synthesis |
| Epidermolysis bullosa (various types including EB simplex, Junctional EB, Dystrophic EB and Kindler syndrome) | | Defects in maintenance of keratinocyte structural stability or adhesion of the keratinocyte to the underlying dermis | Epidermolysis bullosa macular type (EBM), Epidermolysis bullosa 3 progressiva (EBR3), Epidermolysis bullosa 4 pseudojunctual (EBR4), Desmoplakin (DSP), Plakophilin-1 (PKP1), kreatin (KRT5, KRT14), plectin (PLEC), ITGA6, integrin subunit (ITGB4), laminin subunits (LAMA3, LAMP3, LAMB3, LAMC2), collagen (COL17A1, COL7A1 (autosomal dominant), FERMT1, autosomal recessive |
| Hartnup's disease | | Defect in tryptophan uptake in the gastrointestinal tract, light-sensitive skin | SLC6A19, autosomal recessive |
| Hereditary Hemorrhagic Telangiectasia, Osler-Weber-Rendu Syndrome | | Telangiectasia of the skin and mucous membranes | Endoglin (ENG), autosomal dominant |
| Hypercholesterolemia, familial | | elevation of serum cholesterol bound to low density lipoprotein, accumulation in skin and arteriosclerosis | Low-density lipoprotein receptor (LDLR), apolipoprotein B (APOB), autosomal dominant |
| Xeroderma pigmentosa | | skin defect and melanoma due to UV exposure | DNA repair defect, autosomal recessive |
| Male pattern baldness | | Disturbed conversion of testosterone into dihydrotestosterone in the skin | 5-α-reductase |
| | | | |

| | **Genetic liver diseases** | | |
|---|---|---|---|
| Amino acid metabolism disorders | | Disruptions in the multistep process that breaks down the amino acid tyrosine and phenylalanine | FAH, TAT, HPD, autosomal recessive |
| Beta-thalassemia intermedia | | Shortage of mature red blood cells | HBB, autosomal recessive |
| Crigler-Najjar syndrome | | Deficiency in glucuronidation in which bilirubin gets dissolvable in water | UGT1A1, autosomal recessive |
| Fatty acid oxidation disorders | | Deficiency in processing of long-chain fatty acids and very long-chain fatty acids resulting in lethargy and hypoglycemia | HADHA, ACADVL autosomal recessive |
| Fructose metabolism disorders | | Impaired gluconeogenesis causing hypoglycemia | FBP1, ALDOB, autosomal recessive |
| Galactosemia | | Deficiency in processing galactose | GALT, GALK1, GALE, autosomal recessive |
| Glycogen storage diseases | | Disturbed breackdown of glucose 6-phosphate and glycogen leads to accumulation of glycogen as well as abnormal glycogen molecules causing cell damage | G6PC, SLC37A4, AGL, GBE1, autosomal recessive |
| Heme biosynthesis disorder | | Decrease of uroporphyrinogen decarboxylase resulting in accumulation of compounds called porphyrins causing toxic levels in liver | UROD autosomal dominant, ALAS2 X-limked dominant, ALAD autosomal recessive |
| Lipid metabolism (transport) disorders | | Shortage of functional protein, which prevents movement of cholesterol and other lipids, leading to their accumulation in cells | NPC1, NPC2 autosomal recessive, LDLR, autosomal dominant |
| Metal metabolism disorders | | Disorders in the storage and transport of iron and copper resulting in accumulation in tissues and organs | ATP7B, HAMP, HFE, HFE2, autosomal recessive |
| Organic acid disorders (Acidurias/Acidemias) | | Disrupted break down of several protein building blocks (amino acids), certain lipids, and cholesterol | BCKDHA, BCKDHB, and DBT, PCCA and PCCB, MUT, MMAA, MMAB, MMADHC, MCEE, IVD, |
| | | MCCC1 or MCCC2, autosomal recessive | |
| Primary hyperoxaluria type 1 | Disrupted breakdown of glyoxylate leading to renal damage | AGXT, GRHPR, autosomal recessive | |
| Progressive familial intrahepatic cholestasis | Buildup of bile acids in liver cells causing liver damage | ATP8B1, autosomal recessive | |
| Thrombocyte activity disorder | Lack of enzyme activity disrupts the usual balance between bleeding and clotting | ADAMTS13, autosomal recessive | |
| Urea cycle disorders | Disorder of the urea cycle which causes a form of hyperammonemia | OTC (X-linked disorder), CPS1, ASS1 and SLC25A13, ASL, autosomal recessive | |

The above **Table A** shows examples of genes in which a defect leads to a disease which can be treated with the RNA, preferably the mRNA, which may be present in the suspension formulation and the aerosol of the present invention wherein RNA, preferably the mRNA, comprises a ribonucleotide sequence which encodes an intact version of the protein or a functional fragment thereof of the above disclosed defective gene. In particularly preferred embodiments, hereditary diseases can be addressed, which for example affect the lungs, such as SPB (surfactant protein B) deficiency, ABCA3 deficiency, cystic fibrosis and α1-antitrypsin deficiency, or which affect plasma proteins (e.g. congenital hemochromatosis (hepcidin deficiency), thrombotic thrombocytopenic purpura (TPP, ADAMTS 13 deficiency) and cause clotting defects (e.g. haemophilia a and b) and complement defects (e.g. protein C deficiency), immune defects such as for example SCID (caused my mutations in different genes such as: RAG1, RAG2, JAK3, IL7R, CD45, CD3δ, CD3ε) or by deficiencies due to lack of adenosine desaminase for example (ADA-SCID), septic granulomatosis (e.g. caused by mutations of the gp-91-phox gene, the p47-phox gene, the p67-phox gene or the p33-phox gene) and storage diseases like Gaucher's disease, Fabry's disease, Krabbe's disease, MPS I, MPS II (Hunter syndrome), MPS VI, Glycogen storage disease type II or mucopolysaccharidoses.

Other disorders for which the RNA, preferably the mRNA, of the present invention can be useful include disorders such as SMN1-related spinal muscular atrophy (SMA); amyotrophic lateral sclerosis (ALS); GALT-related galactosemia; Cystic Fibrosis (CF); SLC3A1-related disorders including cystinuria; COL4A5-related disorders including Alport syndrome; galactocerebrosidase deficiencies; X-linked adrenoleukodystrophy and adrenomyeloneuropathy; Friedreich's ataxia; Pelizaeus-Merzbacher disease; TSC1 and TSC2-related tuberous sclerosis; Sanfilippo B syndrome (MPS IIIB); CTNS-related cystinosis; the FMR1-related disorders which include Fragile X syndrome, Fragile X-Associated Tremor/Ataxia Syndrome and Fragile X Premature Ovarian Failure Syndrome; Prader-Willi syndrome; hereditary hemorrhagic telangiectasia (AT); Niemann-Pick disease Type C1; the neuronal ceroid lipofuscinoses-related diseases including Juvenile Neuronal Ceroid Lipofuscinosis (JNCL), Juvenile Batten disease, Santavuori-Haltia disease, Jansky-Bielschowsky disease, and PTT-1 and TPP1 deficiencies; EIF2B1, EIF2B2, EIF2B3, EIF2B4 and EIF2B5-related childhood ataxia with central nervous system hypomyelination/vanishing white matter; CACNA1A and CACNB4-related Episodic Ataxia Type 2; the MECP2-related disorders including Classic Rett Syndrome, MECP2-related Severe Neonatal Encephalopathy and PPM-X Syndrome; CDKL5-related Atypical Rett Syndrome; Kennedy's disease (SBMA); Notch-3 related cerebral autosomal dominant arteriopathy with subcortical infarcts and leukoencephalopathy (CADASIL); SCN1A and SCN1B-related seizure disorders; the Polymerase G-related disorders which include Alpers-Huttenlocher syndrome, POLG-related sensory ataxic neuropathy, dysarthria, and ophthalmoparesis, and autosomal dominant and recessive progressive external ophthalmoplegia with mitochondrial DNA deletions; X-Linked adrenal hypoplasia; X-linked agammaglobulinemia; Fabry disease; and Wilson's disease.

In all these diseases, a protein, e.g. an enzyme, is defective, which can be treated with the RNA, preferably the mRNA, encoding any of the above proteins, which makes the protein encoded by the defective gene or a functional fragment thereof available. Transcript replacement therapies/protein replacement therapies do not affect the underlying genetic defect, but increase the concentration of the protein in which the subject is deficient. As an example, in Pompe's disease, the transcript replacement therapy/enzyme replacement therapy replaces the deficient lysosomal enzyme acid alpha-glucosidase (GAA).

Thus, non-limiting examples of proteins which can be encoded by the mRNA are erythropoietin (EPO), growth hormone (somatotropin, hGH), cystic fibrosis transmembrane conductance regulator (CFTR), growth factors such as GM-SCF, G-CSF, MPS, protein C, hepcidin, ABCA3 and surfactant protein B. Further examples of diseases which can be treated with the RNA according to the invention are hemophilia A/B, Fabry's disease, CGD, ADAMTS13, Hurler's disease, X chromosome-mediated A-γ-globulinemia, adenosine deaminase-related immunodeficiency and respiratory distress syndrome in the newborn, which is linked with SP-B. Particularly preferably, the RNA, preferably the mRNA, according to the invention contains the coding sequence for surfactant protein B (SP-B) or for erythropoietin. Further examples of proteins which can be encoded by the RNA, preferably the mRNA, of the present invention according to the invention are growth factors such as human growth hormone hGH, BMP-2 or angiogenesis factors.

Although the above embodiments are described in the context of the RNA, preferably an mRNA molecule, that may be present in the nanoparticles used in the present invention, the present invention, as mentioned above, is not limited to the use of an RNA, preferably an mRNA but may employ other nucleic acid molecules, such as DNA molecules.

Said DNA molecule may encode the above RNA, preferably the above mRNA and, accordingly, harbour the genetic information for the correspondingly transcribed RNA molecule.

Hence, as regards preferred embodiments the same applies, mutatis mutandis, to the DNA molecule of the present invention as has been set forth above and below in the context of the RNA molecule, preferably the mRNA molecule, that may be present in the nanoparticles used in the present invention.

Alternatively, the RNA, preferably the mRNA, may contain a ribonucleotide sequence which encodes a full-length antibody or a smaller antibody (e.g., both heavy and light chains) which can be used in therapeutic settings to, e.g., confer immunity to a subject. Corresponding antibodies and their therapeutic application(s) are known in the art. The antibody may be encoded by a single mRNA strand or by more than one mRNA strand.

In another embodiment, the RNA, preferably the mRNA may encode a functional monoclonal or polyclonal antibody, which may be useful for targeting and/or inactivating a biological target (e.g., a stimulatory cytokine such as tumor necrosis factor). Similarly, the RNA, preferably the mRNA sequence may encode, for example, functional anti-nephrotic factor antibodies useful for the treatment of membranoproliferative glomerulonephritis type II or acute hemolytic uremic syndrome, or alternatively may encode anti-vascular endothelial growth factor (VEGF) antibodies useful for the treatment of VEGF-mediated diseases, such as cancer.

In another embodiment, the RNA, preferably the mRNA may encode a functional monoclonal or polyclonal antibody, which may be useful for neutralizing or otherwise inhibiting a virus or virus replication.

Alternatively, the RNA, preferably the mRNA, may contain a ribonucleotide sequence which encodes an antigen which preferably can be used in preventive or therapeutic settings.

In another embodiment, the mRNA may encode a protein or proteins that can induce an immune modulation, such as cytokines, including chemokines, interferons (such as interferon lambda), interleukins, lymphokines, and tumour necrosis factors.

In another embodiment, the RNA, preferably the mRNA, may contain a ribonucleotide sequence which encodes a polypeptide or a protein which can be used in genome editing technologies. Genome editing is a type of genetic engineering in which DNA is inserted, deleted or replaced in the genome of an organism using nucleases. These nucleases create site-specific breaks at desired locations in the genome. The induced breaks are repaired by non-homologous end-joining or homologous recombination, resulting in targeted mutations in the genome, thereby "editing" the genome. The breaks may either be single-strand breaks or double-strand breaks (DSBs) while double-strand breaks (DSBs) are preferred. Numerous genome editing systems utilizing different polypeptides or proteins are known in the art, i.e., e.g., the CRISPR-Cas system, meganucleases, zinc finger nucleases (ZFNs) and transcription activator-like effector-based nucleases (TALEN). Methods for genome engineering are reviewed in Trends in Biotechnology, 2013, 31 (7), 397-405.

Thus, in a preferred embodiment, the RNA, preferably the mRNA, may contain a ribonucleotide sequence which encodes a polypeptide or protein of the Cas (CRISPR associated protein) protein family, preferably Cas9 (CRISPR associated protein 9). Proteins of the Cas protein family, preferably Cas9, may be used in CRISPR/Cas9 based methods and/or CRISPR/Cas9 genome editing technologies. CRISPR-Cas systems for genome editing, regulation and targeting are reviewed in Nat. Biotechnol., 2014, 32(4):347-355.

In another preferred embodiment, the RNA, preferably the mRNA, may contain a ribonucleotide sequence which encodes a meganuclease. Meganucleases are endodeoxyribonucleases which, in contrast to "conventional" endodeoxyribonucleases, recognize a large recognition site (e.g., a double-stranded DNA sequence of 12 to 40 base pairs). As a result, the respective site occurs only a few times, preferably only once, in any given genome. Meganucleases are therefore considered to be the most specific naturally occurring restriction enzymes and, accordingly, are suitable tools in genome editing technologies.

In another preferred embodiment, the RNA, preferably the mRNA, contains a ribonucleotide sequence which encodes a zinc finger nuclease (ZFN). ZFNs are artificial restriction enzymes generated by fusing a zinc finger DNA-binding domain to a DNA-cleavage domain. Zinc finger domains can be engineered to target specific desired DNA sequences and this enables zinc-finger nucleases to target unique sequences within complex genomes. By taking advantage of the endogenous DNA repair machinery, ZFNs can be used to precisely alter the genome of higher organisms and are, therefore, suitable tools in genome editing technologies.

In another preferred embodiment, the RNA, preferably the mRNA, may contain a ribonucleotide sequence which encodes a transcription activator-like effector nuclease (TALEN). TALENs are restriction enzymes that can be engineered to cut specific sequences of DNA. TALENs are fusion proteins wherein a TAL effector DNA-binding domain is fused to a DNA cleavage domain of a nuclease. Transcription activator-like effectors (TALEs) can be engineered to bind practically any desired DNA sequence. Thus, when combined with a nuclease, DNA can be cut at specific desired locations.

Although the above embodiments are described in the context of the RNA, preferably an mRNA molecule, the present invention, as mentioned above, is not only limited to the use of an RNA, preferably an mRNA, but may employ any nucleic acid molecule, such as a DNA molecule.

Said DNA molecule may encode the above RNA, preferably the above mRNA and, accordingly, harbour the genetic information for the correspondingly transcribed RNA molecule.

Hence, as regards preferred embodiments the same applies, mutatis mutandis, to the DNA molecule as has been set forth above and below in the context of the RNA molecule, preferably the mRNA molecule, that may be present in the nanoparticles used in the present invention.

Alternatively to the above, the RNA contains a ribonucleotide sequence which is not to be expressed as a protein or a polypeptide. Thus, the term RNA should not only be understood to mean any polynucleotide molecule which, if introduced into a cell, is translatable to a polypeptide/protein or fragment thereof. Rather, it is also contemplated that the RNA contains a ribonucleotide sequence which is not translated into a protein. In this context, it is envisaged that the RNA contains a ribonucleotide sequence which preferably provides the genetic information for an antisense RNA, an siRNA or a miRNA sequence or another desired non-coding ribonucleotide sequence.

Thus, the RNA may also be an antisense RNA, an siRNA or a miRNA sequence. Antisense RNA, siRNA or miRNA sequences can be used to silence the effect of a certain RNA molecule at some stage. This may, in particular, be desirable and useful in certain medical settings and in the treatment of a certain disease and, in particular, in RNA-based therapies as described herein above and below.

Silencing the effect of an RNA molecule can be achieved by making use of an RNAi (RNA interference) mechanism by using the nucleic acid strand which is complementary to a certain RNA sequence. The term "RNA interference" or "inhibiting RNA" (RNAi/iRNA) describes the use of double-stranded RNA to target specific mRNAs for degradation, thereby silencing their translation. Preferred inhibiting RNA molecules may be selected from the group consisting of double-stranded RNA (dsRNA), siRNA, shRNA and stRNA. dsRNA matching a gene sequence may be synthesized in vitro and introduced into a cell. The dsRNA may also be introduced into a cell in form of a vector expressing a target gene sequence in sense and antisense orientation, for example in form of a hairpin mRNA. The sense and antisense sequences may also be expressed from separate vectors, whereby the individual antisense and sense molecules form double-stranded RNA upon their expression. It is known in the art that in some occasions the expression of a sequence in sense orientation or even of a promoter sequence suffices to give rise to dsRNA and subsequently to siRNA due to internal amplification mechanisms in a cell. Accordingly, all means and methods which result in a decrease in activity of the polypeptide or protein encoded by the coding region are to be used in accordance with the present invention. For example sense constructs, antisense constructs, hairpin constructs, sense and antisense molecules and combinations thereof can be used to generate/introduce these siRNAs. The dsRNA feeds into a natural process including the highly conserved nuclease dicer which cleaves dsRNA precursor molecules into short interfering RNAs (siRNAs). The generation and preparation of siRNA(s) as well as the method for inhibiting the expression of a target gene is, inter alia, described in WO 02/055693, Wei (2000) Dev. Biol. 15:239-255; La Count (2000) Biochem. Paras. 111:67-76; Baker (2000) Curr. Biol. 10:1071-1074; Svoboda (2000) Development 127:4147-4156 or Marie (2000) Curr. Biol. 10:289-292. These siRNAs build then the sequence specific part of an RNA-induced silencing complex (RISC), a multicomplex nuclease that destroys messenger RNAs homologous to the silencing trigger). Elbashir (2001) EMBO J. 20:6877-6888 showed that duplexes of 21 nucleotide RNAs may be used in cell culture to interfere with gene expression in mammalian cells.

Methods to deduce and construct siRNAs are known in the art and are described in Elbashir (2002) Methods 26:199-213, at the internet web sites of commercial vendors of siRNA, e.g. Qiagen GmbH (https://www1.qiagen.com/GeneGlobe/Default.aspx); Dharmacon (www.dharmacon.com); Xeragon Inc. (http://www.dharmacon.com/Default.aspx), and Ambion (www.ambion.com), or at the web site of the research group of Tom Tuschl (http://www.rockefeller.edu/labheads/tuschl/sirna.html). In addition, programs are available online to deduce siRNAs from a given mRNA sequence (e.g. http://www.ambion.com/techlib/misc/siRNA_finder.html or http://katahdin.cshl.org:9331/RNAi/html/rnai.html). Uridine residues in the 2-nt 3' overhang can be replaced by 2'deoxythymidine without loss of activity, which significantly reduces costs of RNA synthesis and may also enhance resistance of siRNA duplexes when applied to mammalian cells (Elbashir (2001) loc. cit). The siRNAs may also be sythesized enzymatically using T7 or other RNA polymerases (Donze (2002) Nucleic Acids Res 30:e46). Short RNA duplexes that mediate effective RNA interference (esiRNA) may also be produced by hydrolysis with Escherichia coli RNase III (Yang (2002) PNAS 99:9942-9947). Furthermore, expression vectors have been developed to express double stranded siRNAs connected by small hairpin RNA loops in eukaryotic cells (e.g. (Brummelkamp (2002) Science 296:550-553). All of these constructs may be developed with the help of the programs named above. In addition, commercially available sequence prediction tools incorporated in sequence analysis programs or sold separately, e.g. the siRNA Design Tool offered by www.oligoEngine.com (Seattle,WA) may be used for siRNA sequence prediction.

microRNA (miRNA) resembles small interfering RNAs (siRNAs) described above. microRNA (miRNA) is a small non-coding RNA molecule (containing about 22 nucleotides) found in plants, animals and some viruses, that functions in RNA silencing and post-transcriptional regulation of gene expression. miRNAs function via base-pairing with complementary sequences within mRNA molecules. As a result, these mRNA molecules are silenced, by one or more of the following processes: (1) cleavage of the mRNA strand into two pieces, (2) destabilization of the mRNA through shortening of its poly(A) tail, and (3) less efficient translation of the mRNA into proteins by ribosomes. As mentioned, miRNAs resemble the small interfering RNAs (siRNAs) of the RNA interference (RNAi) pathway, except miRNAs derive from regions of RNA transcripts that fold back on themselves to form short hairpins, whereas siRNAs derive from longer regions of double-stranded RNA.

A DNA molecule used in the suspension formulations and the aerosol of the present invention may also be one which encodes the above RNA, e.g. the above siRNA or miRNA, accordingly, harbours the genetic information for the correspondingly transcribed RNA molecule. Hence, as regards preferred embodiments the same applies, mutatis mutandis, to the DNA molecule as has been set forth above in the context of the RNA molecule, preferably the mRNA molecule, that may be present in the nanoparticles used in the present invention.

It will be understood that the nanoparticles in the context of the present invention can comprise a single type of nucleic acid, preferably an RNA such as mRNA, but may alternatively comprise a combination of two or more types of nucleic acids, preferably RNAs, e.g. in the form of particles comprising two or more types of nucleic acids, preferably RNAs, in single particles, or in the form of a blend of particles which differ in the type of nucleic acid, preferably RNA such as mRNA, contained therein.

Together with the therapeutic agent (a), the nanoparticles further comprise, as component (b), a permanently cationic lipid, an ionizable lipid or an ionizable lipidoid, i.e. component (b) is at least one selected from a permanently cationic lipid, an ionizable lipid and an ionizable lipidoid. It will be understood that this encompasses the possibility that the nanoparticles comprise a combination of different permanently cationic lipids, a combination of different ionizable lipids, a combination of different ionizable lipidoids, or a combination of one or more permanently cationic lipids, one or more ionizable lipids, and/or one or more ionizable lipidoids. Preferred as component (b) are an ionizable lipid and an ionizable lipidoid, i.e. it is preferred that the nanoparticles comprise at least one selected from an ionizable lipid and an ionizable lipidoid as component (b). The nanoparticles used in the context of the present invention typically comprise the nucleic acid (a) and the permanently cationic lipid, the ionizable lipid or the ionizable lipidoid (b) in the form of a mixture of components (a) and (b).

The term "permanently cationic lipid" is used in the field of lipid nanoparticles to refer to a lipid which contains a permanent cationic charge, e.g. in the form of a quaternary nitrogen atom.

The terms "ionizable lipid" and "ionizable lipidoid", are used in the field of lipid nanoparticles and lipidoid nanoparticles to refer to a lipid or a lipidoid which is protonated to carry a cationic charge, or which can be protonated to carry a cationic charge. Thus, ionizable lipids and lipidoids, respectively, are also referred to as "protonatable lipids" and "protonatable lipidoids", as "ionizable cationic lipids" and "ionizable cationic lipidoids", or as "titratable lipids" or "titratable lipidoids", respectively. As will be understood by the skilled reader, the reference to an "ionizable lipid" or an "ionizable lipidoid" encompasses the ionizable lipid or lipidoid in its protonated or non-protonated form. As will further be understood, the protonated or non-protonated state of the lipid or lipidoid is generally determined by the pH value of a medium surrounding the lipid or lipidoid, e.g. by the pH value of the aqueous vehicle solution wherein the nanoparticles are suspended. Thus, the terms "ionizable lipid" and "ionizable lipidoid" also include lipids or lipidoids which are positively charged at neutral pH.

Counterions (anions) for the positive charges of positively charged permanently cationic lipids ionizable lipids or ionizable lipidoids in the context of the invention are typically provided by anionic moieties contained in the nucleic acid. If positively charged groups are present in excess compared to the anionic moieties in the nucleic acid, positive charges may be balanced by other pharmaceutically acceptable anions, such as chloride, bromide, or iodide, sulfate, nitrate, phosphate, hydrogenphosphate, dihydrogenphosphate, carbonate, or hydrogencarbonate, or by a polyanion component different from the nucleic acid, which may be present as an optional component in the nanoparticles.

Permanently cationic lipids, ionizable lipids and ionizable lipidoids are well known as components of lipid nanoparticles or lipidoid nanoparticles. In the context of the present invention, there are no particular restrictions imposed on the type of permanently cationic lipid, ionizable lipid or ionizable lipidoid contained in the nanoparticles.

Generally, an ionizable lipid or lipidoid, respectively, comprises a primary, secondary or tertiary amino group which can act as proton acceptor and which may thus be protonated or non-protonated. An ionizable lipidoid generally comprises a plurality of such amino groups, such as two or more, preferably three or more.

Preferably, an ionizable lipid which may be comprised by the nanoparticles is a lipid which comprises a protonatable head group which contains one or more, preferably one, primary, secondary or tertiary amino group(s) as a protonatable or protonated group, and one or more, preferably one or two, hydrophobic moieties, linked to the head group.

Examples of these preferred ionizable lipids are
i) a lipid which comprises a protonatable head group which contains one or more, preferably one, primary, secondary or tertiary amino group(s) as a protonatable or protonated group, and one hydrophobic moiety linked to the head group;
ii) a lipid which comprises one secondary or tertiary amino group as a protonatable or protonated head group, and two hydrophobic moieties linked to the head group.

A hydrophobic moiety comprised in these preferred lipids preferably contains one or more of a linear chain aliphatic residue, e.g. a linear chain residue comprising 8 to 18 carbon atoms, a branched chain aliphatic residue, e.g. a branched chain residue comprising 8 to 18 carbon atoms, or an alicyclic ring structure which may be a condensed ring structure, e.g. an alicyclic ring structure comprising 10 to 18 carbon atoms. In addition, the hydrophobic moiety may comprise one or more linking groups which facilitate the linking of the moiety to the head group, or which allow two or more of the above aliphatic residues to be combined with each other. Furthermore, it may comprise one or more substituents, to the extent that the hydrophobic characteristics of the moiety are maintained.

Preferably, an ionizable lipidoid which may be comprised in the nanoparticles is an oligoamine, more preferably an oligoalkylamine, which comprises at least two, preferably at least three, amino groups selected from a protonatable or protonated secondary and a tertiary amino group, each of which may carry a hydrophobic moiety attached to it. In addition to the amino groups carrying a hydrophobic residue, the lipidoid may comprise further protonatable or protonated amino groups selected from a primary, a secondary and a tertiary amino group. Preferably, the total number of the amino groups is 2 to 10, more preferably 3 to 6. Preferably, the total number of hydrophobic moieties attached to the amino groups is 2 to 6, more preferably 3 to 6. Preferably, the ratio of the number of hydrophobic moieties attached to amino groups to the total number of amino groups in the oligoalkylamine is 0.5 to 2, more preferably 0.75 to 1.5.

A hydrophobic moiety comprised in such a preferred lipidoid preferably contains one or more of a linear chain aliphatic residue, e.g. a linear chain residue comprising 8 to 18 carbon atoms and a branched chain aliphatic residue, e.g. a branched chain residue comprising 8 to 18 carbon atoms. In addition, the hydrophobic moiety may comprise one or more linking groups which facilitate the linking of the moiety to an amino group, or which allow two or more of the above aliphatic residues to be combined with each other. Furthermore, it may comprise one or more substituents, to the extent that the hydrophobic characteristics of the moiety are maintained.

Suitable exemplary ionizable lipids or ionizable lipidoids which can be comprised as component (b) in the in the nanoparticles used in the context of the present invention are disclosed, e.g., in WO 2006/138380 A2, EP2476756 A1, US 2016/0114042 A1, US 8,058,069 B2, US 8,492,359 B2, US 8,822,668 B2, US 8,969,535, US 9,006,417 B2, US 9,018,187 B2, US 9,345,780 B2, US 9,352,042 B2, US 9,364,435 B2, US 9,394,234 B2, US 9,492,386 B2, US 9,504,651 B2, US 9,518,272 B2, DE 19834683 A1, WO 2010/053572 A2, US 9,227,917 B2, US 9,556,110 B2, US 8,969,353 B2, US 10,189,802 B2, WO 2012/000104 A1, WO 2010/053572, WO 2014/028487,WO 2015/095351, US 2013/0156849 A1 (e.g. claims 13, 33, 34), US 9254311 B2 (e.g. claim 14), US 10501512 B2 (e.g. claims 1, 6, 9), US 2014/0010861 A1 (e.g. claims 44 and 78-82), US 2013/0115272 A1 (e.g. claim 12) or by Akinc, A., et al., Nature Biotechnology, 26(5), 2008, 561-569; Sabnis, S. et al., Molecular Therapy, 26(6), 2018, Vol. 26 No 6 June 2018, 1509-1519; Kowalski, P.S., et al., Molecular Therapy, 27(4), 2019, 710-728; Kulkarni, J. A. et al, Nucleic Acid Therapeutics, 28(3), 2018, 146-157; and Li, B. et al., Nano Letters, 15, 2015, 8099-8107.

Preferably, a permanently cationic lipid which may be comprised by the nanoparticles is a lipid which comprises a head group containing one quaternary nitrogen atom and one or more, preferably one or two, hydrophobic moieties, linked to the head group. Preferably, the quaternary nitrogen atom is provided by a group of the formula -N(Me)₃⁺, wherein Me is a methyl group.

A hydrophobic moiety comprised in these preferred lipids preferably contains one or more of a linear chain aliphatic residue, e.g. a linear chain residue comprising 8 to 18 carbon atoms, or a branched chain aliphatic residue, e.g. a branched chain residue comprising 8 to 18 carbon atoms, In addition, the hydrophobic moiety may comprise one or more linking groups which facilitate the linking of the moiety to the head group, or which allow two or more of the above aliphatic residues to be combined with each other. Furthermore, it may comprise one or more substituents, to the extent that the hydrophobic characteristics of the moiety are maintained. As examples of a permanently cationic lipid, reference can be made to DOTMA (Dioleoyl-3-trimethylammonium propane and DOTAP (Dioleoyl-3-trimethylammonium propane).

In one preferred embodiment, component (b) of the nanoparticles comprises, or more preferably consists of, an ionizable lipid or lipidoid of Formula a-I: 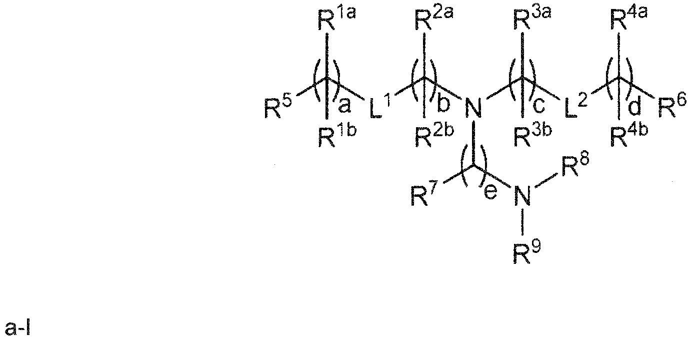 or a pharmaceutically acceptable salt, tautomer, prodrug or stereoisomer thereof, wherein:
one of L¹ or L² is-O(C=O)-, -(C=O)O-, -C(=O)-, -O-, -S(O)ₓ-, -S-S-, - C(=O)S-, SC(=O)-, - NR^{a}C(=O)-, -C(=O)NR^{a}-, NR^{a}C(=O)NR^{a}-, -OC(=O)NR^{a}- or - NR^{a}C(=O)O-, and the other of L¹ or L² is-O(C=O)-, -(C=O)O-, -C(=O)-, -O-, -S(O)ₓ-, -S- S-, -C(=O)S-, SC(=O)-,
-NR^{a}C(=O)-, -C(=O)NR^{a}-, ,NR^{a}C(=O)NR^{a}-, -OC(=O)NR^{a}- or -NR^{a}C(=O)O- or a direct bond;
R^{a} is H or C₁-C₁₂ alkyl;
R^{1a} and R^{1b} are, at each occurrence, independently either (a) H or C₁-C₁₂ alkyl, or (b) R^{1a} is H or C₁-C₁₂ alkyl, and R^{1b} together with the carbon atom to which it is bound is taken together with an adjacent R^{1b} and the carbon atom to which it is bound to form a carbon-carbon double bond;
R^{2a} and R^{2b} are, at each occurrence, independently either (a) H or C₁-C₁₂ alkyl, or (b) R^{2a} is H or C₁-C₁₂ alkyl, and R^{2b} together with the carbon atom to which it is bound is taken together with an adjacent R^{2b} and the carbon atom to which it is bound to form a carbon-carbon double bond;
R^{3a} and R^{3b} are, at each occurrence, independently either (a) H or C₁-C₁₂ alkyl, or (b) R^{3a} is H or C₁-C₁₂ alkyl, and R^{3b} together with the carbon atom to which it is bound is taken together with an adjacent R^{3b} and the carbon atom to which it is bound to form a carbon-carbon double bond;
R^{4a} and R^{4b} are, at each occurrence, independently either (a) H or C₁-C₁₂ alkyl, or (b) R^{4a} is H or C₁-C₁₂ alkyl, and R^{4b} together with the carbon atom to which it is bound is taken together with an adjacent R^{4b} and the carbon atom to which it is bound to form a carbon-carbon double bond;
R⁵ and R⁶ are each independently methyl or cycloalkyl;
R⁷ is, at each occurrence, independently H or C₁-C₁₂ alkyl;
R⁸ and R⁹ are each independently unsubstituted C₁-C₁₂ alkyl; or R⁸ and R⁹, together with the nitrogen atom to which they are attached, form a 5, 6 or 7-membered heterocyclic ring comprising one nitrogen atom; a and d are each independently an integer from 0 to 24;
b and c are each independently an integer from 1 to 24;
e is 1 or 2; and
x is 0, 1 or 2.

In some embodiments, the ionizable lipid has a structure of Formula a-II: 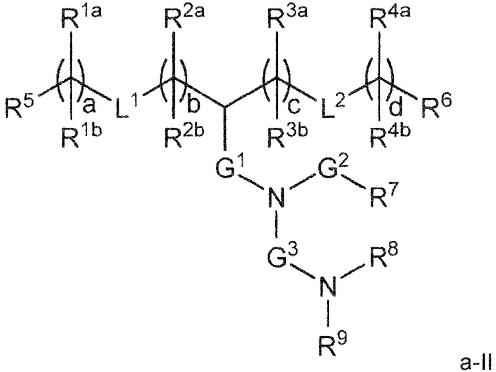 or a pharmaceutically acceptable salt, tautomer, prodrug or stereoisomer thereof, wherein:
one of L¹ or L² is-O(C=O)-, -(C=O)O-, -C(=O)-, -O-, -S(O)ₓ₋, -S-S-, - C(=O)S-, SC(=O)-, - NR^{a}C(=O)-, -C(=O)NR^{a}-, NR^{a}C(=O)NR^{a}-, -OC(=O)NR^{a}- or - NR^{a}C(=O)O-, and the other of L¹ or L² is-O(C=O)-, -(C=O)O-, -C(=O)-, -O-, -S(O)ₓ₋, -S- S-, -C(=O)S-, SC(=O)-,
-NR^{a}C(=O)-, -C(=O)NR^{a}-, ,NR^{a}C(=O)NR^{a}-, -OC(=O)NR^{a}- or -NR^{a}C(=O)O- or a direct bond;
G¹ is C₁-C₂ alkylene, -(C=O)-, -O(C=O)-, -SC(=O)-, -NR^{a}C(=O)- or a direct bond;
G² is-C(=O)-, -(C=O)O-, -C(=O)S-, -C(=O)NR^{a}- or a direct bond;
G³ is C₁-C₆ alkylene;
R^{a} is H or C₁-C₁₂ alkyl;
R^{1a} and R^{1b} are, at each occurrence, independently either: (a) H or C₁-C₁₂ alkyl; or (b) R^{1a} is H or C₁-C₁₂ alkyl, and R^{1b} together with the carbon atom to which it is bound is taken together with an adjacent R^{1b} and the carbon atom to which it is bound to form a carbon-carbon double bond;
R^{2a} and R^{2b} are, at each occurrence, independently either: (a) H or C₁-C₁₂ alkyl; or (b) R^{2a} is H or C₁-C₁₂ alkyl, and R^{2b} together with the carbon atom to which it is bound is taken together with an adjacent R^{2b} and the carbon atom to which it is bound to form a carbon-carbon double bond;
R^{3a} and R^{3b} are, at each occurrence, independently either (a): H or C₁-C₁₂ alkyl; or (b) R^{3a} is H or C₁-C₁₂ alkyl, and R^{3b} together with the carbon atom to which it is bound is taken together with an adjacent R^{3b} and the carbon atom to which it is bound to form a carbon-carbon double bond;
R^{4a} and R^{4b} are, at each occurrence, independently either: (a) H or C₁-C₁₂ alkyl; or (b) R^{4a} is H or C₁-C₁₂ alkyl, and R^{4b} together with the carbon atom to which it is bound is taken together with an adjacent R^{4b} and the carbon atom to which it is bound to form a carbon-carbon double bond;
R⁵ and R⁶ are each independently H or methyl;
R⁷ is C₄-C₂₀ alkyl;
R⁸ and R⁹ are each independently C₁-C₁₂ alkyl; or R⁸ and R⁹, together with the nitrogen atom to which they are attached, form a 5, 6 or 7-membered heterocyclic ring;
a, b, c and d are each independently an integer from 1 to 24; and
x is 0, 1 or 2.

In some embodiments, the ionizable lipid has a structure of Formula a-III: 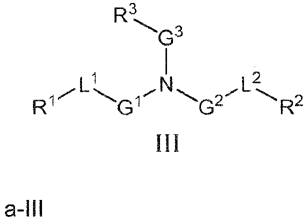 or a pharmaceutically acceptable salt, prodrug or stereoisomer thereof, wherein:
one of L¹ or L² is-O(C=O)-, -(C=O)O-, -C(=O)-, -O-, -S(O)ₓ₋, -S-S-, - C(=O)S-, SC(=O)-, - NR^{a}C(=O)-, -C(=O)NR^{a}-, NR^{a}C(=O)NR^{a}-, -OC(=O)NR^{a}- or - NR^{a}C(=O)O-, and the other of L¹ or L² is-O(C=O)-, -(C=O)O-, -C(=O)-, -O-, -S(O)ₓ₋, -S- S-, -C(=O)S-, SC(=O)-,
-NR^{a}C(=O)-, -C(=O)NR^{a}-, ,NR^{a}C(=O)NR^{a}-, -OC(=O)NR^{a}- or -NR^{a}C(⁼O)O- or a direct bond;
G¹ and G² are each independently unsubstituted C₁-C₁₂ alkylene or C₁-C₁₂ alkenylene;
G³ is C₁-C₂₄ alkylene, C₁-C₂₄ alkenylene, C₃-C₈ cycloalkylene, C₃-C₈ cycloalkenylene;
R^{a} is H or C₁-C₁₂ alkyl;
R¹ and R² are each independently C₆-C₂₄ alkyl or C₆-C₂₄ alkenyl;
R³ is H, OR⁵, CN, -C(=O)OR⁴, -OC(=O)R⁴ or -NR⁵C(=O)R⁴; R⁴ is C₁-C₁₂ alkyl;
R⁵ is H or C₁-C₆ alkyl; and
x is 0, 1 or 2.

In some embodiments, the ionizable lipid has the following Formula IV : 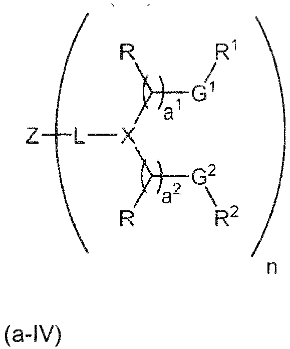 or a pharmaceutically acceptable salt, prodrug or stereoisomer thereof, wherein:
one of G¹ or G² is, at each occurrence, -O(C=O)-, -(C=O)O-, -C(=O)-, -O-, -S(O)_{y}, -S-S-, -C(=O)S-, SC(=O)-, -N(R^{a})C(=O)-, -C(=O)N(R^{a})- , -N(R^{a})C(=O)N(R^{a})-,
-OC(=O)N(R^{a})- or -N(R^{a})C(=O)O-, and the other of G¹ or G² is, at each occurrence, -O(C=O)-, -(C=O)O-, -C(=O)-, -O-, -S(O)_{y}-, -S-S-, -C(=O)S-, -SC(=O)-, -N(R^{a})C(=O)-, -C(=O)N(R^{a})-, -N(R^{a})C(=O)N(R^{a})-, -OC(=O)N(R^{a})- or
-N(R^{a})C(=O)O- or a direct bond;
L is, at each occurrence, ~O(C=O)-, wherein ~ represents a covalent bond to X;
X is CR^{a};
Z is alkyl, cycloalkyl or a monovalent moiety comprising at least one polar functional group when n is 1; or Z is alkylene, cycloalkylene or a polyvalent moiety comprising at least one polar functional group when n is greater than 1;
R^{a} is, at each occurrence, independently H, C₁-C₁₂ alkyl, C₁-C₁₂ hydroxylalkyl, C₁-C₁₂ aminoalkyl, C₁-C₁₂ alkylaminylalkyl, C₁-C₁₂ alkoxyalkyl, C₁-C₁₂ alkoxycarbonyl, C₁-C₁₂ alkylcarbonyloxy, C₁-C₁₂ alkylcarbonyloxyalkyl or C₁-C₁₂ alkylcarbonyl;
R is, at each occurrence, independently either: (a) H or C₁-C₁₂ alkyl; or (b) R together with the carbon atom to which it is bound is taken together with an adjacent R and the carbon atom to which it is bound to form a carbon-carbon double bond;
R¹ and R² have at each occurrence the following structure, respectively: 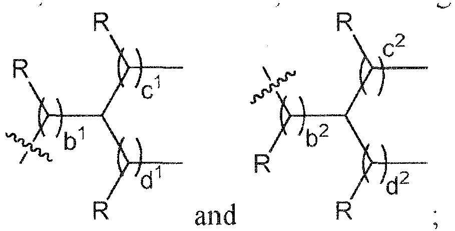
R¹ R² a¹ and a² are, at each occurrence, independently an integer from 3 to 12; b¹ and b² are, at each occurrence, independently 0 or 1;
c¹ and c² are, at each occurrence, independently an integer from 5 to 10; d¹ and d² are, at each occurrence, independently an integer from 5 to 10; y is, at each occurrence, independently an integer from 0 to 2; and n is an integer from 1 to 6,
wherein each alkyl, alkylene, hydroxylalkyl, aminoalkyl, alkylaminylalkyl, alkoxyalkyl, alkoxycarbonyl, alkylcarbonyloxy, alkylcarbonyloxyalkyl and alkylcarbonyl is optionally substituted with one or more substituents.

In some embodiments, the ionizable lipid has the following Formula (a-V): 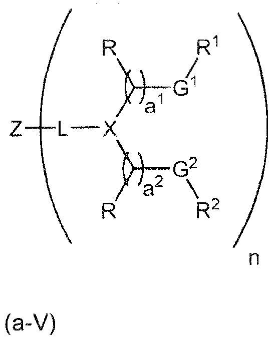 or a pharmaceutically acceptable salt, prodrug or stereoisomer thereof, wherein:
one of G¹ or G² is, at each occurrence, -O(C=O)-, -(C=O)O-, -C(=O)-, -O-, -S(O)_{y}-, -S-S-, -C(=O)S-, SC(=O)-, -N(R^{a})C(=O)-, -C(=O)N(R^{a})- , -N(R^{a})C(=O)N(R^{a})-,
-OC(=O)N(R^{a})- or -N(R^{a})C(=O)O-, and the other of G¹ or G² is, at each occurrence, -O(C=O)-,
-(C=O)O-, -C(=O)-, -O-, -S(O)_{y}-, -S-S-, -C(=O)S-, -SC(=O)-, -N(R^{a})C(=O)-, - C(=O)N(R^{a})-, -N(R^{a})C(=O)N(R^{a})-, -OC(=O)N(R^{a})- or
-N(R^{a})C(=O)O- or a direct bond;
L is, at each occurrence, ~O(C=O)-, wherein ~ represents a covalent bond to X;
X is CRa;
Z is alkyl, cycloalkyl or a monovalent moiety comprising at least one polar functional group when n is 1; or Z is alkylene, cycloalkylene or a polyvalent moiety comprising at least one polar functional group when n is greater than 1;
R^{a} is, at each occurrence, independently H, C₁-C₁₂ alkyl, C₁-C₁₂ hydroxylalkyl, C₁-C₁₂ aminoalkyl, C₁-C₁₂ alkylaminylalkyl, C₁-C₁₂ alkoxyalkyl, C₁-C₁₂ alkoxycarbonyl, C₁-C₁₂ alkylcarbonyloxy, C₁-C₁₂ alkylcarbonyloxyalkyl or C₁-C₁₂ alkylcarbonyl;
R is, at each occurrence, independently either: (a) H or C₁-C₁₂ alkyl; or (b) R together with the carbon atom to which it is bound is taken together with an adjacent R and the carbon atom to which it is bound to form a carbon-carbon double bond;
R¹ and R² have at each occurrence the following structure, respectively: 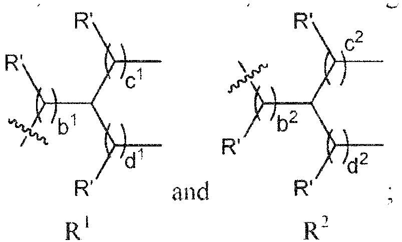
R' is, at each occurrence, independently H or C₁-C₁₂ alkyl; a¹ and a² are, at each occurrence, independently an integer from 3 to 12; b¹ and b² are, at each occurrence, independently 0 or 1;
c¹ and c² are, at each occurrence, independently an integer from 2 to 12; d¹ and d² are, at each occurrence, independently an integer from 2 to 12; y is, at each occurrence, independently an integer from 0 to 2; and n is an integer from 1 to 6,
wherein a', a², c¹, c², d¹ and d² are selected such that the sum of a¹+c¹+d¹ is an integer from 18 to 30, and the sum of a²+c²+d² is an integer from 18 to 30, and wherein each alkyl, alkylene, hydroxylalkyl, aminoalkyl, alkylaminylalkyl, alkoxyalkyl, alkoxycarbonyl, alkylcarbonyloxy, alkylcarbonyloxyalkyl and alkylcarbonyl is optionally substituted with one or more substituent.

In some embodiments, the ionizable lipid is selected from a lipid in Table 1, Table 2, Table 3 or Table 4.

**Table 1:**

| **No.** | **Structure** | **Prep. Method** | **pKa** |
|---|---|---|---|
| I-1 | 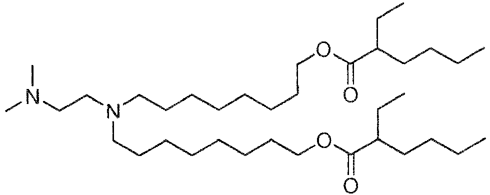 | B | - |
| I-2 | 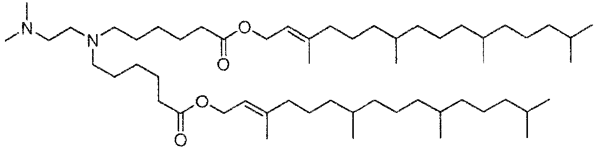 | A | 5.64 |
| I-3 | 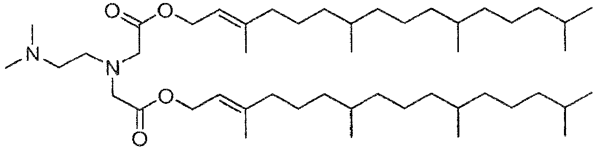 | A | 7.15 |
| I-4 | 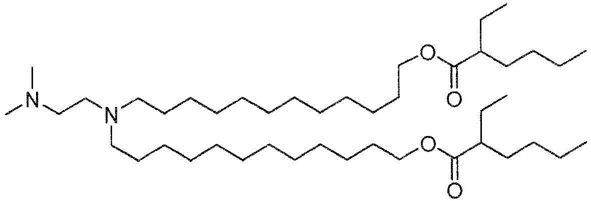 | B | 6.43 |
| I-5 | 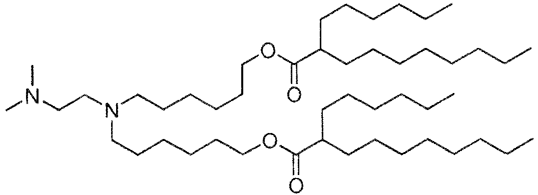 | B | 6.28 |
| I-6 | 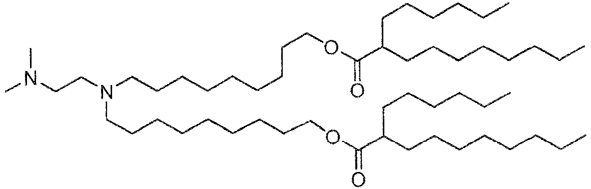 | B | 6.12 |
| I-7 | 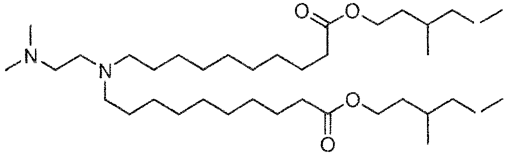 | A | - |
| I-8 | 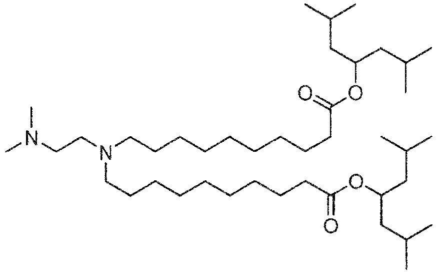 | A | - |
| I-9 | 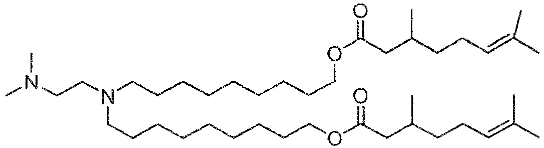 | B | - |
| I-10 | 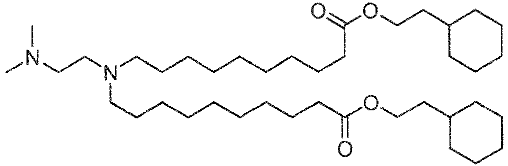 | A | - |
| I-11 | 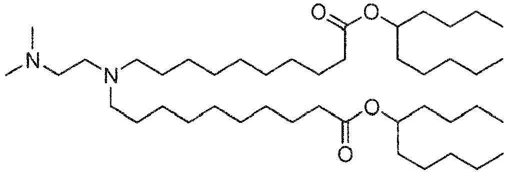 | A | 6.36 |
| I-12 | 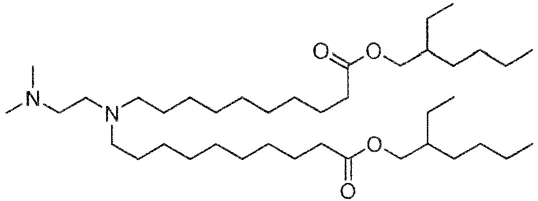 | A | - |
| I-13 | 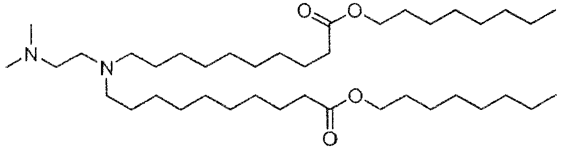 | A | 6.51 |
| I-14 | 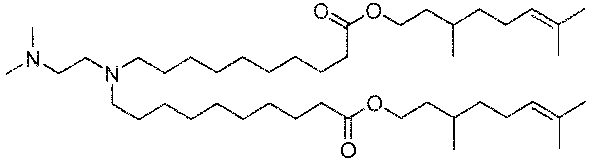 | A | - |
| I-15 | 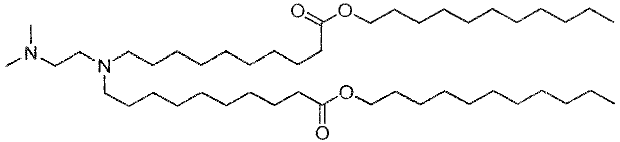 | A | 6.30 |
| I-16 | 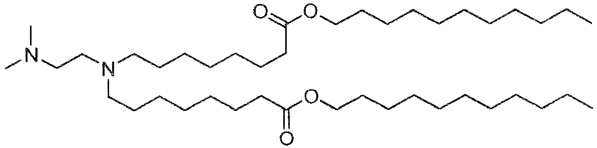 | A | 6.63 |
| I-17 | 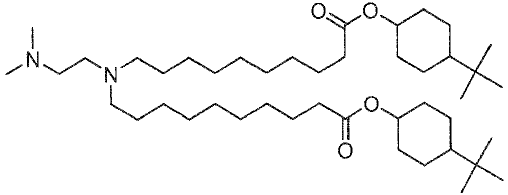 | A | - |
| I-18 | 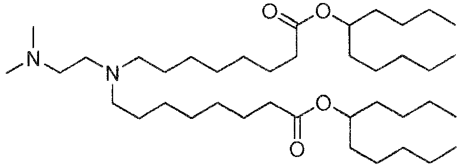 | A | - |
| I-19 | 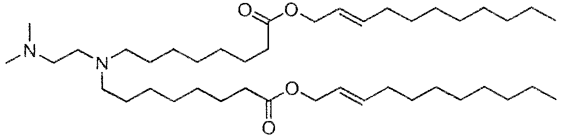 | A | 6.72 |

| **No.** | **Structure** | **Prep. Method** | **pka** |
|---|---|---|---|
| I-20 | 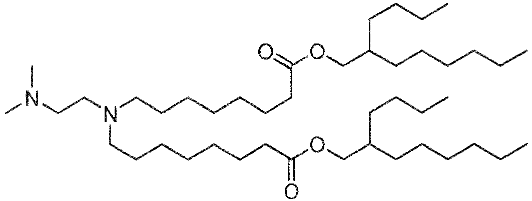 | A | 6.44 |
| I-21 | 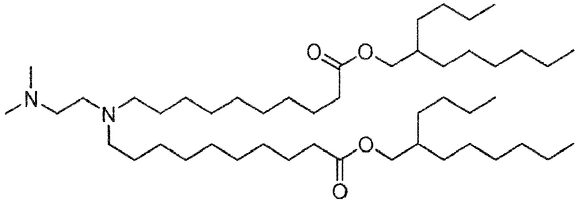 | A | 6.28 |
| I-22 | 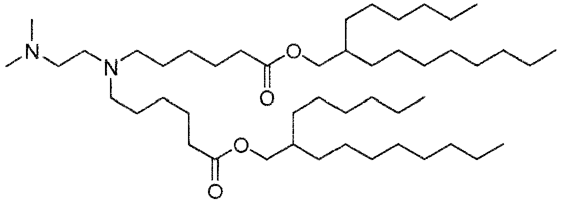 | A | 6.53 |
| I-23 | 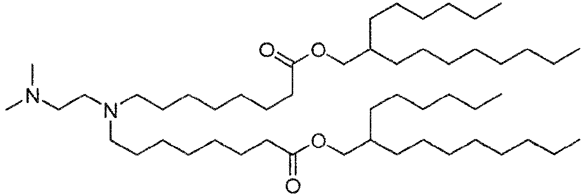 | A | 6.24 |
| I-24 | 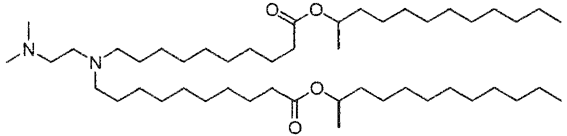 | A | 6.28 |
| I-25 | 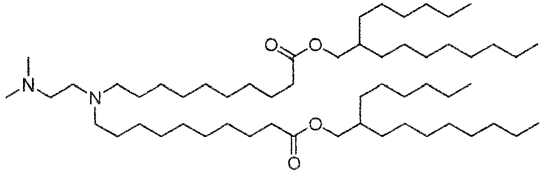 | A | 6.20 |
| I-26 | 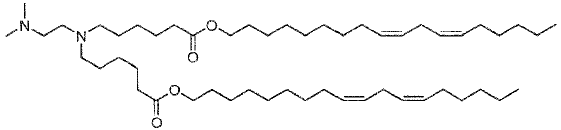 | A | 6.89 |

| **No.** | **Structure** | **Prep. Method** | **pKa** |
|---|---|---|---|
| I-27 | 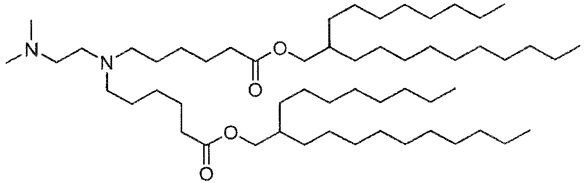 | A | 6.30 |
| I-28 | 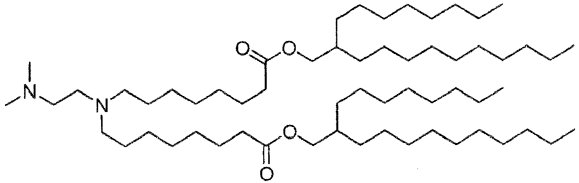 | A | 6.20 |
| I-29 | 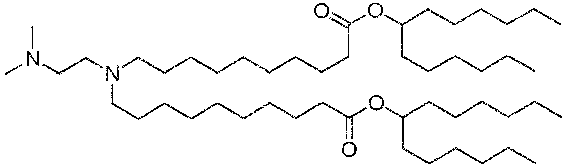 | A | 6.22 |
| I-30 | 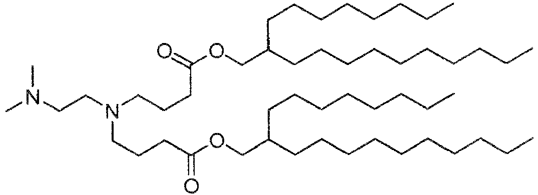 | A | - |
| I-31 | 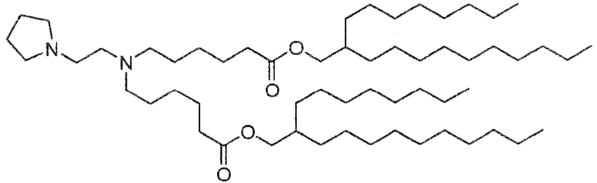 | C | 6.33 |
| I-32 | 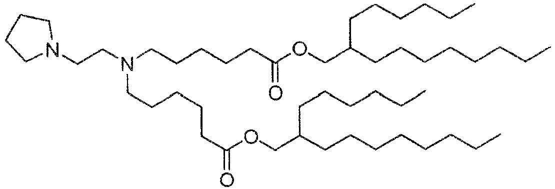 | C | 6.47 |
| I-33 | 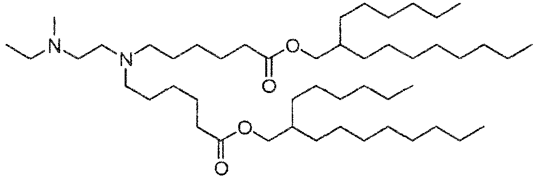 | C | 6.27 |
| I-34 | 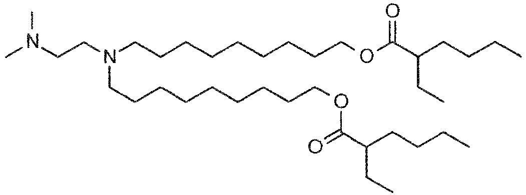 | B | - |
| I-35 | 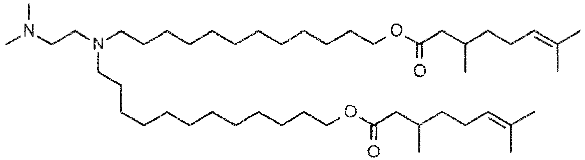 | B | 6.21 |
| I-36 | 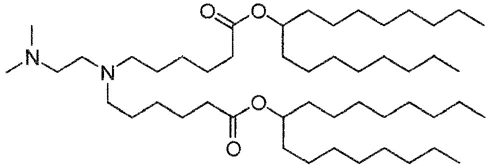 | C | - |
| I-37 | 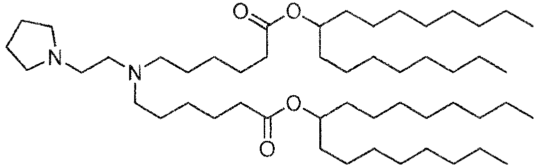 | C | - |
| I-38 | 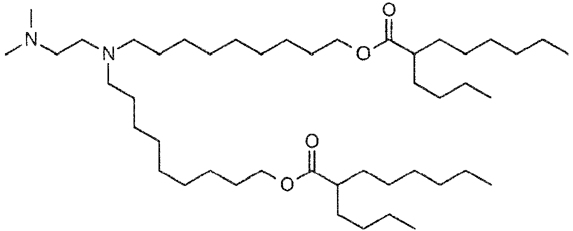 | B | 6.24 |
| I-39 | 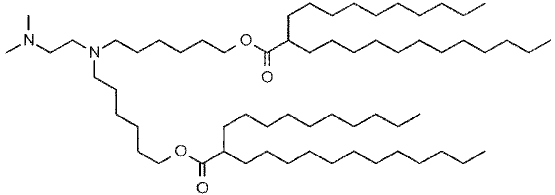 | B | 5.82 |
| I-40 | 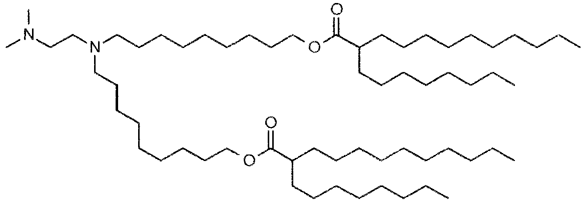 | B | 6.38 |
| I-41 | 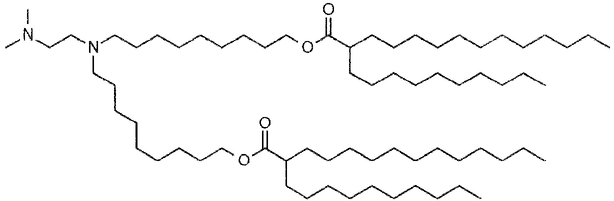 | B | 5.91 |

**Table 2:**

| **No.** | **Structure** | **Prep. Method** | **pKa** |
|---|---|---|---|
| II-1 | 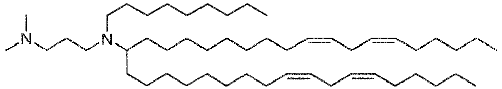 | D | 5.64 |
| II-2 | 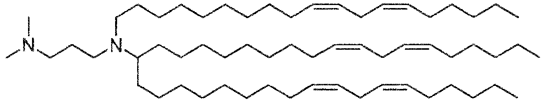 | D | - |
| II-3 | 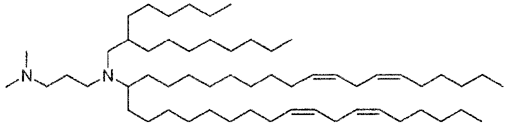 | D | - |
| II-4 | 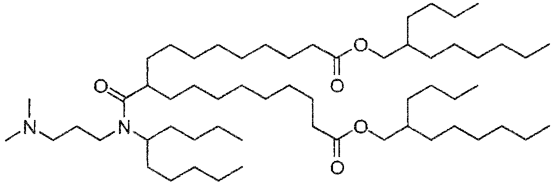 | E | - |
| II-5 | 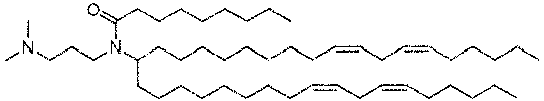 | D | 6.27 |
| II-6 | 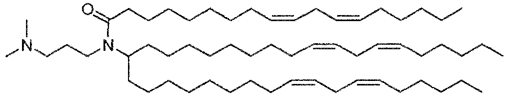 | D | 6.14 |
| II-7 | 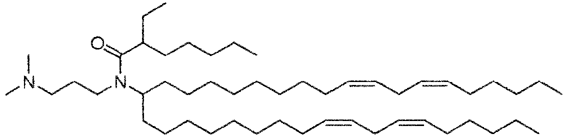 | D | 5.93 |
| II-8 | 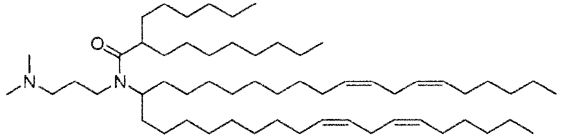 | D | 5.35 |
| II-9 | 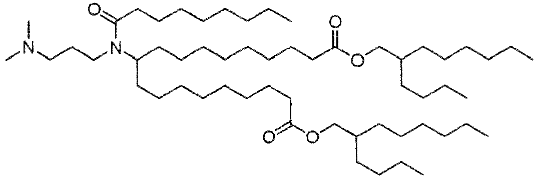 | D | 6.27 |
| II-10 | 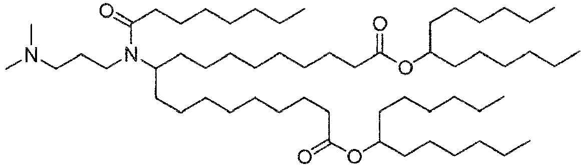 | D | 6.16 |
| II-11 | 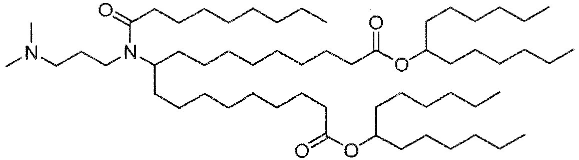 | D | 6.13 |
| II-12 | 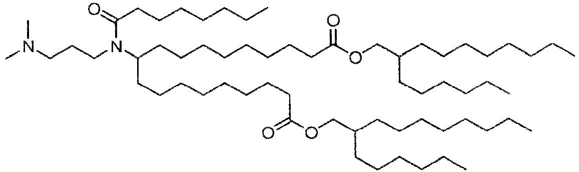 | D | 6.21 |
| II-13 | 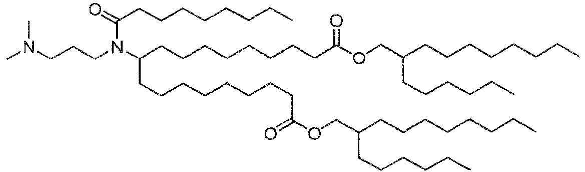 | D | 6.22 |
| II-14 | 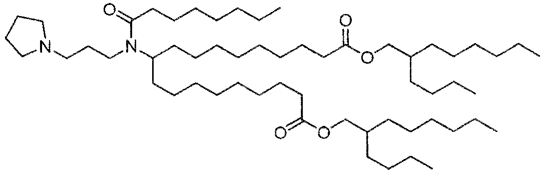 | D | 6.33 |
| II-15 | 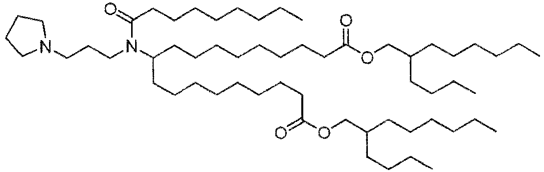 | D | 6.32 |
| II-16 | 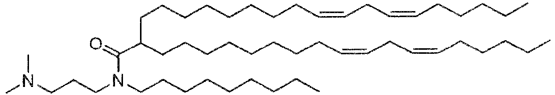 | E | 6.37 |
| II-17 | 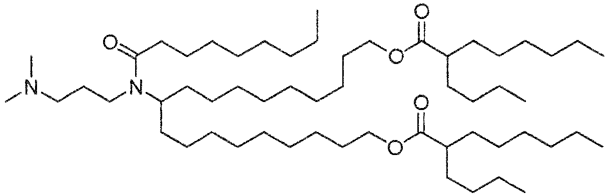 | F | 6.27 |
| II-18 | 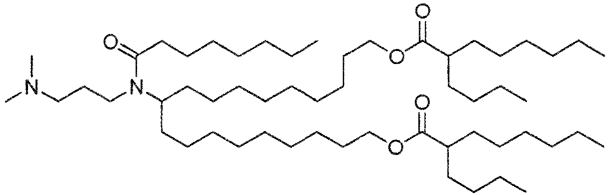 | D | - |
| II-19 | 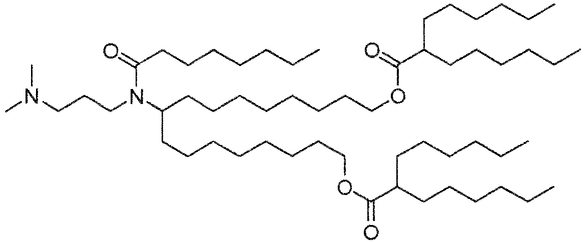 | D | - |
| II-20 | 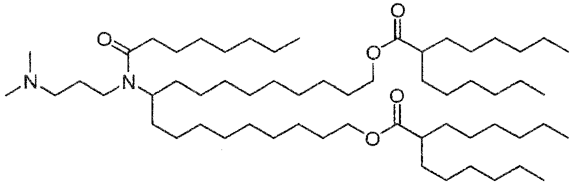 | D | - |
| II-21 | 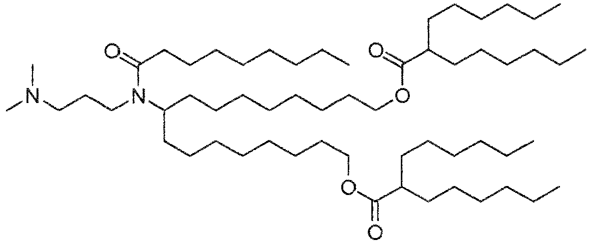 | D | - |
| II-22 | 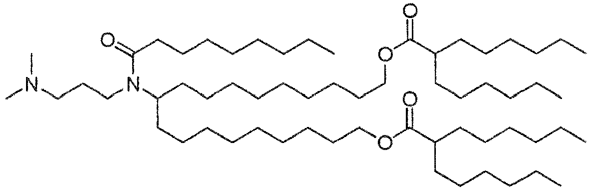 | D | - |
| II-23 | 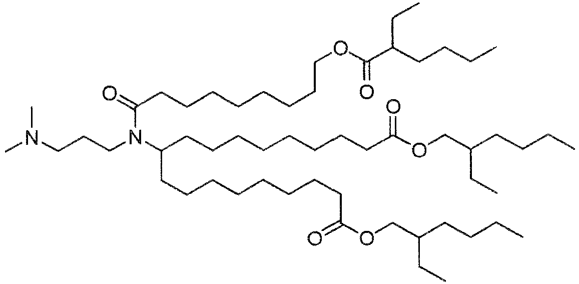 | D | - |
| II-24 | 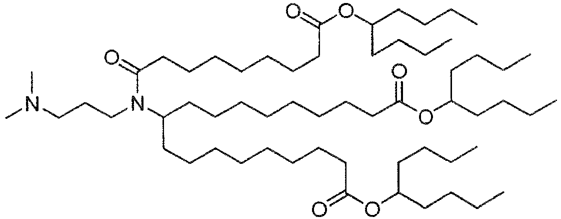 | D | 6.14 |
| II-25 | 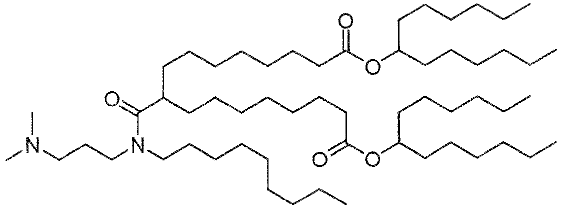 | E | - |
| II-26 | 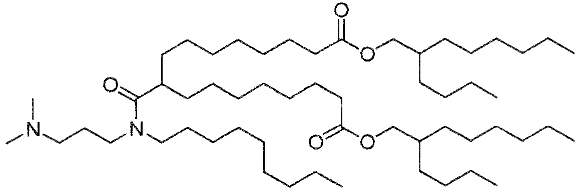 | E | - |
| II-27 | 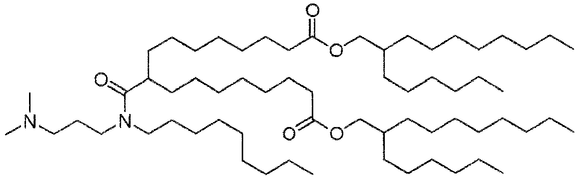 | E | - |
| II-28 | 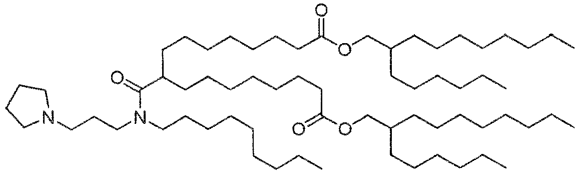 | E | - |
| II-29 | 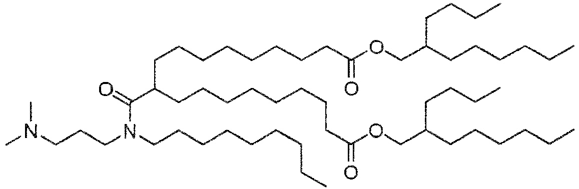 | E | - |
| II-30 | 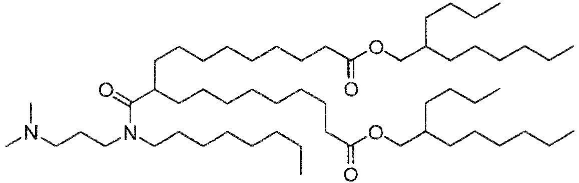 | E | - |
| II-31 | 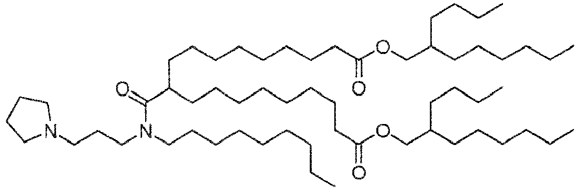 | E | - |
| II-32 | 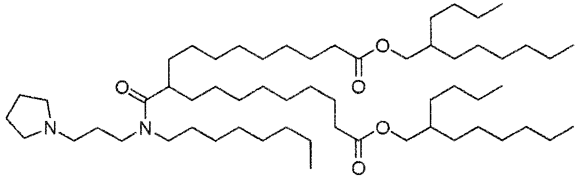 | E | - |
| II-33 | 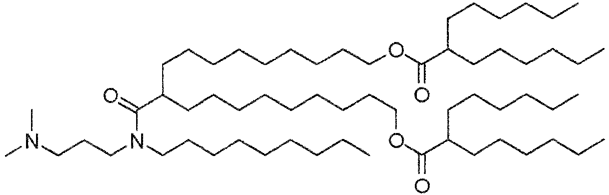 | E | - |
| II-34 | 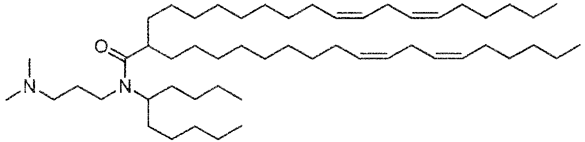 | E | - |
| II-35 | 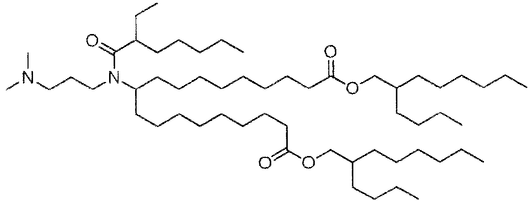 | D | 5.97 |
| II-36 | 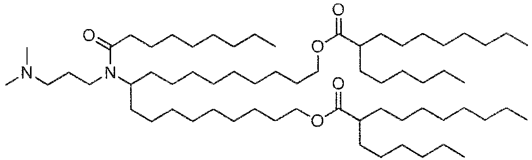 | F | 6.13 |
| II-37 | 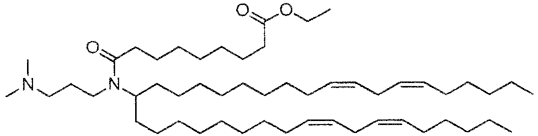 | D | 5.61 |
| II-38 | 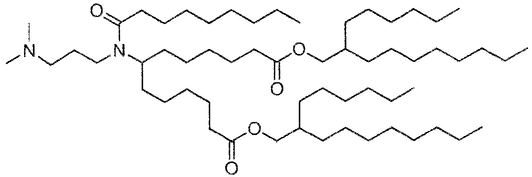 | D | 6.45 |
| II-39 | 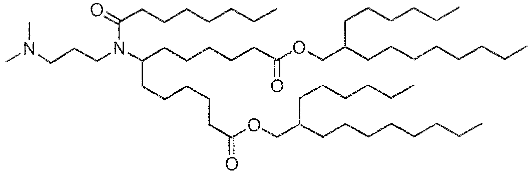 | D | 6.45 |
| II-40 | 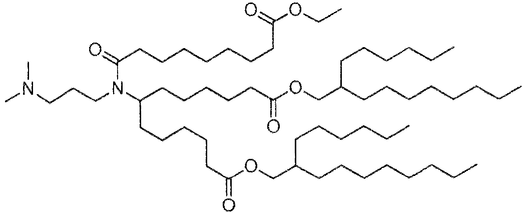 | D | 6.57 |
| II-41 | 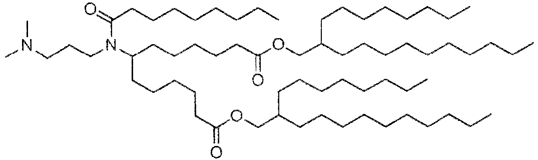 | D | - |
| II-42 | 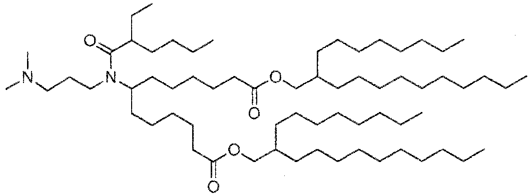 | D | - |
| II-43 | 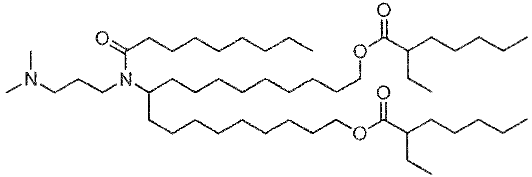 | F | - |
| II-44 | 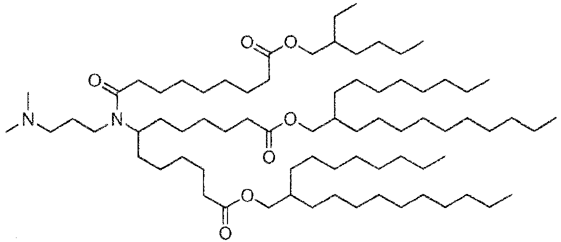 | D | - |
| II-45 | 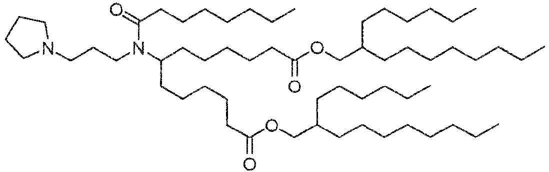 | D | - |
| II-46 | 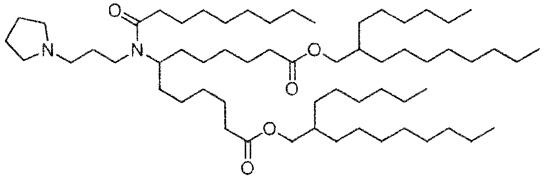 | D | - |

**Table 3:**

| **No.** | **Structure** | **Prep. Method** | **pKa** |
|---|---|---|---|
| III -1 | 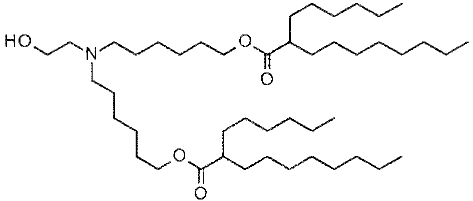 | G | 5.89 |
| III-2 | 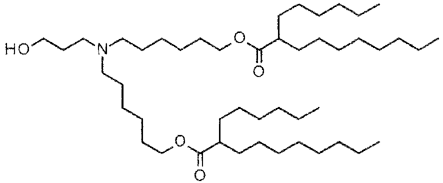 | G | 6.05 |
| III-3 | 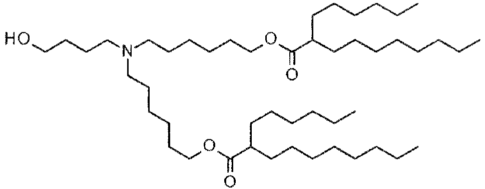 | G | 6.09 |
| III-4 | 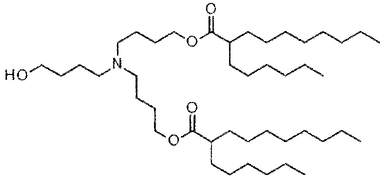 | G | 5.60 |
| III-5 | 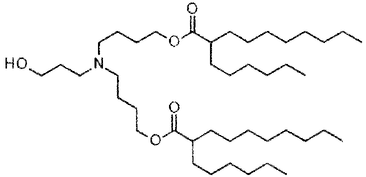 | G | 5.59 |
| III-6 | 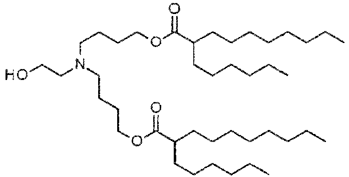 | G | 5.42 |
| III-7 | 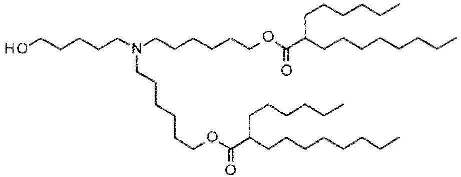 | G | 6.11 |
| III-8 | 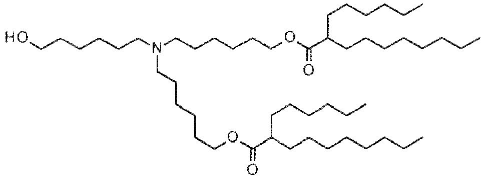 | G | 5.84 |
| III-9 | 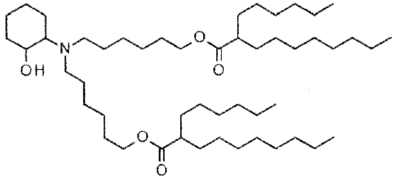 | G | - |
| III-10 | 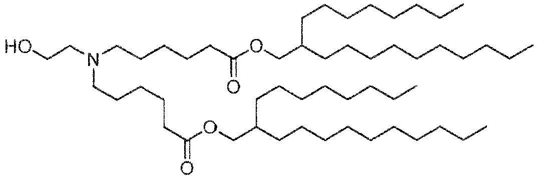 | G | - |
| III-11 | 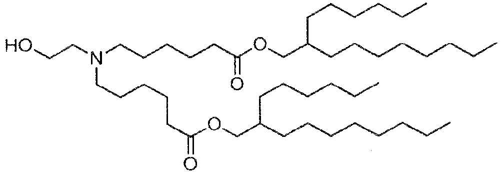 | G | - |
| III-12 | 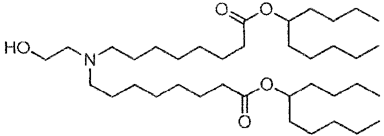 | G | - |
| III-13 | 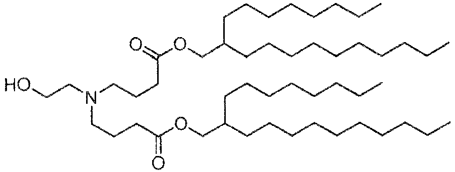 | G | - |
| III-14 | 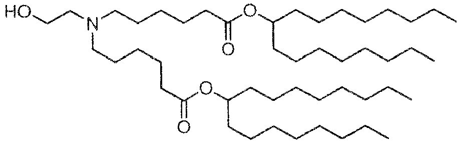 | G | - |
| III-15 | 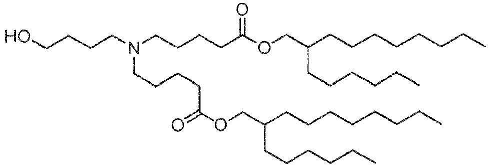 | G | 6.14 |
| III-16 | 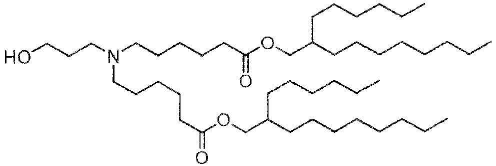 | G | 6.31 |
| III-17 | 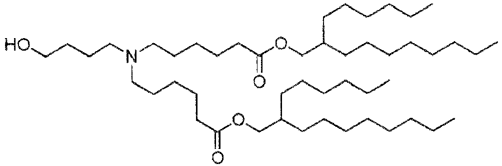 | G | 6.28 |
| III-18 | 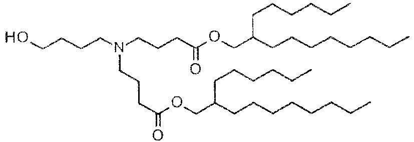 | G | - |
| III-19 | 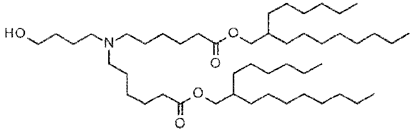 | G | - |
| III-20 | 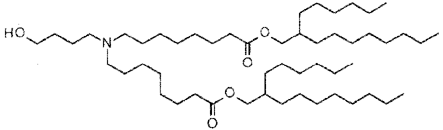 | G | 6.36 |
| III-21 | 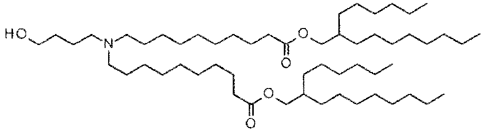 | G | - |
| III-22 | 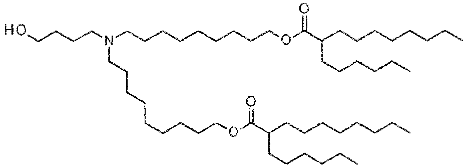 | G | 6.10 |
| III-23 | 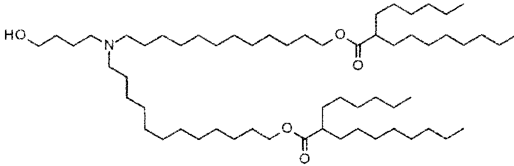 | G | 5.98 |
| III-24 | 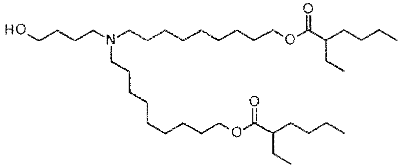 | G | - |
| III-25 | 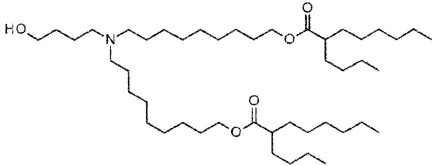 | G | 6.22 |
| III-26 | 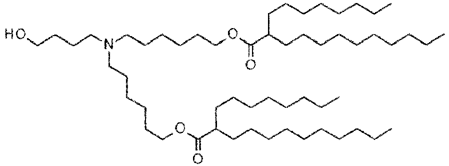 | G | 5.84 |
| III-27 | 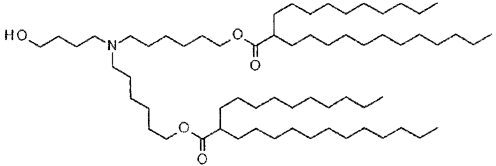 | G | 5.77 |
| III-28 | 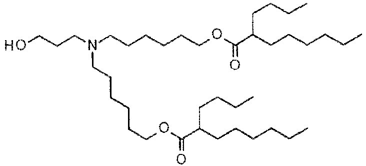 | G | - |
| III-29 | 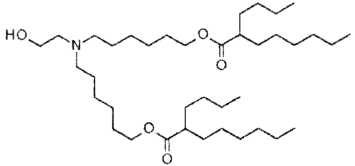 | G | - |
| III-30 | 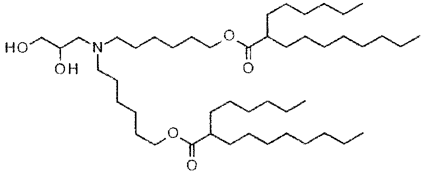 | G | 6.09 |
| III-31 | 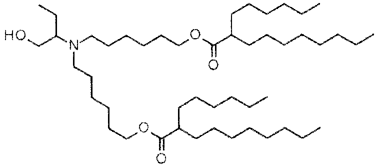 | G | - |
| III-32 | 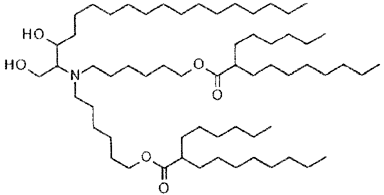 | G | - |
| III-33 | 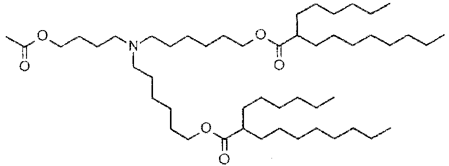 | G | - |
| III-34 | 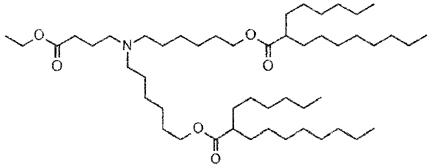 | G | - |
| III-35 | 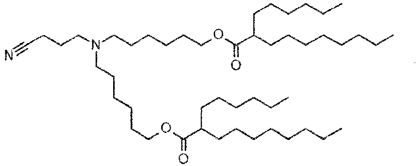 | G | - |
| III-36 | 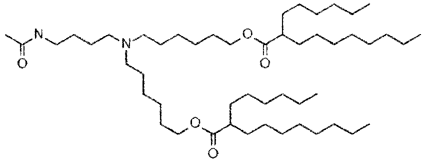 | G | - |
| III-37 | 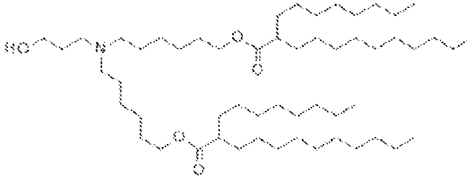 | G | - |
| III-38 | 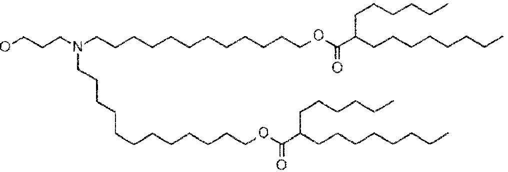 | G | - |
| III-39 | 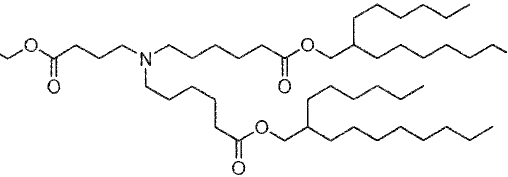 | G | - |
| III-40 | 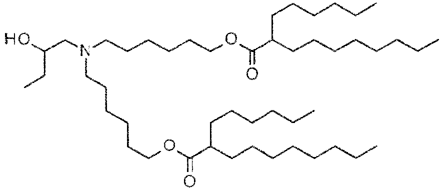 | G | - |
| III-41 | 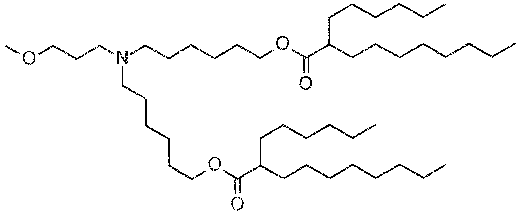 | G | - |
| III-42 | 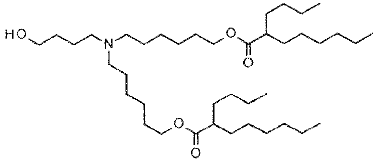 | G | - |
| III-43 | 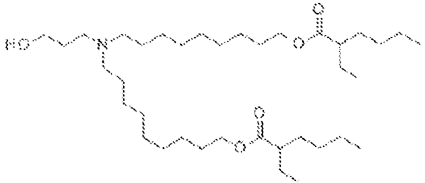 | G | - |
| III-44 | 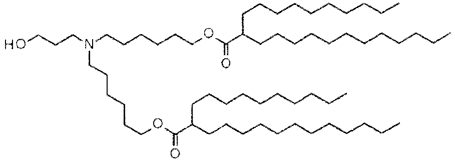 | G | - |
| III-45 | 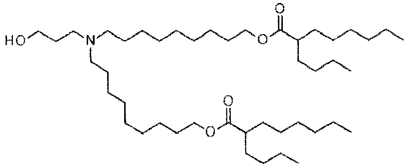 | G | - |
| III-46 | 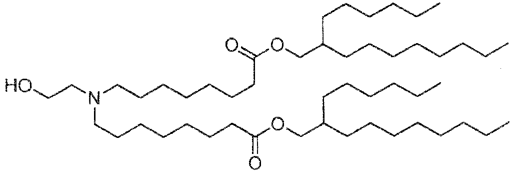 | G | - |
| III-47 | 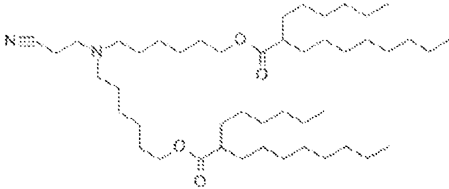 | G | - |
| III-48 | 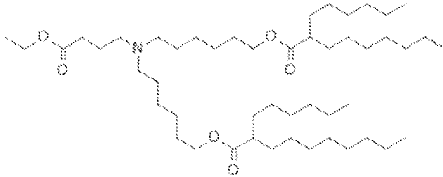 | G | - |
| III-49 | 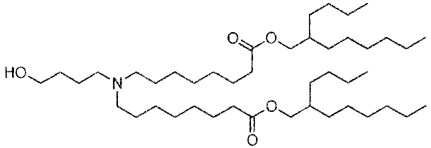 | G | - |

**Table 4:**

| **No.** | **Structure** |
|---|---|
| IV-1 | 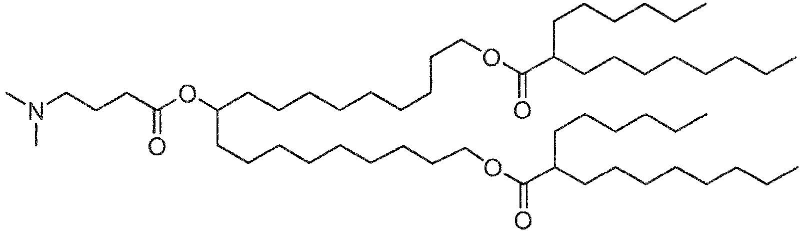 |
| IV-2 | 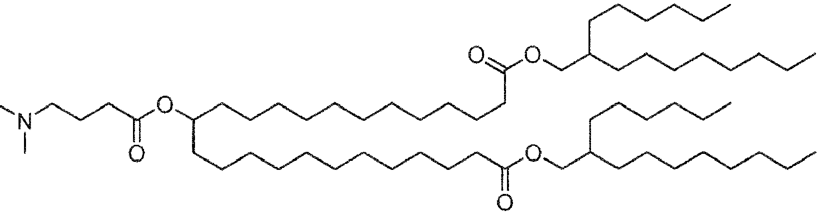 |
| IV-3 | 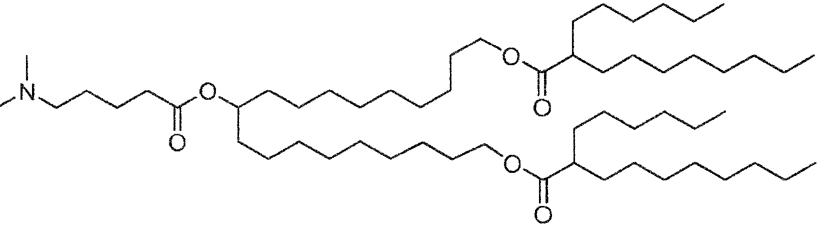 |

In some embodiments the ionizable lipid has one of the following structures: 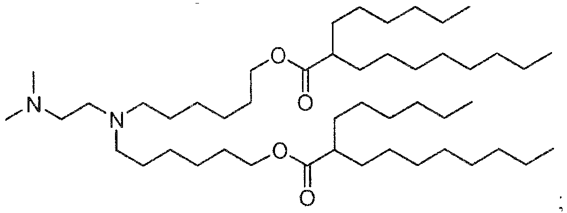 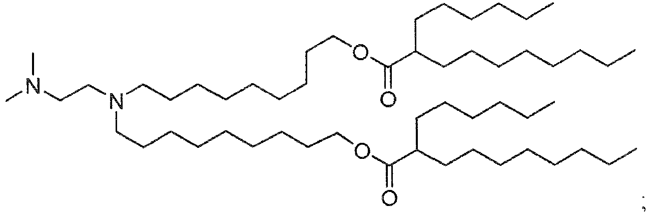 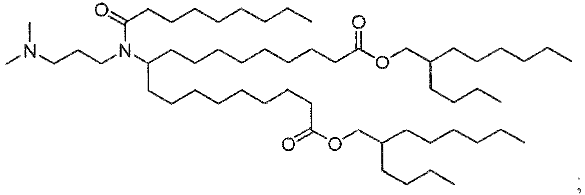 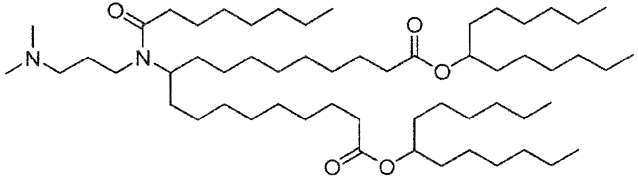 or

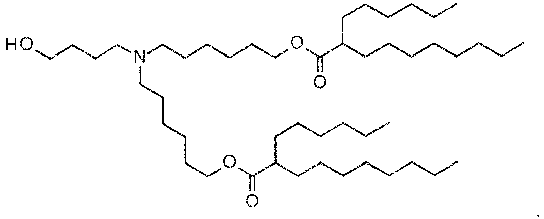

In some embodiments, the ionizable lipid has the following structure: 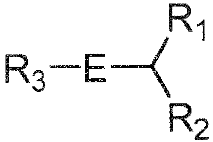
or a pharmaceutically acceptable salt, tautomer, prodrug or stereoisomer thereof, wherein: R₁ and R₂ are each independently for each occurrence optionally substituted C₁₀-C₃₀ alkyl, optionally substituted C₁₀-C₃₀ alkenyl, optionally substituted C₁₀-C₃₀ alkynyl or optionally substituted C₁₀-C₃₀ acyl;
R₃ is H, optionally substituted C₁₀-C₁₀ alkyl, optionally substituted C₂-C₁₀ alkenyl, optionally substituted C₂-C₁₀ alkynyl, alkylhetrocycle, alkylphosphate, alkylphosphorothioate, alkylphosphorodithioate, alkylphosphonate, alkylamine, hydroxyalkyl, ω-aminoalkyl, ω-(substituted)aminoalkyl, ω-phosphoalkyl, ω- thiophosphoalkyl, optionally substituted polyethylene glycol (PEG, mw 100-40K), optionally substituted mPEG (mw 120-40K), heteroaryl, or heterocycle, or linker-ligand; and
E is O, S, N(Q), C(O), N(Q)C(O), C(O)N(Q), (Q)N(CO)O, O(CO)N(Q), S(O), NS(O)₂N(Q), S(O)₂, N(Q)S(O)₂, SS, O=N, aryl, heteroaryl, cyclic or heterocycle; and
Q is H, alkyl, ω-aminoalkyl, ω-(substituted)aminoalkyl, ω-phosphoalkyl or ω-thiophosphoalkyl.

In some embodiments the ionizable lipid has one of the following structures: 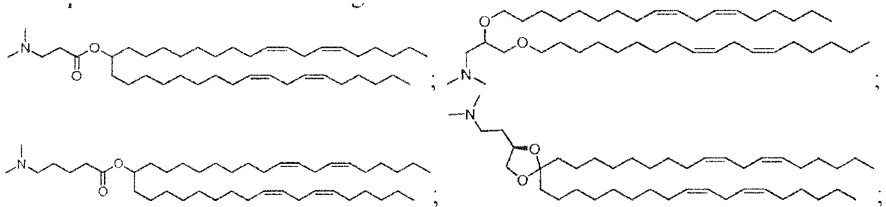 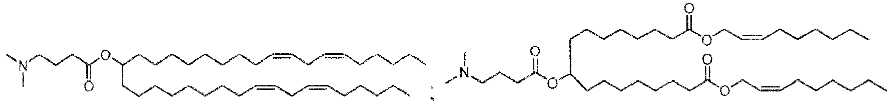 or

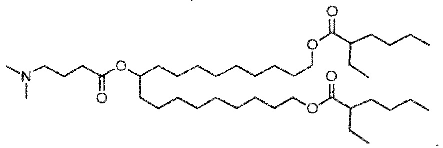

In preferred embodiments the ionizable lipid is [(4-Hydroxybutyl)azanediyl]di(hexane-6,1-diyl) bis(2-hexyldecanoate), also known ALC-0315, and as shown in the following formula:

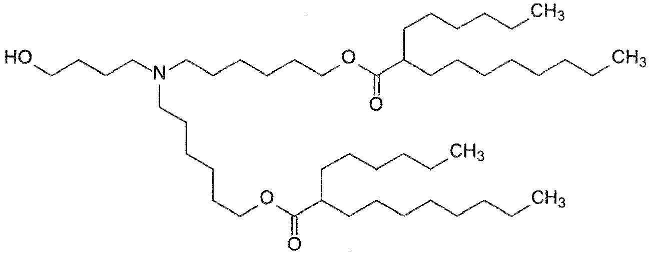

In some embodiments the molar ratio of ionizable lipid to a neutral lipid ranges from about 4.1:1.0 to about 4.9:1.0, 4.5:1.0 to about 4.8:1.0, or 4.7:1.0 to about 4.8:1.0. In some embodiment the molar ratio of cationic lipid to the neutral lipid ranges from about 2:1 to about 8:1, preferably 5:1 to 1:1.

In some embodiments the molar ratio of ionizable lipid to the polymer conjugated lipid ranges from about 35:1 to about 25:1, or 100:1 to about 20:1.

In some embodiments, the ionizable lipid has the following structure:

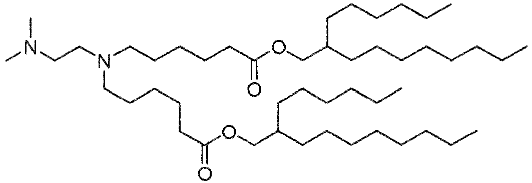

A further aspect of the invention relates to a lipid nanoparticle comprising:
i) a first cationic lipid as an ionizable lipid (a) having a first effective pKa;
ii) a second cationic lipid as an ionizable lipid (a) having a second effective pKa, the second effective pKa being greater than the first effective pKa;
iii) a neutral lipid;
iv) a steroid;
v) a polymer conjugated lipid;
vi) a therapeutic agent, or a pharmaceutically acceptable salt or prodrug thereof, encapsulated within or associated with the lipid nanoparticle, and
vii) a surfactant,
wherein the lipid nanoparticle has an effective pKa between the first and second effective pKa's.

In some embodiments the first effective pKa is less than 5.75. In some embodiments the second effective pKa is greater than 6.25. In some embodiments, the lipid nanoparticle of any one of claims 48-50, wherein the lipid nanoparticle has an effective pKa ranging from 5.90 to 6.35. In some embodiments, the mol ratio of the first cationic lipid to the second cationic lipid ranges from 1:20 to 1:2.

In some embodiments, a LNP or LiNP of the invention comprises a first cationic lipid as an ionizable lipid (a), or a second cationic lipid as an ionizable lipid (a), and one or both has a structure of Formula a-I: 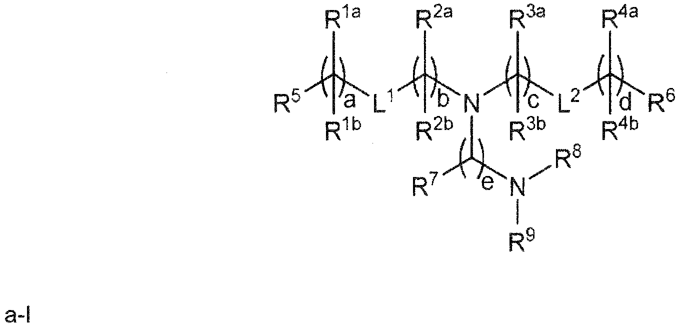 or a pharmaceutically acceptable salt, tautomer, prodrug or stereoisomer thereof, wherein:
one of L¹ or L² is-O(C=O)-, -(C=O)O-, -C(=O)-, -O-, -S(O)ₓ-, -S-S-, - C(=O)S-, SC(=O)-, -NR^{a}C(=O)-, -C(=O)NR^{a}-, NR^{a}C(=O)NR^{a}-, -OC(=O)NR^{a}- or - NR^{a}C(=O)O-, and the other of L¹ or L² is-O(C=O)-, -(C=O)O-, -C(=O)-, -O-, -S(O)ₓ-, -S- S-, -C(=O)S-, SC(=O)-,
-NR^{a}C(=O)-, -C(=O)NR^{a}-, ,NR^{a}C(=O)NR^{a}-, -OC(=O)NR^{a}- or -NR^{a}C(=O)O- or a direct bond;
R^{a} is H or C₁-C₁₂ alkyl;
R^{1a} and R^{1b} are, at each occurrence, independently either (a) H or C₁-C₁₂ alkyl, or (b) R^{1a} is H or C₁-C₁₂ alkyl, and R^{1b} together with the carbon atom to which it is bound is taken together with an adjacent R^{1b} and the carbon atom to which it is bound to form a carbon-carbon double bond;
R^{2a} and R^{2b} are, at each occurrence, independently either (a) H or C₁-C₁₂ alkyl, or (b) R^{2a} is H or C₁-C₁₂ alkyl, and R^{2b} together with the carbon atom to which it is bound is taken together with an adjacent R^{2b} and the carbon atom to which it is bound to form a carbon-carbon double bond;
R^{3a} and R^{3b} are, at each occurrence, independently either (a) H or C₁-C₁₂ alkyl, or (b) R^{3a} is H or C₁-C₁₂ alkyl, and R^{3b} together with the carbon atom to which it is bound is taken together with an adjacent R^{3b} and the carbon atom to which it is bound to form a carbon-carbon double bond; R^{4a} and R^{4b} are, at each occurrence, independently either (a) H or C₁-C₁₂ alkyl, or (b) R^{4a} is H or C₁-C₁₂ alkyl, and R^{4b} together with the carbon atom to which it is bound is taken together with an adjacent R^{4b} and the carbon atom to which it is bound to form a carbon-carbon double bond;
R⁵ and R⁶ are each independently methyl or cycloalkyl;
R⁷ is, at each occurrence, independently H or C₁-C₁₂ alkyl;
R⁸ and R⁹ are each independently unsubstituted C₁-C₁₂ alkyl; or R⁸ and R⁹, together with the nitrogen atom to which they are attached, form a 5, 6 or 7-membered heterocyclic ring comprising one nitrogen atom;
a and d are each independently an integer from 0 to 24;
b and c are each independently an integer from 1 to 24;
e is 1 or 2; and
x is 0, 1 or 2.

In some embodiments, the first and second cationic lipids as ionizable lipids (a) are each independently selected from a lipid of Formula a-I. In some embodiments the first cationic lipid, or the second cationic lipid or both has a structure of Formula a-II: 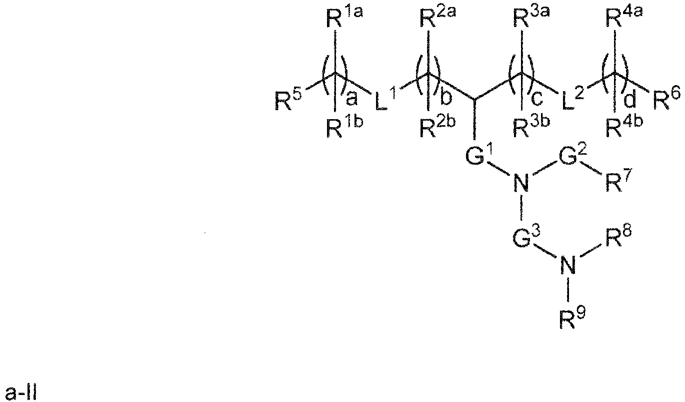 or a pharmaceutically acceptable salt, tautomer, prodrug or stereoisomer thereof, wherein:
one of L¹ or L² is-O(C=O)-, -(C=O)O-, -C(=O)-, -O-, -S(O)ₓ-, -S-S-, - C(=O)S-, SC(=O)-, - NR^{a}C(=O)-, -C(=O)NR^{a}-, NR^{a}C(=O)NR^{a}-, -OC(=O)NR^{a}- or - NR^{a}C(=O)O-, and the other of L¹ or L² is-O(C=O)-, -(C=O)O-, -C(=O)-, -O-, -S(O)ₓ₋, -S- S-, -C(=O)S-, SC(=O)-,
-NR^{a}C(=O)-, -C(=O)NR^{a}-, ,NR^{a}C(=O)NR^{a}-, -OC(=O)NR^{a}- or -NR^{a}C(=O)O- or a direct bond;
G¹ is C₁-C₂ alkylene, -(C=O)-, -O(C=O)-, -SC(=O)-, -NR^{a}C(=O)- or a direct bond;
G² is-C(=O)-, -(C=O)O-, -C(=O)S-, -C(=O)NR^{a}- or a direct bond; G³ is C₁-C₆ alkylene;
R^{a} is H or C₁-C₁₂ alkyl;
R^{1a} and R^{1b} are, at each occurrence, independently either: (a) H or C₁-C₁₂ alkyl; or (b) R^{1a} is H or C₁-C₁₂ alkyl, and R^{1b} together with the carbon atom to which it is bound is taken together with an adjacent R^{1b} and the carbon atom to which it is bound to form a carbon-carbon double bond;
R^{2a} and R^{2b} are, at each occurrence, independently either: (a) H or C₁-C₁₂ alkyl; or (b) R^{2a} is H or C₁-C₁₂ alkyl, and R^{2b} together with the carbon atom to which it is bound is taken together with an adjacent R^{2b} and the carbon atom to which it is bound to form a carbon-carbon double bond;
R^{3a} and R^{3b} are, at each occurrence, independently either (a): H or C₁-C₁₂ alkyl; or (b) R^{3a} is H or C₁-C₁₂ alkyl, and R^{3b} together with the carbon atom to which it is bound is taken together with an adjacent R^{3b} and the carbon atom to which it is bound to form a carbon-carbon double bond;
R^{4a} and R^{4b} are, at each occurrence, independently either: (a) H or C₁-C₁₂ alkyl; or (b) R^{4a} is H or C₁-C₁₂ alkyl, and R^{4b} together with the carbon atom to which it is bound is taken together with an adjacent R^{4b} and the carbon atom to which it is bound to form a carbon-carbon double bond;
R⁵ and R⁶ are each independently H or methyl;
R⁷ is C₄-C₂₀ alkyl;
R⁸ and R⁹ are each independently C₁-C₁₂ alkyl; or R⁸ and R⁹, together with the nitrogen atom to which they are attached, form a 5, 6 or 7-membered heterocyclic ring;
a, b, c and d are each independently an integer from 1 to 24; and
x is 0, 1 or 2.

In some embodiments, the first and second cationic lipids as ionizable lipids (a) are each, independently selected from a lipid of Formula a-II.

In some embodiments, the first cationic lipid as an ionizable lipid (a), or the second cationic lipid as an ionizable lipid (a) or both has a structure of Formula a-III: 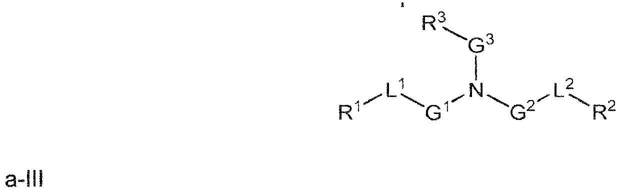 or a pharmaceutically acceptable salt, prodrug or stereoisomer thereof, wherein:
one of L¹ or L² is-O(C=O)-, -(C=O)O-, -C(=O)-, -O-, -S(O)ₓ₋, -S-S-, - C(=O)S-, SC(=O)-, -NR^{a}C(=O)-, -C(=O)NR^{a}-, NR^{a}C(=O)NR^{a}-, -OC(=O)NR^{a}- or - NR^{a}C(=O)O-, and the other of L¹ or L² is-O(C=O)-, -(C=O)O-, -C(=O)-, -O-, -S(O)ₓ₋, -S- S-, -C(=O)S-, SC(=O)-,
-NR^{a}C(=O)-, -C(=O)NR^{a}-, , NR^{a}C(=O)NR^{a}-, -OC(=O)NR^{a}- or -NR^{a}C(=O)O- or a direct bond;
G¹ and G² are each independently unsubstituted C₁-C₁₂ alkylene or C₁-C₁₂ alkenylene;
G³ is C₁-C₂₄ alkylene, C₁-C₂₄ alkenylene, C₃-C₈ cycloalkylene, C₃-C₈ cycloalkenylene;
R^{a} is H or C₁-C₁₂ alkyl;
R¹ and R² are each independently C₆-C₂₄ alkyl or C₆-C₂₄ alkenyl;
R³ is H, OR⁵, CN, -C(=O)OR⁴, -OC(=O)R⁴ or-NR⁵C(=O)R⁴; R⁴ is C₁-C₁₂ alkyl;
R⁵ is H or C₁-C₆ alkyl; and
x is 0, 1 or 2.

In some embodiments, the first and second cationic lipids as ionizable lipids (a) are each, independently, selected from a lipid of Formula a-III.

In some embodiments, the first cationic lipid as an ionizable lipid (a), or the second cationic lipid as an ionizable lipid (a) or both has a structure of Formula a-IV: 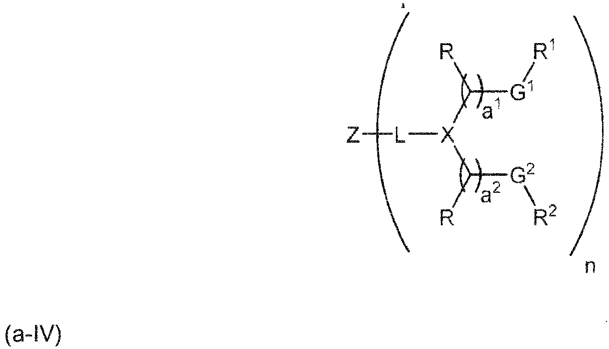 or a pharmaceutically acceptable salt, prodrug or stereoisomer thereof, wherein:
one of G¹ or G² is, at each occurrence, -O(C=O)-, -(C=O)O-, -C(=O)-, -O-, -S(O)_{y}-, -S-S-, -C(=O)S-, SC(=O)-, -N(R^{a})C(=O)-, -C(=O)N(R^{a})- , -N(R^{a})C(=O)N(R^{a})-,
-OC(=O)N(R^{a})- or -N(R^{a})C(=O)O-, and the other of G¹ or G² is, at each occurrence, -O(C=O)-,
-(C=O)O-, -C(=O)-, -O-, -S(O)_{y}-, -S-S-, -C(=O)S-, -SC(=O)-, -N(R^{a})C(=O)-, - C(=O)N(R^{a})-,
-N(R^{a})C(=O)N(R^{a})-, -OC(=O)N(R^{a})- or-N(R^{a})C(=O)O- or a direct bond; L is, at each occurrence, -O(C=O)-, wherein ~ represents a covalent bond to X;
X is CRa;
Z is alkyl, cycloalkyl or a monovalent moiety comprising at least one polar functional group when n is 1; or Z is alkylene, cycloalkylene or a polyvalent moiety comprising at least one polar functional group when n is greater than 1;
R^{a} is, at each occurrence, independently H, C₁-C₁₂ alkyl, C₁-C₁₂ hydroxylalkyl, C₁-C₁₂ aminoalkyl, C₁-C₁₂ alkylaminylalkyl, C₁-C₁₂ alkoxyalkyl, C₁-C₁₂ alkoxycarbonyl, C₁-C₁₂ alkylcarbonyloxy, C₁-C₁₂ alkylcarbonyloxyalkyl or C₁-C₁₂ alkylcarbonyl;
R is, at each occurrence, independently either: (a) H or C₁-C₁₂ alkyl; or (b) R together with the carbon atom to which it is bound is taken together with an adjacent R and the carbon atom to which it is bound to form a carbon-carbon double bond;
R¹ and R² have, at each occurrence, the following structure, respectively: 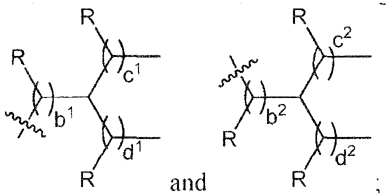
R¹ R²
a¹ and a² are, at each occurrence, independently an integer from 3 to 12; b¹ and b² are, at each occurrence, independently 0 or 1;
c¹ and c² are, at each occurrence, independently an integer from 5 to 10; d¹ and d² are, at each occurrence, independently an integer from 5 to 10; y is, at each occurrence, independently an integer from 0 to 2; and n is an integer from 1 to 6,
wherein each alkyl, alkylene, hydroxylalkyl, aminoalkyl, alkylaminylalkyl, alkoxyalkyl, alkoxycarbonyl, alkylcarbonyloxy, alkylcarbonyloxyalkyl and alkylcarbonyl is optionally substituted with one or more substituent.

In some embodiments, the first and second cationic lipids as ionizable lipids (a) are each, independently, selected from a lipid of Formula a-IV.

In some embodiments, the first cationic lipid as an ionizable lipid (a), or the second cationic lipid as an ionizable lipid (a), or both has a structure of Formula a-V: 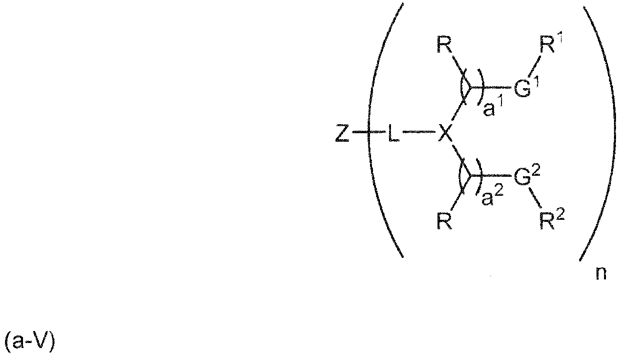 or a pharmaceutically acceptable salt, prodrug or stereoisomer thereof, wherein:
one of G¹ or G² is, at each occurrence, -O(C=O)-, -(C=O)O-, -C(=O)-, -O-, -S(O)_{y}-, -S-S-, -C(=O)S-, SC(=O)-, -N(R^{a})C(=O)-, -C(=O)N(R^{a})- , -N(R^{a})C(=O)N(R^{a})-,
-OC(=O)N(R^{a})- or -N(R^{a})C(=O)O-, and the other of G¹ or G² is, at each occurrence, -O(C=O)-,
-(C=O)O-, -C(=O)-, -O-, -S(O)_{y}-, -S-S-, -C(=O)S-, -SC(=O)-, -N(R^{a})C(=O)-, - C(=O)N(R^{a})-,
-N(R^{a})C(=O)N(R^{a})-, -OC(=O)N(R^{a})- or-N(R^{a})C(=O)O- or a direct bond;
L is, at each occurrence, ~O(C=O)-, wherein ~ represents a covalent bond to X;
X is CRa;
Z is alkyl, cycloalkyl or a monovalent moiety comprising at least one polar functional group when n is 1; or Z is alkylene, cycloalkylene or a polyvalent moiety comprising at least one polar functional group when n is greater than 1;
R^{a} is, at each occurrence, independently H, C₁-C₁₂ alkyl, C₁-C₁₂ hydroxylalkyl, C₁-C₁₂ aminoalkyl, C₁-C₁₂ alkylaminylalkyl, C₁-C₁₂ alkoxyalkyl, C₁-C₁₂ alkoxycarbonyl, C₁-C₁₂ alkylcarbonyloxy, C₁-C₁₂ alkylcarbonyloxyalkyl or C₁-C₁₂ alkylcarbonyl;
R is, at each occurrence, independently either: (a) H or C₁-C₁₂ alkyl; or (b) R together with the carbon atom to which it is bound is taken together with an adjacent R and the carbon atom to which it is bound to form a carbon-carbon double bond;
R¹ and R² have at each occurrence the following structure, respectively: 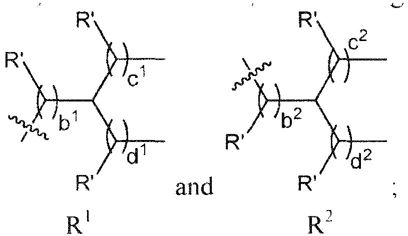
R' is, at each occurrence, independently H or C₁-C₁₂ alkyl; a¹ and a² are, at each occurrence, independently an integer from 3 to 12; b¹ and b² are, at each occurrence, independently 0 or 1;
c¹ and c² are, at each occurrence, independently an integer from 2 to 12; d¹ and d² are, at each occurrence, independently an integer from 2 to 12; y is, at each occurrence, independently an integer from 0 to 2; and n is an integer from 1 to 6,
wherein a', a², c¹, c², d¹ and d² are selected such that the sum of a¹+c¹+d¹ is an integer from 18 to 30, and the sum of a²+c²+d² is an integer from 18 to 30, and wherein each alkyl, alkylene, hydroxylalkyl, aminoalkyl, alkylaminylalkyl, alkoxyalkyl, alkoxycarbonyl, alkylcarbonyloxy, alkylcarbonyloxyalkyl and alkylcarbonyl is optionally substituted with one or more substituent.

In some embodiments, the first and second cationic lipids as ionizable lipids (a) are each, independently, selected from a lipid of Formula a-V.

In some embodiments, the first and second cationic lipids as ionizable lipids (a) have the following structures, respectively: 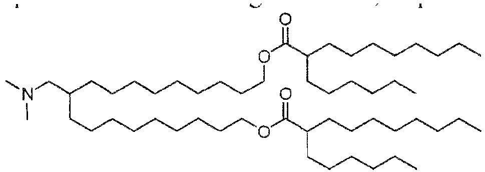 and

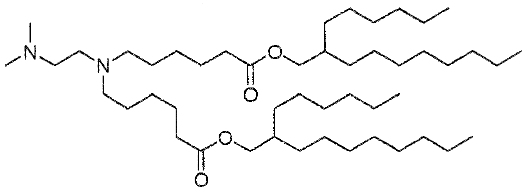

In some embodiments, the first cationic lipid as an ionizable lipid (a), the second cationic lipid as an ionizable lipid (a), or both have one of the following structures: 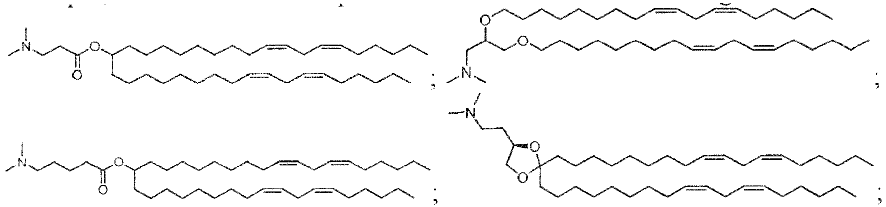 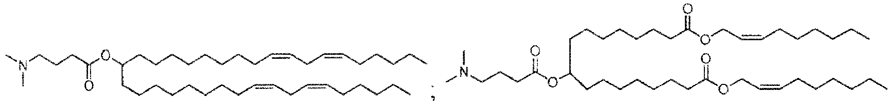 or

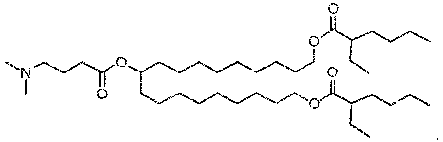

In some embodiments, when a compound of formula a-II is used, the total mol percent of cationic lipid as an ionizable lipid (a) in the lipid nanoparticle ranges from 40 to 55 mol percent based on total lipid present in the lipid nanoparticle. In some embodiments, the molar ratio of total cationic lipid to a neutral lipid ranges from about 2:1 to about 8:1. In some embodiments, the molar ratio of total cationic lipid to steroid ranges from 5:1 to 1:1. In some embodiments, the molar ratio of total cationic lipid to polymer conjugated lipid ranges from about 100:1 to about 20:1.

In some embodiments, the neutral lipid is distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoyl-phosphatidylethanolamine (POPE) and dioleoyl-phosphatidylethanolamine 4-(N-maleimidomethyl)-cyclohexane- 1carboxylate (DOPE-mal), dipalmitoyl phosphatidyl ethanolamine (DPPE), dimyristoylphosphoethanolamine (DMPE), distearoyl-phosphatidylethanolamine (DSPE), 16-O-monomethyl PE, 16-O-dimethyl PE, 18-1-trans PE, 1-stearioyl-2- oleoylphosphatidyethanol amine (SOPE) or 1,2-dielaidoyl-sn-glycero-3-phophoethanolamine (transDOPE), preferably the neutral lipid is DSPC, DPPC, DMPC, DOPC, POPC, DOPE or SM. In some embodiments, the neutral lipid is DSPC. In some embodiments, the steroid is cholesterol.

In some embodiments, the polymer conjugated lipid is present in a concentration ranging from 1.0 to 2.5 molar percent, preferably, about 1.7 molar percent, wherein the polymer conjugated lipid is present in a concentration of about 1.5 molar percent.

In some embodiments, the polymer conjugated lipid is a pegylated lipid. In some embodiments, the pegylated lipid is PEG-DAG, PEG-PE, PEG-S-DAG, PEG-cer or a PEG dialkyoxypropylcarbamate. In some embodiments, the pegylated lipid has the following Formula (a-VI): 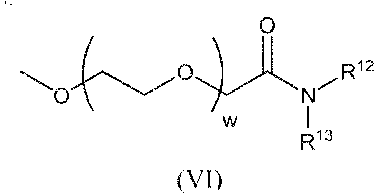 or a pharmaceutically acceptable salt, tautomer or stereoisomer thereof, wherein:
R¹² and R¹³ are each independently a straight or branched, saturated or unsaturated alkyl chain containing from 10 to 30 carbon atoms, wherein the alkyl chain is optionally interrupted by one or more ester bonds; and
w has a mean value ranging from 30 to 60.

Optionally, R¹² and R¹³ are each independently straight, saturated alkyl chains containing from 12 to 16 carbon atoms. Optionally, the average w ranges from 42 to 55, preferably, the average w is about 49. In some embodiments, the pegylated lipid has the following Formula (Via): 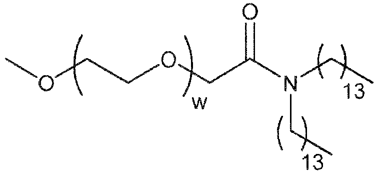 wherein the average w is about 49.

In some embodiments, the lipid nanoparticle forms a plurality of the nanoparticles having a polydispersity of less than 0.12. Preferably, the polydispersity is less than 0.08.

In some embodiments, the mean diameter ranges from 50 nm to 100 nm, preferably the diameter ranges from 60 nm to 85 nm.

An aspect of the invention relates to a method for administering a therapeutic agent to a patient in need thereof, the method comprising administering the lipid nanoparticle of the invention or the pharmaceutical composition of the invention to the patient.

A further aspect of the invention relates to a method for treating a disease in a patient in need thereof, the method comprising administering the lipid nanoparticle comprising a surfactant, or the pharmaceutical composition comprising a surfactant of the invention, to the patient, wherein the therapeutic agent is effective to treat the disease.

In one preferred embodiment, component (b) of the nanoparticles used in the various aspects of the invention comprises, or more preferably consists of, an ionizable lipidoid of the following formula (b-1) or a protonated form thereof. The ionizable lipidoid of the following formula (b-1) or its protonated forms which can be used as a preferred component (b) in the context of the present invention are described in detail in the PCT application WO 2014/207231 A1.

Thus, component (b) preferably comprises, or consists of, a lipidoid of the following formula (b-1) 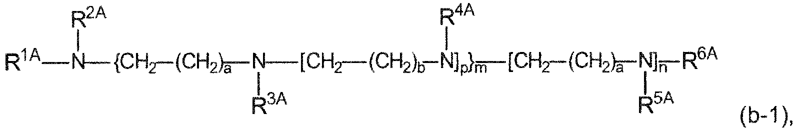 wherein the variables a, b, p, m, n and R^{1A} to R^{6A} are defined as follows:
a is 1 and b is an integer of 2 to 4; or a is an integer of 2 to 4 and b is 1,
p is 1 or 2,
m is 1 or 2; n is 0 or 1 and m+n is ≥ 2; and
R^{1A} to R^{6A} are independently of each other selected from hydrogen; -CH₂-CH(OH)-R^{7A}, -CH(R^{7A})-CH₂-OH, -CH₂-CH₂-(C=O)-O-R^{7A}, -CH₂-CH₂-(C=O)-NH-R^{7A} ; -CH₂-R^{7A}; -C(NH)-NH₂; a poly(ethylene glycol) chain; and a receptor ligand; wherein R^{7A} is selected from C3-C18 alkyl and C3-C18 alkenyl having one C-C double bond;
provided that at least two residues among R^{1A} to R^{6A} are selected from -CH₂-CH(OH)-R^{7A}, -CH(R^{7A})-CH₂-OH, -CH₂-CH₂-(C=O)-O-R^{7A}, -CH₂-CH₂-(C=O)-NH-R^{7A} and -CH₂-R^{7A} wherein R^{7A} is selected from C3-C18 alkyl or C3-C18 alkenyl having one C-C double bond;
or a protonated form thereof, wherein one or more of the nitrogen atoms contained in the compound of formula (I) are protonated to provide a compound carrying a positive charge.

Preferably, R^{1A} to R^{6A} are independently selected from hydrogen; a group -CH₂-CH(OH)-R^{7A}, -CH(R^{7A})-CH₂-OH, -CH₂-CH₂-(C=O)-O-R^{7A}, -CH₂-CH₂-(C=O)-NH-R^{7A}; and -CH₂-R^{7A} wherein R^{7A} is selected from C3-C18 alkyl and C3-C18 alkenyl having one C-C double bond; provided that at least two residues among R^{1A} to R^{6A}, more preferably at least three residues among R^{1A} to R^{6A}, and still more preferably at least four residues among R^{1A} to R^{6A} are a group selected from -CH₂-CH(OH)-R^{7A}, -CH(R^{7A})-CH₂-OH, -CH₂-CH₂-(C=O)-O-R^{7A}, -CH₂-CH₂-(C=O)-NH-R^{7A} and -CH₂-R^{7A} wherein R^{7A} is selected from C3-C18 alkyl and C3-C18 alkenyl having one C-C double bond. More preferably, R^{1A} to R^{6A} are independently selected from hydrogen and a group -CH₂-CH(OH)-R^{7A} wherein R^{7A} is selected from C3-C18 alkyl and C3-C18 alkenyl having one C-C double bond; provided that at least two residues among R^{1A} to R^{6A}, more preferably at least three residues among R^{1A} to R^{6A}, and still more preferably at least four residues among R^{1A} to R^{6A} are a group -CH₂-CH(OH)-R^{7A}, wherein R^{7A} is selected from C3-C18 alkyl and C3-C18 alkenyl having one C-C double bond.

Preferably, R^{7A} is selected from C8-C18 alkyl and C8-C18 alkenyl having one C-C double bond, and more preferably from C8-C12 alkyl and C8-C12 alkenyl having one C-C double bond. Generally, alkyl groups are preferred over alkenyl groups as R^{7A}.

As far as any of the groups R^{1A} to R^{6A} is a protecting group for an amino group, such as described for example in WO2006/138380, preferred embodiments thereof are t-butoxycarbonyl (Boc), 9-fluorenylmethoxycarbonyl (Fmoc), or carbobenzyloxy (Cbz).

As far as any of the groups R^{1A} to R^{6A} are a receptor ligand, useful examples are given in Philipp and Wagner in "Gene and Cell Therapy - Therapeutic Mechanisms and Strategy", 3rd Edition, Chapter 15. CRC Press, Taylor & Francis Group LLC, Boca Raton 2009. Preferred receptor ligands for lung tissue are described in Pfeifer et al. 2010, Ther Deliv. 1(1):133-48. Preferred receptor ligands include synthetic cyclic or linear peptides such as derived from screening peptide libraries for binding to a particular cell surface structure or particular cell type, cyclic or linear RGD peptides, synthetic or natural carbohydrates such as sialic acid, galactose or mannose or synthetic ligands derived from reacting a carbohydrate for example with a peptide, antibodies specifically recognizing cell surface structures, folic acid, epidermal growth factor and peptides derived thereof, transferrin, anti-transferrin receptor antibodies, nanobodies and antibody fragments, or approved drugs that bind to known cell surface molecules.

As far as any of the groups R^{1A} to R^{6A} are a poly(ethylene glycol) chain, the preferred molecular weight of the poly(ethylene glycol) chain is 100 - 20,000 g/mol, more preferably 1,000 - 10,000 g/mol and most preferred is 1,000 - 5,000 g/mol.

The variable p in formula (b-1) is preferably 1.

In formula (b-1), m is 1 or 2; n is 0 or 1 and m+n is ≥ 2. In other words, if m is 1, n must also be 1, and if m is 2, n can be 0 or 1. If n is 0, m must be 2. If n is 1, m can be 1 or 2.

The variable n in formula (b-1) is preferably 1. It is more preferred that m is 1 and n is 1.

Thus, the combination of p = 1, m = 1 and n = 1 is likewise preferred.

As for the variables a and b in formula (b-1), it is preferred that one of a and b is 1, and the other one is 2 or 3. It is more preferred that a is 1 and b is 2, or that a is 2 and b is 1. Most preferably, a is 1 and b is 2.

In view of the above, it is further preferred that the compound of formula (b-1) is a compound of formula (b-1a) and that component (b) comprises or consists of a lipidoid of the following formula (b-1a):

R^{1A}-NR^{2A}-CH₂-(CH₂)ₐ-NR^{3A}-CH₂-(CH₂)_{b}-NR^{4A}-CH₂-(CH₂)ₐ-NR^{5A}-R^{6A} (b-1a),

wherein a, b, and R^{1A} to R^{6A} are defined as in formula (b-1), including preferred embodiments thereof;
or a protonated form thereof wherein one or more of the nitrogen atoms indicated in formula (b-1a) are protonated to provide a compound carrying a positive charge.

In accordance with a still further preferred embodiment, the compound of formula (b-1) is a compound of formula (b-1b) and component (b) comprises or consists of a lipidoid compound of the following formula (b-1b), 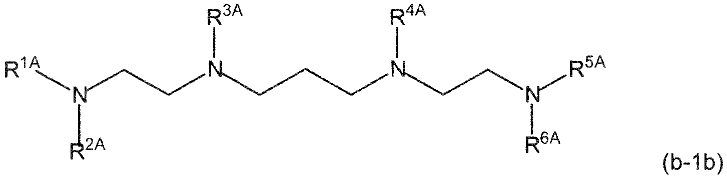 wherein R^{1A} to R^{6A} are defined as in formula (la), including preferred embodiments thereof;
or a protonated form thereof wherein one or more of the nitrogen atoms indicated in formula (b-1b) are protonated to provide a compound carrying a positive charge.

Thus, in a accordance with a particularly preferred embodiment, component (b) comprises or consists of a lipidoid compound of the above formula (b-1b) or a protonated form thereof, and R^{1A} to R^{6A} are independently selected from hydrogen and -CH₂-CH(OH)-R^{7A}, wherein R^{7A} is selected from C8-C18 alkyl and C8-C18 alkenyl having one C-C double bond, provided that at least two residues among R^{1A} to R^{6A} are -CH₂-CH(OH)-R^{7A}, more preferably at least three residues among R^{1A} to R^{6A}, and still more preferably at least four residues among R^{1A} to R^{6A} are -CH₂-CH(OH)-R^{7A}, wherein R^{7A} is selected from C8-C18 alkyl and C8-C18 alkenyl having one C-C double bond.

As an example of a suitable lipidoid compound that can be used as an ionizable lipidoid in the context of the invention, reference can be made to the cationic lipidoid dL_05(R) with the following structure:

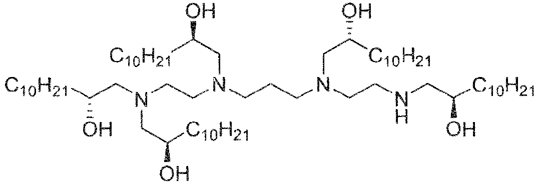

In accordance with a further exemplary embodiment, component (b) comprises or consists of an ionizable lipid of formula (b-2) 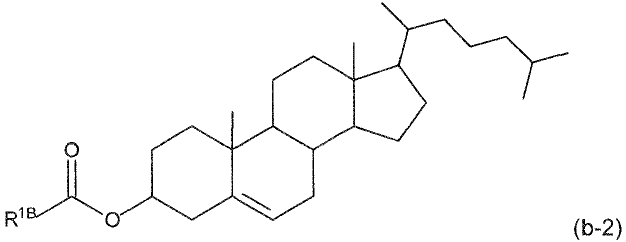 wherein R^{1B} is an organic group comprising one or more primary, secondary or tertiary amino groups,
or a protonated form thereof, wherein one or more of the nitrogen atoms contained in the primary, secondary or tertiary amino groups comprised by R^{1B} are protonated to provide a compound carrying a positive charge.

Preferably, the compound of formula (b-2) has the following structure:

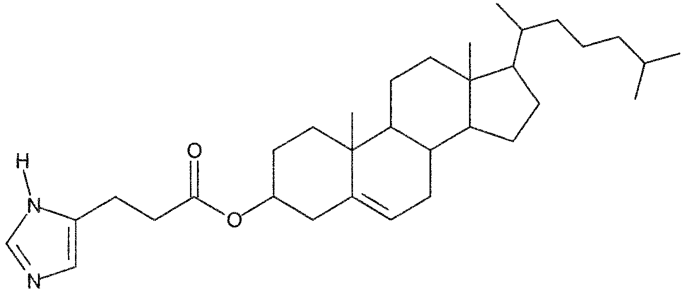

In accordance with another exemplary embodiment, component (b) comprises or consists of an ionizable lipid of formula (b-3) 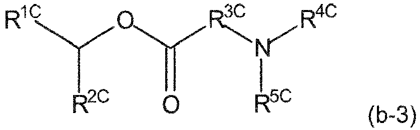 wherein
R^{1C} and R^{2C} are independently selected from a C8-C18 alkyl group and a C8-C18 alkenyl group, preferably from a C12-C18 alkyl group and a C12-C18 alkenyl group,
R^{3C} is a C1-C6 alkanediyl group, preferably a C2 or C3 alkanediyl group, and
R^{4C} and R^{5C} are independently hydrogen or C1-C3 alkyl, and are preferably methyl;
or a protonated form thereof, wherein one or more of the nitrogen atoms contained in the compound of formula (b-3) are protonated to provide a compound carrying a positive charge. As an example of an ionizable lipid of formula (b-3), reference can be made to DLin-MC3-DMA (6Z,9Z,28Z,31Z)-heptatriaconta-6,9,28,31-tetraene-19-yl 4-(dimethylamino)butanoate).

In accordance with still another exemplary embodiment, component (b) comprises or consists of an ionizable lipid of formula (b-4) 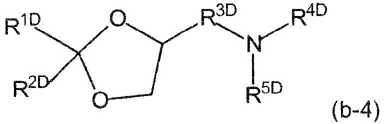 wherein
R^{1D} and R^{2D} are independently selected from a C8-C18 alkyl group and a C8-C18 alkenyl group, preferably from a C12-C18 alkyl group and a C12-C18 alkenyl group,
R^{3D} is a C1-C6 alkanediyl group, preferably a C2 alkanediyl groupy, and
R^{4D} and R^{5D} are independently hydrogen or C1-C3 alkyl, and are preferably methyl;
or a protonated form thereof, wherein one or more of the nitrogen atoms contained in the compound of formula (b-4) are protonated to provide a compound carrying a positive charge.

In accordance with still another exemplary embodiment, component (b) comprises or consists of an ionizable lipidoid of formula (b-5) 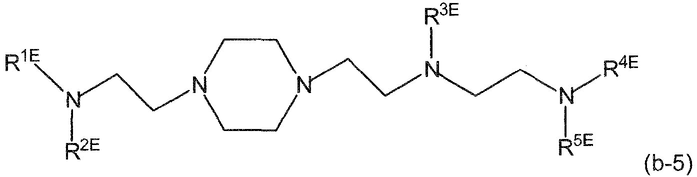
wherein R^{1E} to R^{5E} are independently of each other selected from hydrogen, -CH₂-CH(OH)-R^{7E}, -CH(R^{7E})-CH₂-OH,
-CH₂-CH₂-(C=O)-O-R^{7E}, -CH₂-CH₂-(C=O)-NH-R^{7E} and -CH₂-R^{7E} wherein R^{7E} is selected from C3-C18 alkyl or C3-C18 alkenyl having one C-C double bond, provided that at least two residues among R^{1E} to R^{5E} are selected from -CH₂-CH(OH)-R^{7E}, -CH(R^{7E})-CH₂-OH, -CH₂-CH₂-(C=O)-O-R^{7E}, -CH₂-CH₂-(C=O)-NH-R^{7E} and -CH₂-R^{7E} wherein R^{7E} is selected from C3-C18 alkyl or C3-C18 alkenyl having one C-C double bond;
or a protonated form thereof, wherein one or more of the nitrogen atoms contained in the compound of formula (b-5) are protonated to provide a compound carrying a positive charge.

In formula (b-5), R^{1E} to R^{5E} are preferably independently -CH₂-CH(OH)-R^{7E}, wherein R^{7E} is selected from C8-C18 alkyl or C8-C18 alkenyl having one C-C double bond.

Still another exemplary ionizable lipid suitable for use in the present invention which may be comprised in component (b) or of which component (b) may consist is the ionizable lipid disclosed as "cationic lipid of Formula I" in the PCT application WO 2012/000104 A1, starting on page 104 of this document, and including all specific embodiments thereof also discussed in this document.

Further exemplary ionizable lipidoids suitable for use in the present invention which may be comprised in component (b) or of which component (b) may consist are the ionizable lipidoids disclosed and claimed as "aminoalcohol lipidoids" in the PCT application WO 2010/053572 A2, including the compounds of all of the general formulae shown in the summary of the invention on page 4 of the document, and further defined in the remaining application.

Still further exemplary ionizable lipidoids suitable for use in the present invention which may be comprised in component (b) or of which component (b) may consist are the ionizable lipidoids disclosed as amine containing lipidoids of formulae I to V in the PCT application WO 2014/028487 A1, including specific embodiments thereof.

A further preferred example of an ionizable lipid suitable for use in the present invention which may be comprised in component (b) or of which component (b) may consist is the ionizable lipid ((4-hydroxybutyl)azanediyl)bis(hexan-6,1-diyl)bis(2-hexyldecanoate) (ALC-0315) or a protonated form thereof, wherein the nitrogen atom of the compound is protonated to provide a compound carrying a positive charge.

Still a further preferred example of an ionizable lipid suitable for use in the present invention which may be comprised in component (b) or of which component (b) may consist is the ionizable lipid (6Z,9Z,28Z,31Z)-heptatriaconta-6,9,28,31-tetraene-19-yl 4-(dimethylamino)butanoate or a protonated form thereof wherein the nitrogen atom of the compound is protonated to provide a compound carrying a positive charge.

Yet a further preferred example of an ionizable lipid suitable for use in the present invention which may be comprised in component (b) or of which component (b) may consist is the ionizable lipid heptadecan-9-yl 8-((2-hydroxyethyl)(6-oxo-6-(undecyloxy)hexyl)amino)octanoate (SM-102) or a protonated form thereof, wherein the nitrogen atom of the compound is protonated to provide a compound carrying a positive charge.

As preferred optional components in addition to (a) the therapeutic agent, preferably a nucleic acid, and (b) at least one selected from a permanently cationic lipid, an ionizable lipid and an ionizable lipidoid, preferably the ionizable lipid or the ionizable lipidoid, the nanoparticles may comprise one or more of the following components (c1) to (c6):
(c1) a non-ionizable lipid having a sterol structure;
(c2) a phosphoglyceride lipid;
(c3) a PEG-conjugated lipid;
(c4) a polysarcosine-conjugated lipid;
(c5) a PASylated lipid; and
(c6) a cationic polymer.

As will be understood by the skilled reader, the possibility that the nanoparticles comprise one or more of the components (c1) to (c6) not only encompasses combinations among (c1) to (c6), but also combinations of different components of one type, e.g. two components (c2), or combinations of different components of one type with other components of (c1) to (c6).

Component (c1) is a lipid having a sterol structure. As such, suitable lipids are compounds which have a steroid core structure with a hydroxyl group at the 3-position of the A-ring.

An exemplary non-ionizable lipid having a sterol structure which may be comprised by component (c1) or of which component (c1) may consist has a structure of formula (c1-1) 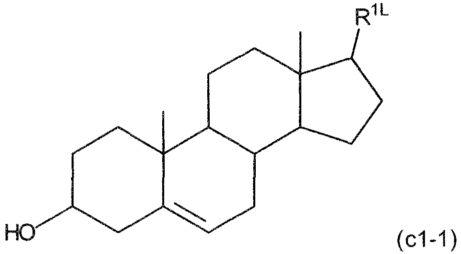 wherein R^{1L} is a C3-C12 alkyl group.

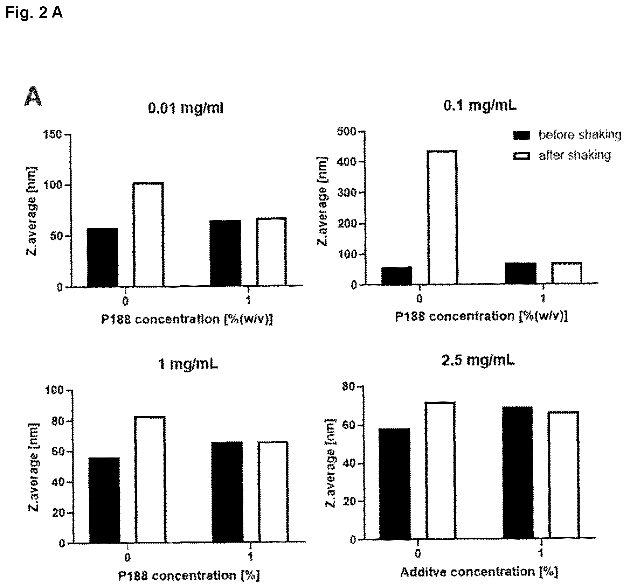

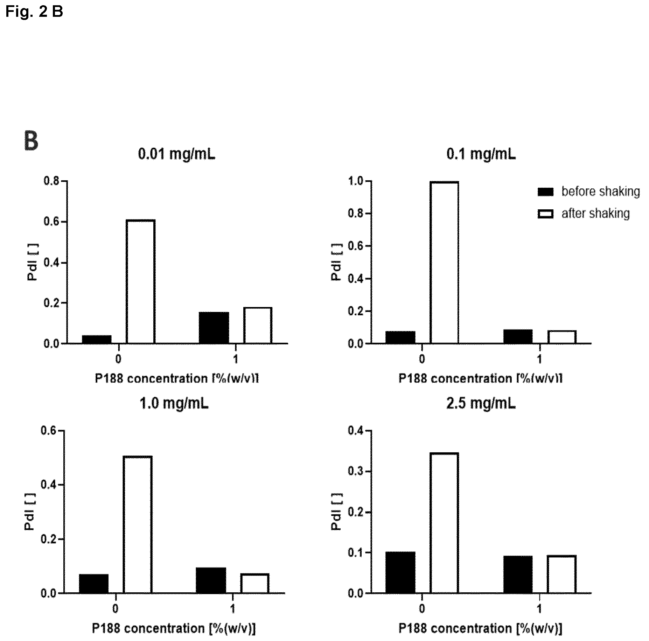

Further exemplary non-ionizable lipids having a sterol structure which may be comprised by component (c1) or of which component (c1) may consist include those disclosed by S. Patel et al., Naturally-occurring cholesterol analogues in lipid nanoparticles induce polymorphic shape and enhance intracellular delivery of mRNA, Nature Communications, 2020, 11:983, in particular those illustrated in Fig. 2 of the publication.

Preferably, component (c1) comprises or consists of cholesterol.

Component (c2) is a phosphoglyceride.

Preferably, component (c2) comprises or consists of a phospholipid selected from a compound of formula (c2-1) 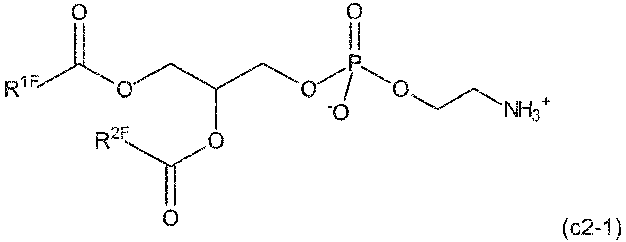 wherein
R^{1F} and R^{2F} are independently selected from a C8-C18 alkyl group and a C8-C18 alkenyl group, preferably from a C12-C18 alkyl group and a C12-C18 alkenyl group,
or a pharmaceutically acceptable salt thereof;
and a phospholipid of formula (c2-2) 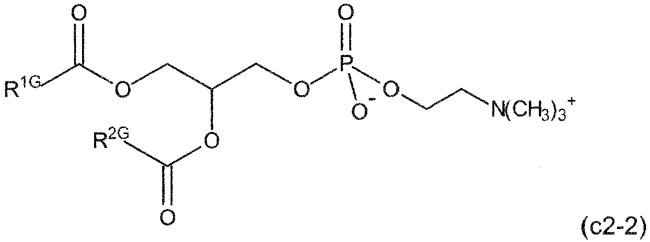 wherein
   R^{1G} and R^{2G} are independently selected from a C8-C18 alkyl group and a C8-C18 alkenyl group, preferably from a C12-C18 alkyl group and a C12-C18 alkenyl group,
or a pharmaceutically acceptable salt thereof.

More preferably, component (c2) comprises or consists of 1,2-dipalmitoyl-sn-glycero-3-phosphocholine (DPPC) or a pharmaceutically acceptable salt thereof or 1,2-Distearoyl-sn-glycero-3-phosphocholine (DSPC) or a pharmaceutically acceptable salt thereof.

Exemplary salt forms of the compound of formula (c2-1) include salts formed by the acidic - OH group with a base, or salts formed by the amino group with an acid. As salts formed with a base, mention may be made of alkali metal salts such as sodium or potassium salts; alkaline-earth metal salts such as calcium or magnesium salts and ammonium salts. As exemplary salts formed with an acid, mention may be made of a salt formed with the acidic groups of the nucleic acid, but other salts are not excluded, and mineral acid salts such as chloride, bromide, or iodide, sulfate salts, nitrate salts, phosphate salts, hydrogenphosphate salts, or dihydrogenphosphate salts, carbonate salts, and hydrogencarbonate salts may be mentioned as examples.

Exemplary salt forms of the compound of formula (c2-2) include salts formed by the acidic - OH group attached to the P atom with a base, or salts formed by the quaternary amino group with an anion. As salts formed with a base, mention may be made of alkali metal salts such as sodium or potassium salts; alkaline-earth metal salts such as calcium or magnesium salts and ammonium salts. As exemplary salts formed with anion, mention may be made of a salt formed with the acidic groups of the nucleic acid, but other salts are not excluded, and mineral acid salts such as chloride, bromide, or iodide, sulfate salts, nitrate salts, phosphate salts, hydrogenphosphate salts, or dihydrogenphosphate salts, carbonate salts, and hydrogencarbonate salts may be mentioned as examples.

Component (c3) is a PEG-conjugated lipid, i.e. a lipid which is covalently linked with a polyethylene glycol chain.

Preferably, component (c3) comprises or consists of a PEG-conjugated lipid selected from a compound of formula (c3-1) 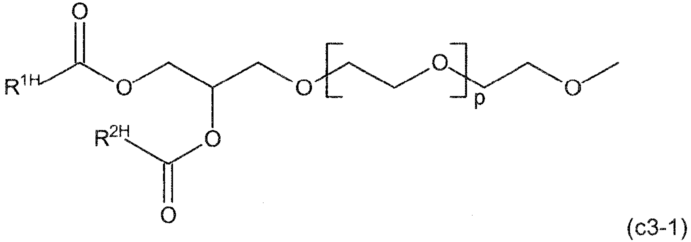 wherein
R^{1H} and R^{2H} are independently selected from a C8-C18 alkyl group and a C8-C18 alkenyl group, preferably from a C12-C18 alkyl group and a C12-C18 alkenyl group, and p is an integer of 5 to 200, preferably 10 to 100, and more preferably 20 to 60;
a compound of formula (c3-2) 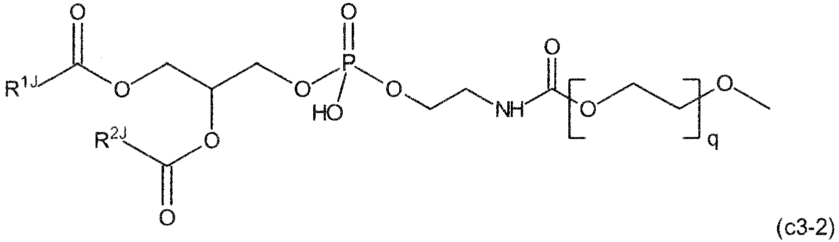 wherein
   R^{1J} and R^{2J} are independently selected from a C8-C18 alkyl group and a C8-C18 alkenyl group, preferably from a C12-C18 alkyl group and a C12-C18 alkenyl group, and q is an integer of 5 to 200, preferably 10 to 100, and more preferably 20 to 60 or a pharmaceutically acceptable salt thereof,
or a compound of formula (c3-3) 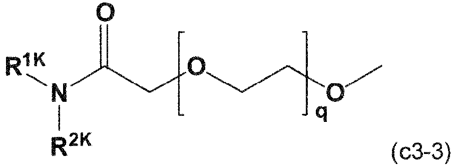 wherein
   R^{1K} and R^{2K} are independently a C8-C18 alkyl group or a C8-C18 alkenyl group, preferably a C12-C18 alkyl group or a C12-C18 alkenyl group, and q is an integer of 5 to 200, preferably 10 to 100, and more preferably 20 to 60.

Exemplary salt forms of the compound of formula (c3-2) include salts formed by the acidic - OH group attached to the P atom with a base. As salts formed with a base, mention may be made of alkali metal salts such as sodium or potassium salts; alkaline-earth metal salts such as calcium or magnesium salts and ammonium salts.

More preferably, component (c3) comprises or consists of 1,2-dimyristoyl-sn-glycerolmethoxy(polyethylene glycol) (DMG-PEG), and still more preferably component d) comprises or consists of 1,2-dimyristoyl-sn-glycerolmethoxy(polyethylene glycol)-2000 (DMG-PEG2k) or 2-[(polyethylenglycol)-2000]-N,N-ditetradecylacetamid (ALC-0159).

Component (c4) is a polysarcosine-conjugated lipid, i.e. a lipid which is covalently linked with a polymeric moiety of the formula (c4-1):

-[C(O)-CH₂-N(CH₃)]ᵣ- (c4-1)

wherein r denotes the number of repeating units, and is preferably 10 to 100.

Component (c5) is a PASylated lipid, e.g. a lipid which is covalently linked with a polymeric moiety formed by proline (pro)/alanine (ala)/serine (ser) repetitive residues.

With regards to the PASylated lipid used herein, the content of WO 2017/109087 A1 and EP 3394266 B1 is incorporated herein by reference. In particular the definitions and embodiments as recited below as incorporated herein by reference, specifically the embodiments reciting a nucleic acid coding for a PAS polypeptide. The PASylated lipid can comprise e.g.a polypeptide consisting of at least 100 amino acid residues of proline, alanine and, optionally, serine, wherein said polypeptide forms a random coil.

Component (c6) is a cationic polymer. Such polymers suitable for use in the formation of nanoparticles comprising a nucleic acid are known in the art. Exemplary suitable cationic polymers are discussed in A.C. Silva et al., Current Drug Metabolism, 16, 2015, 3-16, and in the literature referred to therein, in J.C. Kasper et al., J. Contr. Rel. 151 (2011), 246-255, in WO 2014/207231 and in the literature referred to therein, and in WO 2016/097377 and in the literature referred to therein.

Suitable cationic oligomers or polymers include in particular cationic polymers comprising a plurality of units wherein an amino group is contained. The amino groups may be protonated to provide the cationic charge of the polymer.

Polymers are preferred which comprise a plurality of units independently selected from the following (1), (2), (3) and (4):

-CH₂-CH₂-NH- (1)

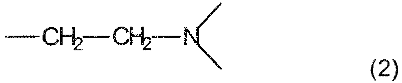

-CH₂-CH₂-CH₂-NH- (3)

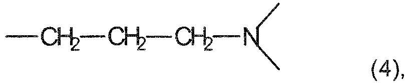

wherein one or more of the nitrogen atoms of the repeating units (1), (2), (3) and/or (4) may be protonated to provide the cationic charge of the polymer.

Particularly preferred as cationic polymers are the following four classes of polymers comprising a plurality of units wherein an amino group is contained.

As the first preferred class, poly(ethylene imine) ("PEI") is mentioned, including branched poly(ethylene imine) ("brPEI").

The second preferred class of cationic polymers are polymers comprising a plurality of groups of the following formula (c6-1) as a side chain and/or as a terminal group, as they are disclosed as groups of formula (II) in WO 2014/207231 (applicant ethris GmbH): 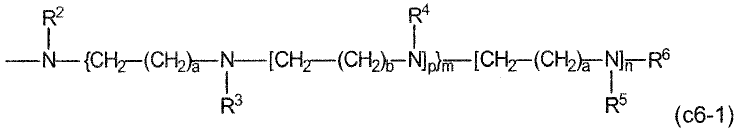 wherein the variables a, b, p, m, n and R² to R⁶ are defined as follows, independently for each group of formula (c6-1) in a plurality of such groups:
a is 1 and b is an integer of 2 to 4; or a is an integer of 2 to 4 and b is 1,
p is 1 or 2,
m is 1 or 2; n is 0 or 1 and m+n is ≥ 2; and
R² to R⁵ are, independently of each other, selected from hydrogen; a group -CH₂-CH(OH)-R⁷,-CH(R⁷)-CH₂-OH, -CH₂-CH₂-(C=O)-O-R⁷, -CH₂-CH₂-(C=O)-NH-R⁷ or -CH₂-R⁷ wherein R⁷ is selected from C3-C18 alkyl or C3-C18 alkenyl having one C-C double bond; a protecting group for an amino group; and a poly(ethylene glycol) chain;
R⁶ is selected from hydrogen; a group -CH₂-CH(OH)-R⁷, -CH(R⁷)-CH-OH, -CH₂-CH₂-(C=O)-O-R⁷, -CH₂-CH₂-(C=O)-NH-R⁷ or -CH₂-R⁷ wherein R⁷ is selected from C3-C18 alkyl or C3-C18 alkenyl having one C-C double bond; a protecting group for an amino group; -C(NH)-NH₂; a poly(ethylene glycol) chain; and a receptor ligand,
and wherein one or more of the nitrogen atoms indicated in formula (c6-1) may be protonated to provide a cationic group of formula (c6-1).

As regards further preferred definitions of these polymers, and of the variables contained in formula (c6-1) above, the respective disclosure in WO 2014/207231 with regard to its groups of formula (II) also applies for the invention described herein.

The third preferred class of cationic polymers are polymers comprising a plurality of groups of the following formula (c6-2) as repeating units, as they are disclosed as groups of formula (III) in WO 2014/207231 (applicant ethris GmbH): 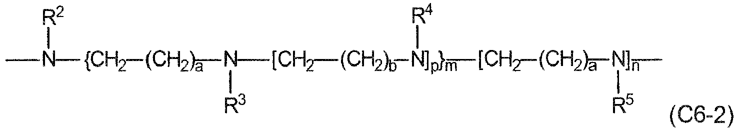 wherein the variables a, b, p, m, n and R² to R⁵ are defined as follows, independently for each group of formula (c6-2) in a plurality of such groups:
a is 1 and b is an integer of 2 to 4; or a is an integer of 2 to 4 and b is 1,
p is 1 or 2,
m is 1 or 2; n is 0 or 1 and m+n is ≥ 2; and
R² to R⁵ are, independently of each other, selected from hydrogen; a group
-CH₂-CH(OH)-R⁷, -CH(R⁷)-CH₂-OH, -CH₂-CH₂-(C=O)-O-R⁷, -CH₂-CH₂-(C=O)-NH-R⁷ or -CH₂-R⁷, wherein R⁷ is selected from C3-C18 alkyl or C3-C18 alkenyl having one C-C double bond; a protecting group for an amino group; -C(NH)-NH₂; and a poly(ethylene glycol) chain;
and wherein one or more of the nitrogen atoms indicated in formula (c6-2) may be protonated to provide a cationic group of formula (c6-2).

As regards further preferred definitions of these polymers, and of the variables contained in formula (c6-2) above, the respective disclosure in WO 2014/207231 with regard to its repeating units of formula (III) also applies for the invention described herein.

The fourth preferred class of cationic polymers is provided by a statistical copolymer as it is disclosed in WO 2016/097377 (applicant ethris GmbH). It comprises a plurality of repeating units (a) independently selected from repeating units of the following formulae (a1) and (a2):

-CH₂-CH₂-NH- (a1)

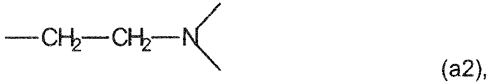

and a plurality of repeating units (b) independently selected from repeating units of the following formulae (b1) to (b4):

-CH₂-CH₂-CH₂-NH- (b1)

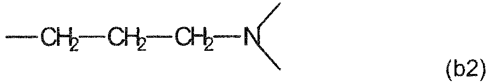

-CH₂-CH₂-CH₂-CH₂-NH- (b3)

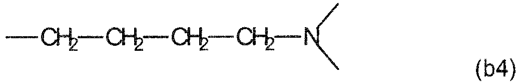

and the molar ratio of the sum of the repeating units (a) to the sum of the repeating units (b) lies within the range of 0.7/1.0 to 1.0/0.7, and one or more of the nitrogen atoms of the repeating units (a) and/or (b) contained in the copolymer may be protonated to provide a cationic copolymer.

As regards further preferred definitions of this copolymer, the respective disclosure in WO 2016/097377 also applies for the invention described herein. As noted therein, a particularly preferred copolymer is a linear copolymer which comprises repeating units (a1) and (b1), or which consists of repeating units (a1) and (b1).

As an optional component of the nanoparticles, a polyanion component which is different from a nucleic acid may also be comprised, especially in addition to a nucleic acid if the nanoparticles comprise a nucleic acid as the preferred therapeutic agent . Examples of such a polyanion are polyglutamic acid and chondroitin sulfate. If such a polyanion component different from the nucleic acid is used in the nanoparticles, its amount is preferably limited such that the amount of anionic charges provided by the polyanion component is not higher than the amount of the anionic charges provided by the nucleic acid.

As explained above, the lipid or lipidoid nanoparticles which are suspended in the aqueous vehicle solution comprise (a) a therapeutic agent, which is preferably a nucleic acid and (b) at least one selected from a permanently cationic lipid, an ionizable lipid and an ionizable lipidoid, and preferably an ionizable lipid or an ionizable lipidoid. If a lipidoid is comprised, the nanoparticles shall be referred to herein as lipidoid nanoparticles.

Preferably, the nanoparticles comprise, more preferably consist of,
a nucleic acid as the therapeutic agent (a),
the at least one selected from a permanently cationic lipid, an ionizable lipid and an ionizable lipidoid, preferably the ionizable lipid or ionizable lipidoid (b),
and optionally one or more of
the non-ionizable lipid having a sterol structure (c1);
the phosphoglyceride lipid (c2);
the PEG-conjugated lipid (c3);
the polysarcosine-conjugated lipid (c4);
the PASylated lipid (c5);
the cationic polymer (c6).

Exemplary suspensions comprising nanoparticles formed from the components listed above, which are also suitable for use in the context of the present invention, include those disclosed by S. Patel et al., Naturally-occurring cholesterol analogues in lipid nanoparticles induce polymorphic shape and enhance intracellular delivery of mRNA, Nature Communications, 2020, 11:983.

It will be understood that components of the nanoparticles, and in particular components (a) and (b), and optionally one or more of (c1) to (c6), are typically contained as a mixture in the nanoparticles.

In terms of the amounts of these components, it is further preferred that the nanoparticles comprise, more preferably consist of:
the therapeutic agent, which is preferably a nucleic acid, and
30 to 65 mol% of the at least one selected from a permanently cationic lipid, an ionizable lipid and an ionizable lipidoid, preferably of the ionizable lipid or ionizable lipidoid (b),
and one or more of the following components:
   10 to 50 mol% of the lipid having a sterol structure (c1),
   4 to 50 mol% of the phosphoglyceride lipid (c2),
   0.5 to 10 mol% of one of the PEG-conjugated lipid (c3), the polysarcosine-conjugated lipid (c4) and the PASylated lipid (c5), or of any combination thereof,
   0.5 to 10 mol% of the cationic polymer (c6),
   such that the sum of (b) and (c1) to (c6) amounts to 100 mol%.

As regards the 30 to 65 mol% of the at least one selected from a permanently cationic lipid, an ionizable lipid and an ionizable lipidoid as component (b), it will be understood that the indicated molar percentage refers to the total amount of these constituents of the nanoparticle if two or more of the cationic lipid, an ionizable lipid and an ionizable lipidoid are present as component (b). Likewise, it will be understood that the molar percentages for components (c1) to (c6) are indicated with the proviso that not all of these components need to be present in the nanoparticles. Thus, for example, the cationic polymer can be present or absent in the context of this preferred embodiment, but if it is present, it is used in the amount of 0.5 to 10 mol%. As further indicated above, the amount of component(s) (c1), (c2), (c3), (c4), (c5) and/or (c6) in the context of this preferred embodiment is such that the sum of (b) and (c1) to (c6) amounts to 100 mol%.

It is still further preferred that the nanoparticles comprise, or consist of
the nucleic acid (a),
the at least one selected from a permanently cationic lipid, an ionizable lipid and an ionizable lipidoid, preferably the ionizable lipid or ionizable lipidoid (b),
the non-ionizable lipid having a sterol structure (c1),
the phosphoglyceride lipid (c2), and
the PEG-conjugated lipid (c3).

In terms of the amounts of these components, it is still further preferred that the nanoparticles comprise, more preferably consist of:
the nucleic acid (a),
30 to 65 mol% of the at least one selected from a permanently cationic lipid, an ionizable lipid and an ionizable lipidoid, preferably of the ionizable lipid or ionizable lipidoid (b),
10 to 50 mol% of the lipid having a sterol structure (c1),
4 to 50 mol% of the phosphoglyceride lipid (c2), and
0.5 to 10 mol% of the PEG-conjugated lipid (c3),
such that the sum of (b) and (c1) to (c3) amounts to 100 mol%.

In line with the above information related to preferred therapeutic agents, in particular nucleic acids and related to the preferred components of the lipid composition other than the therapeutic agent, the lipidoid nanoparticles in the context of the present invention preferably comprise
(a) mRNA as a nucleic acid;
(b) an ionizable lipidoid of formula (b-1b) 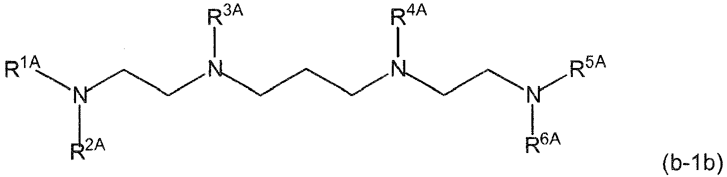
   wherein R^{1A} to R^{6A} are independently selected from hydrogen and -CH₂-CH(OH)-R^{7A}, wherein R^{7A} is selected from C8-C18 alkyl and C8-C18 alkenyl having one C-C double bond, provided that at least two residues among R^{1A} to R^{6A} are -CH₂-CH(OH)-R^{7A}, more preferably at least four residues among R^{1A} to R^{6A} are -CH₂-CH(OH)-R^{7A}, wherein R^{7A} is selected from C8-C18 alkyl and C8-C18 alkenyl having one C-C double bond;
   or a protonated form thereof wherein one or more of the nitrogen atoms indicated in formula (b-1b) are protonated to provide a cationic lipidoid;
(c1) a non-ionizable lipid having a sterol structure of formula (c1-1) 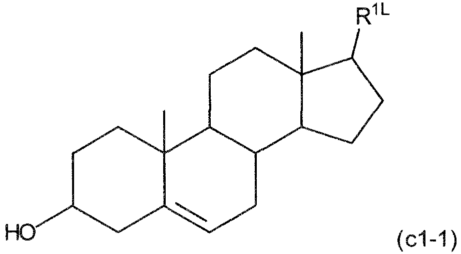 wherein R^{1L} is a C3-C12 alkyl group;
(c2) a phosphoglyceride of formula (c2-2) 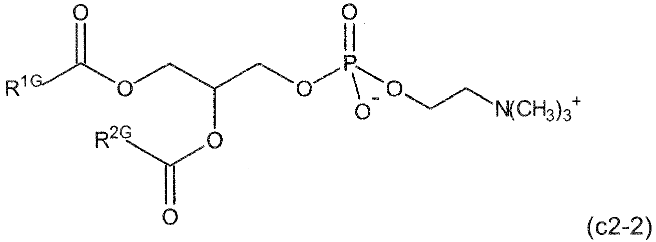
   wherein R^{1G} and R^{2G} are independently selected from a C8-C18 alkyl group and a C8-C18 alkenyl group, preferably from a C12-C18 alkyl group and a C12-C18 alkenyl group,
   or a pharmaceutically acceptable salt thereof; and
(c3) a PEG conjugated lipid of formula (c3-1) 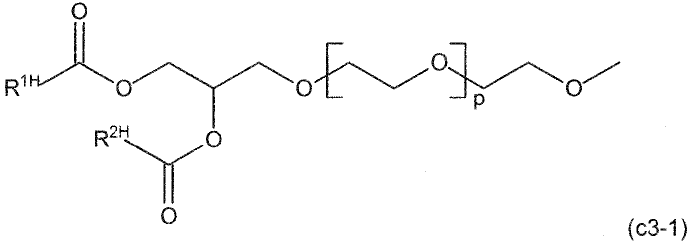
wherein R^{1H} and R^{2H} are independently selected from a C8-C18 alkyl group and a C8-C18 alkenyl group, preferably from a C12-C18 alkyl group and a C12-C18 alkenyl group, and p is an integer of 5 to 200, preferably 10 to 100, and more preferably 20 to 60. In such a lipidoid particle composition, the lipidoid dL_05(R) with the formula shown above would be a particularly preferred variant of the ionizable lipid.

Another preferred exemplary composition of a lipid nanoparticle suitable for use in the context of the present invention comprises a nucleic acid, more preferably mRNA, as a therapeutic agent, the ((4-hydroxybutyl)azandiyl)bis(hexan-6,1-diyl)bis(2-hexyldecanoate) or a protonated form thereof wherein the nitrogen atom of the compound is protonated to provide a compound carrying a positive charge as a ionizable lipidoid (b), and optionally further comprise one or more of the following components (d1) to (d8):
(d1) 2-[(polyethylene glycol)-2000]-N,N-ditetradecylacetamide (ALC-0159)
(d2) 1,2-distearoyl-sn-glycero-3-phosphocholine (DSPC)
(d3) cholesterol
(d4) potassium chloride
(d5) potassium dihydrogen phosphate
(d6) sodium chloride
(d7) disodium phosphate dihydrate
(d8) sucrose.

More preferably, they further comprise at least (d1), (d2) and (d3), and still more preferably they comprise all of (d1) to (d8).

Still another preferred exemplary composition of a lipid nanoparticle suitable for use in the context of the present invention comprises a nucleic acid, more preferably mRNA, as a therapeutic agent, heptadecan-9-yl 8-((2-hydroxyethyl)(6-oxo-6-(undecyloxy)hexyl)amino)octanoate (SM-102) or a protonated form thereof wherein the nitrogen atom of the compound is protonated to provide a compound carrying a positive charge as an ionizable lipid (b), and optionally comprise one or more of the following components (e1) to (e7):
(e1) 1,2-distearoyl-sn-glycero-3-phosphocholine (DSPC),
(e2) cholesterol,
(e3) 1,2-dimyristoyl-rac-glycero-3-methoxypolyethylene glycol-2000 (PEG2000 DMG),
(e4) tromethamol hydrochloride
(e5) sodium acetate trihydrate
(e6) acetic acid
(e7) sucrose.

More preferably, they further comprise at least (e1), (e2) and (e3), and still more preferably they comprise all of (e1) to (e7).

Still another preferred exemplary composition of a lipid nanoparticle suitable for use in the context of the present invention comprises a nucleic acid, more preferably mRNA, as a therapeutic agent, DLin-MC3-DMA ((6Z,9Z,28Z,31Z)-heptatriaconta-6,9,28,31-tetraen-19-yl-4-(dimethylamino) butanoate) or a protonated form thereof wherein the nitrogen atom of the compound is protonated, and optionally one or more of the following components (e1) to (e7):
(e1) 1,2-distearoyl-sn-glycero-3-phosphocholine (DSPC),
(e2) cholesterol,
(e3) PEG2000-C-DMG (α-(3'-{[1,2-di(myristyloxy)propanoxy]carbonylamino}propyl)-ω-methoxy, polyoxyethylene),
(e4) 2-amino-2-(hydroxymethyl)propan-1,3-diol (tromethamol) hydrochloride
(e5) Disodium hydrogen phosphate, heptahydrate
(e6) Potassium dihydrogen phosphate, anhydrous
(e7) Sodium chloride

More preferably, components (e1), (e2) and (e3) are present, and still more preferably they comprise all of (e1) to (e6).

The composition of the nanoparticles is preferably such that the weight ratio in the nanoparticles of the sum of the weights of components other than the nucleic acid to the weight of the nucleic acid is in the range of 50:1 to 1:1, more preferably 40:1 to 2:1 and most preferably 30:1 to 3:1.

The N/P ratio, i.e. the ratio of the number of amine nitrogen atoms provided by the ionizable lipid or the ionizable lipidoid to the number of phosphate groups provided by the nucleic acid of the nanoparticles, if a nucleic acid is comprise as a therapeutic agent, is preferably in the range of 0.5 to 20, more preferably in the range of 0.5 to 10.

The suspended lipid or lipidoid nanoparticles preferably have a Z-average diameter in the range of 10 to 500 nm, more preferably in the range of 10 to 250 nm, still more preferably 20 to 200 nm. The indicated particle diameter is the hydrodynamic diameter of the particles, as determined by dynamic light scattering (DLS). Measurements are generally carried out at 25 °C. Due to the stabilizing effect of the nonionic surfactant, this average diameter can be maintained even after exposure of the suspension to a physical stress condition.

The polydispersity index of the suspended nanoparticles is preferably in the range of 0.02 to 0.4, more preferably in the range of 0.03 to 0.2. The polydispersity index can be determined by dynamic light scattering (DLS). Measurements are generally carried out at 25 °C. Due to the stabilizing effect of the nonionic surfactant, this polydispersity index can be maintained even after exposure of the suspension to a physical stress condition.

It is possible to provide a suspension containing different lipid or lipidoid nanoparticles as defined above, i.e. particles which differ in terms of their components. However, preferably the nanoparticles contained in the suspension are composed of the same components.

The nanoparticles can be conveniently prepared by mixing a solution containing the nucleic acid, e.g. in an aqueous solvent containing a buffer, such as a citrate buffer with a pH of 4.5, and optionally containing a salt such as sodium chloride, and a solution containing the ionizable lipid or ionizable lipidoid in an organic solvent, e.g. in ethanol. Further optional components can be incorporated e.g. by adding them to one of the two solutions. The nanoparticles generated in this manner can be further processed by chromatography and/or dialysis and/or tangential flow filtration (TFF) in order to obtain the nanoparticles in a desired liquid composition. Preferably, they are further processed using TFF.

In order to provide the nanoparticle suspension, it is also possible to rely on lyophilized nanoparticles prepared following the above-mentioned procedure followed by freeze drying, which are subsequently re-suspended in an aqueous vehicle solution.

In the stable suspension provided by the various aspects of the invention, the nanoparticles are suspended in an aqueous vehicle solution.

The vehicle solution is an aqueous solution, i.e. a solution wherein the main solvent, in terms of the total volume of solvent(s), is water, preferably a solution containing more than 70 % of water, more preferably more than 90 % of water, as a solvent, indicated as the volume percentage of water in the total volume of solvent(s) contained in the vehicle solution (at a temperature of 25 °C). Most preferably, water is the only solvent in the vehicle solution. Thus, the vehicle solution is a liquid at room temperature (e.g. 25 °C).

The weight per volume ratio of the nanoparticles in the vehicle solution is preferably in the range 0.1 g/L to 300 g/L, more preferably 0.2 g/L to 300 g/L, still more preferably 0.5 g/L to 250 g/L and most preferably 0.5 g/L to 125 g/L (as measured at 25 °C).

If the nanoparticles comprise a nucleic acid as a therapeutic agent, the concentration of the nucleic acid provided by the lipid or lipidoid nanoparticles in the suspension preferably ranges from 0.01 to 10 mg/ml, more preferably from 0.02 to 10 mg/ml, still more preferably from 0.05 to 5 mg/mL, and most preferably from 0.05 to 2.5 mg/ml, based on the total volume of the suspension (as measured at 25 °C).

As noted above, the lipid or lipidoid nanoparticles contained in the suspension preferably have a Z-average diameter in the range of 10 to 500 nm, more preferably in the range of 10 to 250 nm, still more preferably 20 to 200 nm. The indicated particle diameter is the hydrodynamic diameter of the particles, as determined by dynamic light scattering (DLS). Measurements are generally carried out at 25 °C.

The polydispersity index of the nanoparticles contained in the suspension is preferably in the range of 0.02 to 0.4, more preferably in the range of 0.03 to 0.2. The polydispersity index can be determined by dynamic light scattering (DLS). Measurements are generally carried out at 25 °C.

By using a surfactant, preferably a nonionic surfactant, the nanoparticle suspension is stabilized in the context of the invention against particle aggregation under a physical stress condition. To achieve this effect, the surfactant is incorporated into the suspension, preferably incorporated as an excipient into the aqueous vehicle solution.

In some embodiments, the LNPs and/or LiNPs have not been lyophilized. In some embodiments, the surfactant is added before a lyophilization process. In some embodiments, the surfactant is not present in the vehicle solution during a lyophilization process.

Typically, the presence of the surfactant does not cause a change in the biological activity of the nanoparticle. Biological activity means expression level in the target cell(s) of the therapeutic nucleic acid. The biological activity can e.g. be quantified by in vitro transfection of cell lines (e.g. HEK-293) with the nanoparticle followed by quantification of the produced nucleic acid by Southern/northern blot or of protein via an ELISA. The detected protein level calculated as average of three measurements per concentration must not differ more than 10%, preferably not more than 5%, more preferably is not statistically different when performing the identical assay with the same LNP or LiNP without a surfactant.

Typically, the presence of the surfactant does not cause a change in the physical properties of the nanoparticle measured as the hydrodynamic diameter of the nanoparticle and as the proportion of encapsulated nucleic acid.

The hydrodynamic diameter of the nanoparticle can e.g. be measured via dynamic light scattering (also Photon correlation spectroscopy). Optionally, the average of three measurements of the hydrodynamic diameter of the nanoparticle in presents of the surfactant must not differ more than 5%, preferably not more than 1%, more preferably not statistically different of the same nanoparticle in absence of the surfactant. The viscosity change of the surfactant must be taken into account during the measurement. The percentage of encapsulated nucleic acid can e.g. be determined by measuring the fluorescence intensity in a RiboGreen assay. The nanoparticle is analyzed under two different conditions, untreated samples for external nucleic acid and samples treated with Triton X-100 for total mRNA. The percent content of encapsulated nucleic acid is calculated. Optionally, the value calculated form an average of three measurements of the nanoparticle in absence of the surfactant should not differ more than 5%, optionally not more than 3%, better not statistically different) from the same nanoparticle in presents of the surfactant.

As will be understood by the skilled reader, a measure which is taken for the stabilization of a nanoparticle suspension against particle aggregation may prevent the aggregation of the nanoparticles, or may reduce the degree of aggregation of the nanoparticles compared to a situation where the concerned measure is not applied. Preferably, the stabilization of the nanoparticle suspension is evidenced by an increase of the Z-average particle size of the suspended particles under a physical stress condition of less than 50 %, more preferably less than 20 %, still more preferably less than 10 % and most preferably by the absence of such an increase.

Similarly, it will be understood that stabilization of the nanoparticle suspension against particle aggregation under a physical stress condition means that an aggregation of the nanoparticles is prevented or reduced which would be observed in the absence of the stabilization when the nanoparticle suspension is exposed to a physical stress condition.

Conditions of physical stress to which the nanoparticle suspension can be exposed are frequently physical stress conditions that are encountered during the handling or during a transport of the suspension. They include, e.g., a quick movement of a volume of the suspension which would cause a collision of nanoparticles contained in a non-stabilized suspension. As examples of a physical stress condition, reference may be made to shaking, stirring, vibrating, mixing, inverting, tapping, or dropping of the nanoparticle suspension, or, e.g., to a physical stress condition caused by pumping the nanoparticle suspension or by its withdrawal into a syringe. As will be understood by the skilled reader, conditions of physical stress include not only conditions to which the nanoparticle suspension is exposed during its regular handling, but also conditions to which the suspension may be exposed exceptionally (such as a transport under difficult conditions) or inadvertently (such as dropping a sample of the suspension).

Diverse types of surfactants can be used in the context of the present invention, and it is preferred that a nonionic surfactant is used to stabilize the suspension. Thus, it is preferred that the surfactant comprises, more preferably is, a nonionic surfactant. Examples of suitable nonionic surfactants include fatty alcohol ethoxylates, fatty acid ethoxylates, block copolymers of ethylene oxide and propylene oxide, alkylphenol ethoxylates or oligomers of alkylphenol ethoxylates, fatty acid esters of sorbitol, ethoxylated fatty acid esters of sorbitol, fatty acid esters of glycerol, ethoxylated castor oil and ethoxylated vitamin E.

Thus, the surfactant used in the context of the invention in the nanoparticle suspensions preferably comprises, still more preferably is, at least one selected from the group of fatty alcohol ethoxylates, fatty acid ethoxylates, block copolymers of ethylene oxide and propylene oxide, alkylphenol ethoxylates or oligomers of alkylphenol ethoxylates, fatty acid esters of sorbitol, ethoxylated fatty acid esters of sorbitol, fatty acid esters of glycerol, ethoxylated castor oil and ethoxylated vitamin E.

In accordance with another embodiment, the surfactant used in the context of the invention in the nanoparticle suspensions preferably comprises, still more preferably is, at least one selected from the group of fatty alcohol ethoxylates, fatty acid ethoxylates, alkylphenol ethoxylates or oligomers of alkylphenol ethoxylates, fatty acid esters of sorbitol, ethoxylated fatty acid esters of sorbitol, fatty acid esters of glycerol, ethoxylated castor oil and ethoxylated vitamin E.

Preferably, the block copolymer of ethylene oxide and propylene oxide is a poloxamer. The poloxamer is preferably one which contains one poly(propylene oxide) block B of formula (p-1): 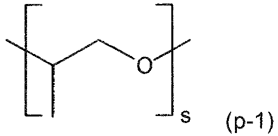
wherein s is an integer of 15 to 60, and
two poly(ethylene oxides) blocks A of formula (p-2): 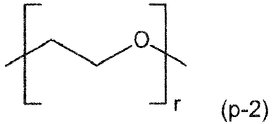
wherein r is, independently for each block, an integer of 8 to 150, preferably 10 to 150.

Thus, it is preferred that the surfactant comprises, more preferably is, a poloxamer, e.g. the preferred poloxamer as discussed above. However, the invention in its various aspects also provides and relates to suspensions which do not comprise a poloxamer.

It is particularly preferred that a nonionic surfactant used for the stabilization in accordance with the invention comprises, or preferably is, at least one selected from the group of poloxamer 124, poloxamer 188, poloxamer 338, poloxamer 407, polysorbate 20, polysorbate 80, polyoxyethylenelaurylether, poyloxyethylene-35 castor oil, D-α-tocopherol polyethylene glycol 1000 succinate, and Tyloxapol. In accordance with another embodiment, the nonionic surfactant used for the stabilization in accordance with the invention comprises, or is, at least one selected from the group of polysorbate 20, polysorbate 80, polyoxyethylenelaurylether, poyloxyethylene-35 castor oil, D-α-tocopherol polyethylene glycol 1000 succinate, and Tyloxapol.

The vehicle solution wherein the nanoparticles are suspended generally comprises the surfactant dissolved therein. As will be appreciated by the skilled reader, this does not exclude the possibility that a certain amount of the surfactant molecules is adsorbed to the lipid or lipidoid nanoparticles which are contained in the suspension.

In the context of the invention, it has been found that a beneficial effect of the surfactant can be achieved already with relatively low concentrations of a surfactant, e.g of 0.01 % (w/v) in the suspension. Thus, typically, the surfactant is contained in amounts of 0.01 % (w/v) or more in the suspension, with regard to the total volume of the suspension of the nanoparticles in the aqueous vehicle solution (typically measured at 25 °C).

For example, the use in accordance with the invention involves an incorporation of the surfactant into the nanoparticle suspension, preferably into the aqueous vehicle solution, in an amount of 0.01 to 10 % (w/v), preferably 0.1 to 10 % (w/v), more preferably 0.25 to 5 % (w/v), still more preferably 0.33 to 2.5 % (w/v), even more preferably 0.45 to 1.5 % (w/v), and most preferably 0.5 to 1.5 % (w/v), with regard to the total volume of the suspension of the nanoparticles in the aqueous vehicle solution. As will be appreciated, the indication of the concentration of a substance in % (w/v) or (weight/volume) corresponds to the amount of the substance in g in a volume of 100 mL, typically measured at 25 °C, so that 1 % (w/v) corresponds to 1 g the surfactant per 100 mL of the total volume of the suspension.

Likewise, it is the methods in accordance with the invention may involve the incorporation of the surfactant into the nanoparticle suspension in an amount of e.g. 0.01 to 10 % (w/v), preferably 0.1 to 10 % (w/v), more preferably 0.25 to 5 % (w/v), still more preferably 0.33 to 2.5 % (w/v), even more preferably 0.45 to 1.5 % (w/v), and most preferably 0.5 to 1.5 % (w/v), with regard to the total volume of the suspension of the nanoparticles in the aqueous vehicle solution (typically measured at 25 °C).

While concentrations of 0.5 to 1.5% (w/v) are particularly preferred, as outlined above, the invention in its various aspects also provides and relates to suspensions which wherein the concentration of the surfactant is lower, e.g. in the range of 0.01 to 0.45 % (w/v), or 0.1 to 0.40 % (w/v).

In the suspension of lipid nanoparticles or of lipidoid nanoparticles in an aqueous vehicle solution in the context of the various aspects of the invention, it is generally preferred that the surfactant is essentially not attached to the nanoparticles, e.g. that it is essentially not contained in the nanoparticles and essentially not adhering to the nanoparticles. For example, more than 90 wt%, preferably more than 95 wt% of the total amount of the surfactant which is contained in or incorporated into the suspension of lipid nanoparticles or of lipidoid nanoparticles in an aqueous vehicle solution in the context of the various aspects of the invention, is present in the aqueous vehicle solution without being attached to the nanoparticles.

In addition to the surfactant, other excipients may be present in the vehicle solution. Preferably, the vehicle solution further comprises at least one of a sugar and a salt, more preferably sucrose and NaCl.

The surfactant can be conveniently incorporated into the nanoparticle suspension, e.g. by a method including adding the surfactant to a suspension comprising an aqueous vehicle solution and the lipid or lipidoid nanoparticles, or including adding the lipid or lipidoid nanoparticles to an aqueous vehicle solution comprising the surfactant. For example, as noted above, if the nanoparticles are provided in lyophilized form, they can be re-suspended in an aqueous vehicle solution containing a surfactant.

To that extent, the invention also provides a method for the preparation of a suspension of lipid nanoparticles or lipidoid nanoparticles as defined herein, which comprises generating a preparation of lipid nanoparticles or lipidoid nanoparticles by mixing of at least one selected from a permanently cationic lipid, an ionizable lipid and an ionizable lipidoid dissolved in an organic phase with a therapeutic agent dissolved in an aqueous solution, and
combining the nanoparticles with a surfactant to obtain a suspension of the nanoparticles in an aqueous vehicle solution.

Preferably, the method comprises the following steps:
i) generating a preparation of lipid nanoparticles or lipidoid nanoparticles by mixing of at least one selected from a permanently cationic lipid, an ionizable lipid and an ionizable lipidoid dissolved in an organic phase with a therapeutic agent dissolved in an aqueous solution,
ii) diluting the preparation of lipid nanoparticles or lipidoid nanoparticles by dilution with a first solution,
iii) concentrating the diluted preparation of lipid nanoparticles or lipidoid nanoparticles by buffer exchange using ultra/diafiltration by TFF, wherein a second solution is used for the ultra/diafiltration,
iv) obtaining a suspension of lipid nanoparticles or lipidoid nanoparticles in an aqueous vehicle solution comprising a surfactant,

wherein the first solution comprises between 0.01% w/v and 10% w/v surfactant, preferably between 0.1% w/v and 10% surfactant, more preferably between 0.25% w/v surfactant and 5% w/v surfactant, still more preferably between 0.33% w/v surfactant and 2.5% w/v surfactant, even more preferably between 0.45% w/v surfactant and 1.5% w/v surfactant, most preferably between 0.5% w/v and 1.5% w/v surfactant, and/or
wherein the second solution comprises between 0.01% w/v and 10% w/v surfactant, preferably between 0.1% w/v and 10% surfactant, more preferably between 0.25% w/v surfactant and 5% w/v surfactant, still more preferably between 0.33% w/v surfactant and 2.5% w/v surfactant, even more preferably between 0.45% w/v surfactant and 1.5% w/v surfactant, most preferably between 0.5% w/v and 1.5% w/v surfactant,
and wherein the final concentration of surfactant from combined first and second solution is between 0.01% w/v and 10% w/v surfactant, preferably between 0.1% w/v and 10% surfactant, more preferably between 0.25% w/v surfactant and 5% w/v surfactant, still more preferably between 0.33% w/v surfactant and 2.5% w/v surfactant, even more preferably between 0.45% w/v surfactant and 1.5% w/v surfactant, most preferably between 0.5% w/v and 1.5% w/v surfactant, with regard to the total volume of the suspension of the nanoparticles in the aqueous vehicle solution.

In the above method, it is preferred that an incorporation of a surfactant into the suspension does not occur before or during step i).

Moreover, it is preferred that the surfactant is added both with the first and the second solution. For example, wherein 30 to 70 wt% of the surfactant, preferably 40 to 60 wt%, and more preferably 45 to 55 wt% of the surfactant, based on the total weight of the surfactant in the suspension obtained in step iv), can be added with the first solution, and 70 to 30 wt% of the surfactant, preferably 60 to 40 wt%, and more preferably 55 to 45 wt% of the surfactant, based on the total weight of the surfactant in the suspension obtained in step iv), can be added with the second solution, such that the sum of the amount of surfactant added with the first and the second solution is 100 wt%. Generally, it is preferred that approximately half of the surfactant is added with the first solution and approximately half of the surfactant is added with the second solution.

The therapeutic agent, preferably nucleic acid, such as RNA, preferably mRNA, which is present in the lipid or lipidoid nanoparticles used in the context of the present invention is particularly useful in a medical setting and in the treatment or prevention of diseases and disorders, preferably in the treatment or prevention of a disease or disorder relying on a nucleic acid as an active agent. Thus, the suspension is generally provided as or used as a medicament or as a pharmaceutical composition. The invention also provides a formulation of of lipid nanoparticles or lipidoid nanoparticles comprising a suspension of lipid nanoparticles or lipidoid nanoparticles in accordance with the invention as described herein.

In particular, the nanoparticle suspension or the formulation is suitable for administration to a subject. In this manner, the therapeutic agent, preferably the nucleic acid such as RNA, preferably the mRNA, contained in the nanoparticles of the suspension can also be administered to the subject.

Via administration to a subject, the therapeutic agent, preferably the nucleic acid contained in the lipid or lipidoid nanoparticles particles may be delivered to target cells. The term "delivered to target cells" preferably means transfer of the nucleic acid into the cell. The administration can be accomplished in various ways known to the skilled practitioner, including an administration to or via the respiratory tract, e.g. by an aerosolization of the suspension, or an intramuscular or intravenous administration.

By administering the suspension to a subject, diseases or disorders can be treated or prevented. The term "disease" refers to any conceivable pathological condition that can be treated, prevented or vaccinated against by employing the suspension Said diseases may, e.g., be inherited, acquired, infectious or non-infectious, age-related, cardiovascular, metabolic, intestinal, neoplastic (in particular cancer) or genetic. A disease can be based, for example, on irregularities of physiological processes, molecular processes, biochemical reactions within an organism that in turn can be based, for instance, on the genetic equipment of an organism, on behavioural, social or environmental factors such as the exposure to chemicals or radiation.

To that extent, the invention also provides the suspension of lipid nanoparticles or lipidoid nanoparticles or the formulation of lipid nanoparticles or lipidoid nanoparticles according to the invention as described herein for use in the treatment or prevention of a disease. Likewise, the suspension of lipid nanoparticles or lipidoid nanoparticles or the formulation of lipid nanoparticles or lipidoid nanoparticles according to the invention as described therein can be used in a method for the treatment or prevention of a disease, which method includes administering the suspension or formulation to a subject in need thereof.

In a related aspect, the invention also provides the suspension of lipid nanoparticles or lipidoid nanoparticles or the formulation of lipid nanoparticles or lipidoid nanoparticles according to the invention as described herein for use as a medicament.

For example, the invention provides the suspension of lipid nanoparticles or lipidoid nanoparticles or the formulation of lipid nanoparticles or lipidoid nanoparticles according to the invention as described herein for use in vaccination or immunization. Likewise, the suspension of lipid nanoparticles or lipidoid nanoparticles or the formulation of lipid nanoparticles or lipidoid nanoparticles according to the invention as described therein can be used in a method for vaccination or immunization, which method includes administering the suspension or formulation to a subject in need thereof.

In line with a further aspect, the invention provides a method of inducing an immune response against a target pathogen in a subject in need thereof, the method comprising administering a suspension of lipid nanoparticles or lipidoid nanoparticles or the formulation of lipid nanoparticles or lipidoid nanoparticles according to the invention as described herein to the subject.

In another example, the invention provides the suspension of lipid nanoparticles or lipidoid nanoparticles or the formulation of lipid nanoparticles or lipidoid nanoparticles according to the invention as described therein for use in the treatment of cancer. Likewise, the suspension of lipid nanoparticles or lipidoid nanoparticles or the formulation of lipid nanoparticles or lipidoid nanoparticles according to the invention as described therein can be used in a method for the treatment of cancer, which method includes administering the suspension or formulation to a subject in need thereof.

In a further aspect, the invention provides a method of avoiding or for alleviating side effects in a therapy with lipid nanoparticles or lipidoid nanoparticles comprising at least one therapeutic agent as they are described herein, wherein the method comprises the steps:
i) determine whether lipid nanoparticles or lipidoid nanoparticles in a pharmaceutical composition comprising the lipid nanoparticles or lipidoid nanoparticles aggregate when subjected to a mechanical stress or a temperature stress, by determining their aggregation level before and after subjecting said pharmaceutical composition to said mechanical or temperature stress
ii) if the lipid nanoparticles or lipidoid nanoparticles show aggregation after the test of step (i), then add to the lipid nanoparticles or lipidoid nanoparticles formulation a surfactant as defined herein to obtain a LNP or LiNP suspension with a final surfactant concentration between 0.01% w/v and 10% w/v surfactant, preferably between 0.1% w/v and 10% surfactant, more preferably between 0.25% w/v surfactant and 5% w/v surfactant, still more preferably between 0.33% w/v surfactant and 2.5% w/v surfactant, even more preferably between 0.45% w/v surfactant and 1.5% w/v surfactant, most preferably between 0.5% w/v and 1.5% w/v surfactant,
iii) reconstitute with mixing to generate a stable suspension of lipid nanoparticles or lipidoid nanoparticles.

In a related aspect, the invention further provides a method of reducing one or more side effects associated with a vaccine formulation or an anticancer formulation comprising lipid nanoparticles or lipidoid nanoparticles carrying a nucleic acid as they are described herein, the method comprising modifying the vaccine formulation or an anticancer formulation by adding a surfactant as described herein to a vaccine formulation or anticancer formulation comprising a suspension of the lipid nanoparticles or lipidoid nanoparticles. Preferably, the surfactant represents between 0.01% w/v and 10% w/v surfactant, preferably between 0.1% w/v and 10% surfactant, more preferably between 0.25% w/v surfactant and 5% w/v surfactant, still more preferably between 0.33% w/v surfactant and 2.5% w/v surfactant, even more preferably between 0.45% w/v surfactant and 1.5% w/v surfactant, most preferably between 0.5% w/v and 1.5% w/v surfactant.

As described above, the surfactant is preferably at least one nonionic surfactant selected from the group consisting of fatty alcohol ethoxylates, fatty acid ethoxylates, block copolymers of ethylene oxide and propylene oxide, alkylphenol ethoxylates or oligomers of alkylphenol ethoxylates, fatty acid esters of sorbitol, ethoxylated fatty acid esters of sorbitol, fatty acid esters of glycerol, ethoxylated castor oil and ethoxylated vitamin E, more preferably at least one or still more preferably one selected from the group consisting of poloxamer 124, poloxamer 188, poloxamer 338, poloxamer 407, polysorbate 20, polysorbate 80, polyoxyethylenelaurylether, poyloxyethylene-35 castor oil, D-α-tocopherol polyethylene glycol 1000 succinate, and Tyloxapol. In line with the above, and with a preferred embodiment, the surfactant is the block copolymer of ethylene oxide and propylene oxide, preferably a poloxamer selected from the group of poloxamer 124, poloxamer 188, poloxamer 338, poloxamer 407. In line with an alternative embodiment, the poloxamer may be a poloxamer other than poloxamer 188 or poloxamer 407.

In a related aspect, the invention provides a method of reducing the occurrence or severity of one or more side effects associated with a a LNP/LiNP based vaccine in a subject, the method comprising administering a vaccine formulation or an anticancer formulation comprising the suspension of lipid nanoparticles or lipidoid nanoparticles as described herein to the subject.

The reduction of the occurrence or severity of one or more side effects may be caused by a reduction of LNP/LiNP aggregation, as it may be measured, e.g., by determining the hydrodynamic diameter of the nanoparticle, for example by via dynamic light scattering or photon correlation spectroscopy.

Generally, the therapeutic agent, preferably nucleic acid, is included in an effective amount in the nanoparticles. The term "effective amount" refers to an amount sufficient to induce a detectable therapeutic response or a preventive effect in the subject to which the pharmaceutical composition is to be administered. In accordance with the above, the content of the nucleic acid is not limited as far as it is useful for treatment or prevention as described above. As noted above, the composition wherein the particles comprising the nucleic acid are contained, preferably comprises the particles in an amount so as to provide the nucleic acid contained in the particles at a concentration of 0.01 to 10 mg/ml, more preferably 0.02 to 10 mg/ml, still more preferably 0.05 to 5 mg/ml and most preferably 0.05 to 2.5 mg/ml, based on the total volume of the composition. Likewise, it will be understood that, in cases where the suspension of lipid nanoparticles or lipidoid nanoparticles or the formulation of lipid nanoparticles or lipidoid nanoparticles according to the invention as described therein is administered to a subject, it will be administered in an effective amount.

Exemplary subjects include a mammal such as a dog, cat, pig, cow, sheep, horse, rodent, e.g., rat, mouse, and guinea pig, or a primate, e.g., gorilla, chimpanzee, and human. In a most preferable embodiment, the subject is a human.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

### Examples

### 1 Example 1 - Nanoparticle shaking resistance in presence of different excipients at varying excipient concentrations

### 1.1 Methods:

### 1.1.1 Nanoparticle preparation

Lipidoid nanoparticles were formulated from the ionizable lipidoid (dL_05(R), Scheme 1), the helper lipids DPPC (1,2-dipalmitoyl-sn-glycero-3-phosphocholine, Avanti Polar Lipids) and cholesterol (Avanti Polar Lipids) and the PEG lipid DMG-PEG2k (1,2-Dimyristoyl-sn-glycerolmethoxy(polyethylene glycol)-2000, Avanti Polar lipids) at the molar ratios of 8.00/5.29/4.41/0.88 respectively. Proper volumes of lipid stock solutions in HPLC grade ethanol of concentrations of 50, 20, 20 and 20 mg/mL, respectively, were combined. The formulation process was performed by a rapid solvent exchange. The lipid mixture in ethanol was combined with the mRNA in citrate buffer (10 mM citric acid, 150 mM NaCl, pH 4.5) at a volumetric ratio of 1:4 using a NanoAssemblr benchtop (Precision NanoSystems). The resulting formulation had an mRNA concentration of 0.2 mg/mL with an N/P ratio of 8. After 30 minutes of incubation at room temperature, the formulation was purified via dialysis against water using a Slide-A-Lyzer MINI dialysis device (20k, 2mL, Thermo scientific). If concentration of the suspension was required a speed vac (concentrator plus, Eppendorf) was used at 45°C in V-AQ mode.

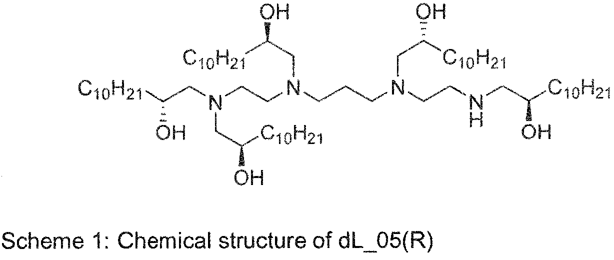

### 1. 1.2 Mixing of nanoparticle with excipients

Excipients used in this experiment are listed in Table 5. Dilutions of excipients were prepared at the stock concentrations indicated in Table 5 according to their solubility in water. LNPs were mixed with the excipients to result in at excipient concentration of 0%(w/v), 0.01%(w/v), 0.1%(w/v), 1%(w/v) and 10%(w/v) (where possible, otherwise indicated in graph) and an LNP concentration of 0.2mg mRNA/mL.

**Table 5: Excipients used within this experiment**

| **Material** | **Supplier** | **Stock concentration [%w/v]** |
|---|---|---|
| Kolliphor P188 | Sigma | 20 |
| Kolliphor P338 Geismar | BASF | 20 |
| Kolliphor P407 Geismar | BASF | 2 |
| Tween-20 | Sigma | 20 |
| Tween-80 | Roth | 20 |
| BRIJ35 | Merck | 10 |
| Tyloxapol | Sigma | 2 |
| VitE-PEG1000 | Sigma | 20 |
| Kolliphor EL | Sigma | 20 |

### 1.1.3 Shaking of nanoparticle suspension

For stress testing 100µL of the LNP/excipient mixes was shaken at maximum speed on a vortex (Vortex Genie 2, Scientific Industries) for 1min.

### 1.1.4 Measurement of complex size and Pdl

Hydrodynamic diameter (Z.average, size) and polydisperity index (Pdl) of nanoparticles was measured by dynamic light scattering (DLS) using a Zetasizer Nano-ZS (Malvern Instruments) with an automatic attenuator and reported as intensity particle size distribution. Samples were measured undiluted at 25 °C.

### 1.2 Results

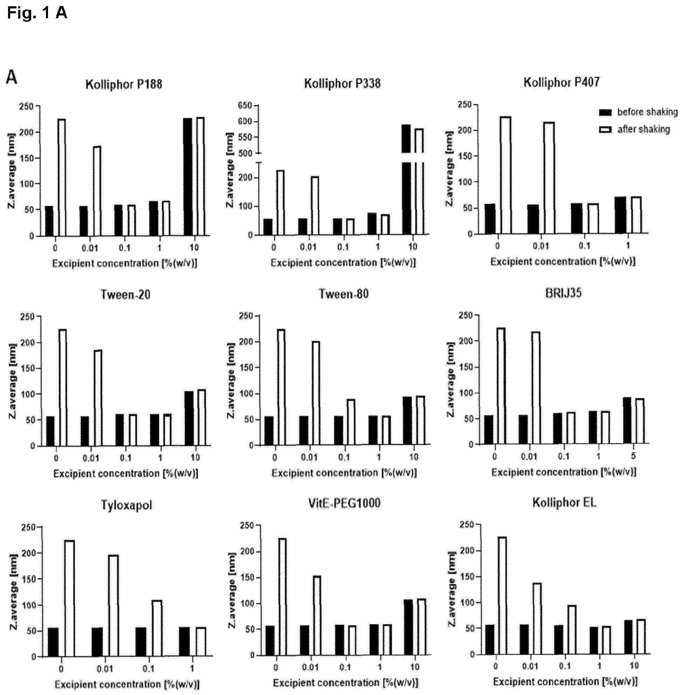

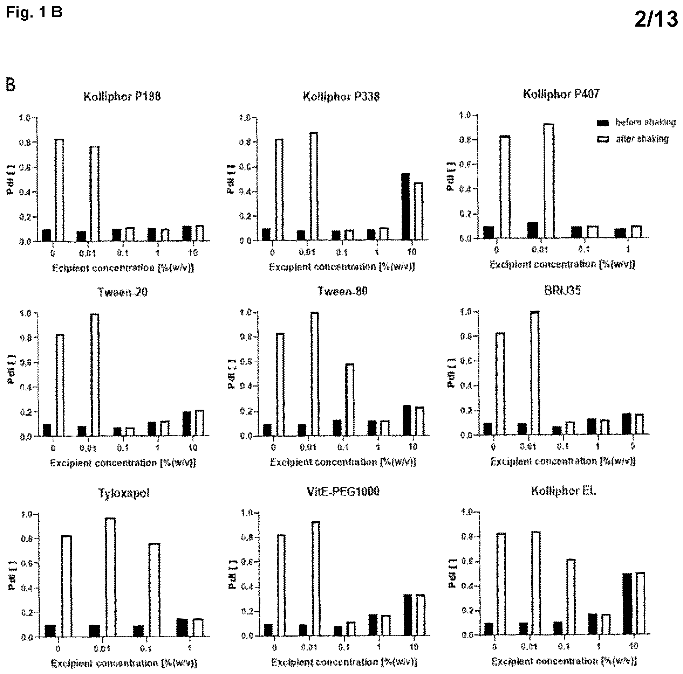

This experimental set aimed at testing the particle stability of the LNP suspension under physical stress conditions. Results are shown in Figure 1. In absence of excipients (0%(w/v)) the LNP aggregates (size and Pdl increase before vs after shaking) after shaking indicating loss of suspension integrity. In presence of the used excipients the integrity of the nanoparticles remains stable at an excipient concentration >0.01%(w/v). The general size increase at 10%(w/v) can in generally be explained via the viscosity increase of the suspension triggered via the high excipient concentration. This leads to a reduced Brownian motion of the nanoparticle which is interpreted by the DLS software as larger particle. As there is no difference in size between before and after shaking the nanoparticle stability can still be demonstrated.

### 2 Example 2 - Nanoparticle shaking resistance at different nanoparticle concentrations

### 2.1 Methods:

### 2.1.1 Nanoparticle preparation

See section 1.1.1

### 2.1.2 Mixing of nanoparticle with excipients

*See section 1.1.2.* For this set of experiments solely Poloxamer 188 (Kolliphor P188) was used at an excipient concentration of 1%(w/v). As a control one sample without excipient was used. LNPs were used at mRNA concentrations of 0.01mg/mL, 0.1mg/mL, 1mg/mL and 2.5mg/mL.

### 2.1.3 Shaking of nanoparticle suspension

See section 1.1.3

### 2.1.4 Measurement of complex size and Pdl

See section 1.1.4

### 2.2 Results

This experiment aimed at the identification of concentration limits of LNPs present in the suspension. Results are displayed in Figure 2. Results indicate that shaking results in a reduced nanoparticle quality at all tested concentrations. Aggregate formation (increased size and/or Pdl) was detected for all LNP concentrations in absence of excipient. In contrast to that the presence of excipient stabilizes the nanoparticle suspension at all tested LNP concentrations demonstrating that the advantageous effect applies for a broad LNP concentration range.

### 3 Example 3 - Shaking resistance of MC3-LNPs in presence of different excipients at varying excipient concentrations

### 3.1 Methods:

### 3.1.1 Nanoparticle preparation

For formulation of MC3 LNPs lipid-stocks of DLin-MC3-DMA ((6Z,9Z,28Z,31Z)-heptatriaconta-6,9,28,31-tetraene-19-yl 4-(dimethylamino)butanoate), DSPC, Cholesterol and DMPE-PEG2k were prepared at 10mg/mL, 20mg/mL, 20mg/mL and 20mg/mL, respectively, in ethanol. The ethanol stocks were mixed (557µL DLin-MC3-DMA, 69µL DSPC, 129µL Cholesterol, 35µL DMPE-PEG2k, 461µL ethanol) and fused with the aqueous mRNA-solution (0.2667mg/mL in citrate buffer) using a NanoAssemblr device at volumetric ratio of 3:1 (mRNA:lipids) and a total flow of 12mL/min. After 30 min incubation at RT, the formulation was purified via dialysis against water using a Slide-A-Lyzer MINI dialysis device (20k, 2mL, Thermo scientific). If concentration of the suspension was required a speed vac (concentrator plus, Eppendorf) was used at 45°C in V-AQ mode.

### 3.1.2 Mixing of nanoparticle with excipients

*See section 1.1.2.* In addition to the excipients already listed in Table 5 Kolliphor P124, Geismar (BASF) was also tested as excipient. The substance stock solution was prepared at 20%(w/v) and was handled identical to the other excipients.

### 3.1.3 Shaking of nanoparticle suspension

See section 1.1.3

### 3.1.4 Measurement of complex size and Pdl

See section 1.1.4

### 3.2 Results

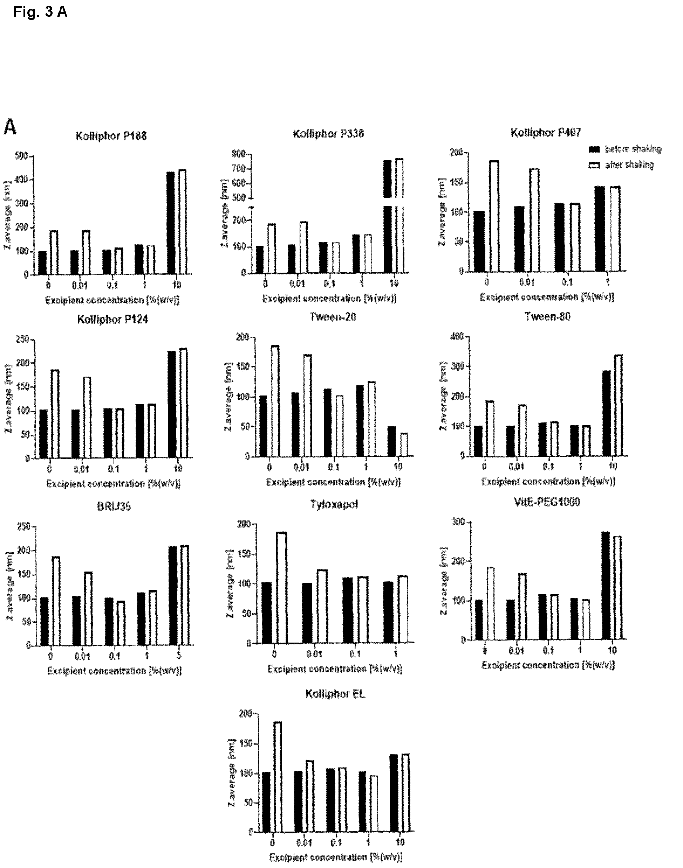

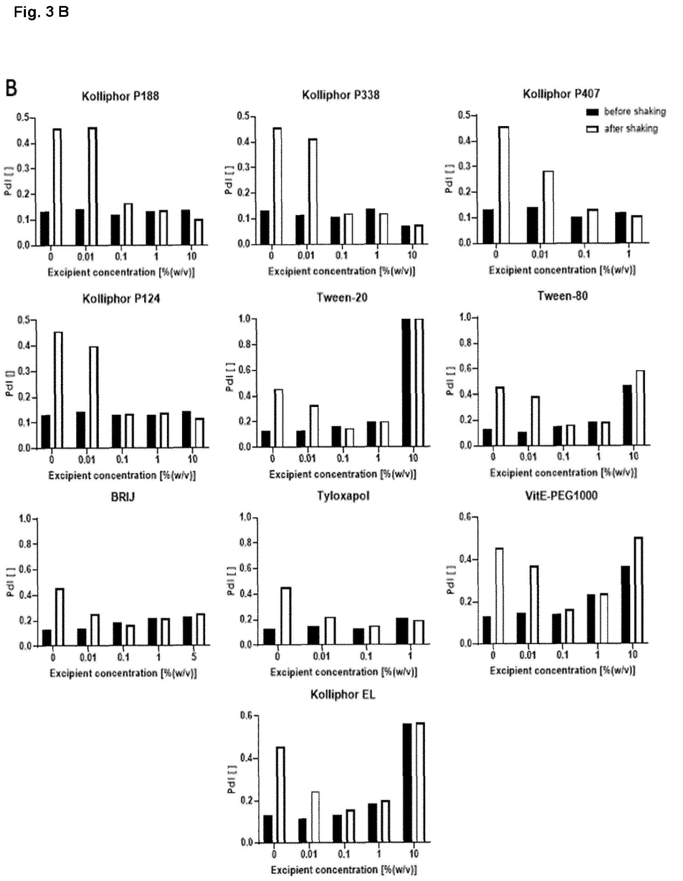

This experiment aimed at determination if the observed protective effect of the excipients is a unique property of the tested LNP (containing dL_05(R)) or can be regarded as general property. For that purpose, the LNP used in Example 1 was replaced by MC3-LNP, a known LNP formulation used for delivery of various nucleic acids. Data is summarized in Figure 3. Results show high similarity with the data generated in Example 1. Shaking in absence of excipients results in reduction of particle quality (increased size and Pdl). The presence of the different excipients at a concentration of more than 0.01%(w/v) results in stabilization of the suspension and prevents aggregation during physical stress. These data demonstrate that the protective effect is independent of the used LNP composition.

### 4 Example 4 - Shaking resistance of ALC-0315 LNPs in presence of different excipients at varying excipient concentrations

### 4.1 Methods:

### 4.1.1 Nanoparticle preparation

For formulation of ALC-0315 LNPs lipid-stocks of ALC-0315 (((4-hydroxybutyl)azandiyl)bis(hexan-6,1-diyl)bis(2-hexyldecanoat)), DSPC, Cholesterol and ALC-0159 (2-[(Polyethylenglykol)-2000]-N,N-ditetradecylacetamid) were prepared at 25mg/mL, 20mg/mL, 20mg/mL and 25mg/mL, respectively, in ethanol. The ethanol stocks were heated to 50°C for 30min and mixed. For an exemplary amount of 1mg mRNA the following volumes were combined: 574µL ALC-0315, 156µL DSPC, 311µL Cholesterol, 71µL ALC-0519, 138µL ethanol. The mix was fused with the aqueous mRNA-solution (3750µL, 0.2667mg/mL in citrate buffer) using a NanoAssemblr device at volumetric ratio of 3:1 (mRNA:lipids) and a total flow of 12mL/min. After a 30 minute incubation at room temperature, the formulation was purified via dialysis against water using a Slide-A-Lyzer MINI dialysis device (20k, 2mL, Thermo scientific). If concentration of the suspension was required, a speed vac (concentrator plus, Eppendorf) was used at 45°C in V-AQ mode.

### 4.1.2 Mixing of nanoparticle with excipients

*See section 1.1.2.* In addition to the excipients already listed in Table 5, Kolliphor P124, Geismar (BASF) was also tested as excipient. The substance stock solution was prepared at 20%(w/v) and was handled identical to the other excipients. An excipient concentration range of 0.1%(w/v) to 10%(w/v) was tested.

*4.1.3 Shaking of nanoparticle suspension*

See section 1.1.3

### 4.1.4 Measurement of complex size and Pdl

See section 1.1.4

### 4.2 Results

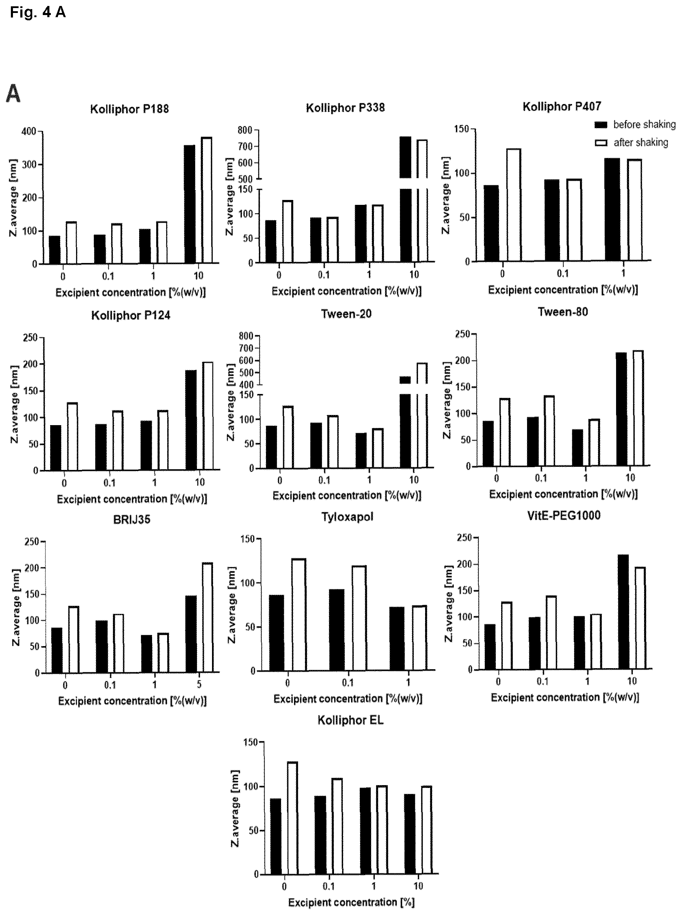

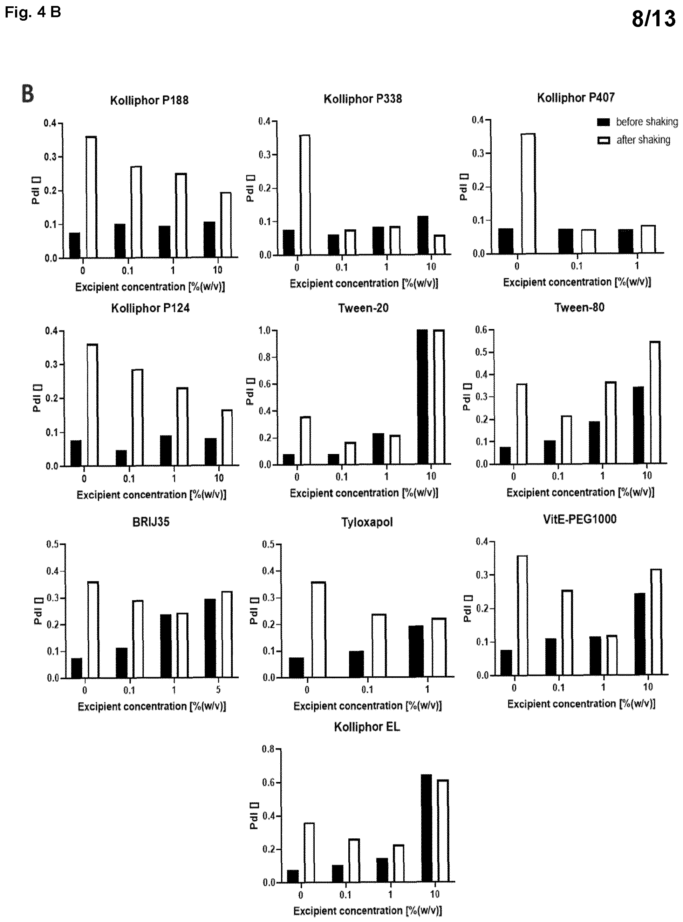

This experiment was aimed at determining if the observed protective effect of the excipients is a unique property of the tested LNP (containing dL_05(R)) or can be regarded as general property. For that purpose, the LNP used in Example 1 was replaced by ALC-0315 containing LNP with the lipid composition of the Covid vaccine Comirnaty. The results are summarized in Figure 4 and show a high similarity with the data generated in Example 1 and 3. Shaking in absence of excipients results in reduction of particle quality (increased size and Pdl). The presence of the different excipients at a concentration of more than 0.01%(w/v) results in stabilization of the suspension and prevents aggregation during physical stress. These data further demonstrate that the protective effect is independent of the used LNP composition.

### 5 Example 5 - Shaking resistance of Comirnaty^{®} in the presence of different excipients at varying excipient concentrations

### 5.1 Methods:

### 5.1.1 Mixing of nanoparticle with excipients

Comirnaty^{®} was sourced commercially. Excipients used in this Example are listed in Table . Dilutions of excipients were prepared at the stock concentrations indicated in Table according to their solubility in water. LNPs were mixed with the excipients to result in an excipient concentration of 0%(w/v), 0.01%(w/v), 0.1%(w/v), 0.5%(w/v), 1%(w/v), 2.5%(w/v) and 5%(w/v) (where possible) and an LNP concentration of 0.075mg mRNA/mL.

**Table 6: Excipients used in Example 5**

| **Material** | **Supplier** | **Stock concentration [%w/v]** |
|---|---|---|
| Kolliphor P188 | Sigma | 20 |
| Kolliphor P338 Geismar | BASF | 20 |
| Kolliphor P407 Geismar | BASF | 2 |
| Tween-20 | Sigma | 50 |
| Tween-80 | Roth | 20 |
| BRIJ35 | Merck | 10 |
| Tyloxapol | Sigma | 2 |
| VitE-PEG1000 | Sigma | 20 |
| Kolliphor EL | Sigma | 20 |
| Kollisolv P124 Geismar | BASF | 50 |

### 5.1.2 Shaking of nanoparticle suspension

See section 1.1.3

### 5.1.3 Measurement of complex size and Pdl

See section 1.1.4

### 5.2 Results

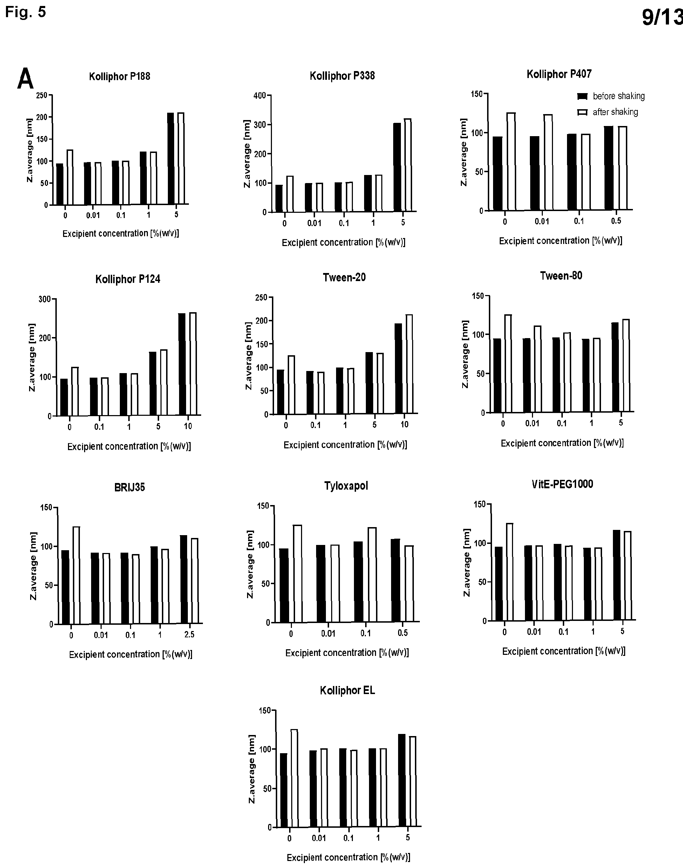

This experiment aimed at determining if the observed protective effect of the present excipients is a unique property of the tested LNP (containing dL_05) or can be regarded as a general property. For that purpose the LNP used in Example 1 was replaced by a commercially available mRNA/LNP formulation used for vaccination: Comirnaty^{®} (BioNTech/Pfizer). Data is summarized in Figure 5. Results show high similarity with the data generated in Example 1. Shaking in the absence of excipients results in a reduction of particle quality (increased hydrodynamic diameter by >20%). The presence of the different excipients at a concentration of more than 0.01%(w/v) results in a stabilization of the suspension and prevents aggregation during physical stress.

### 6 Example 6 - Shaking resistance of SpikeVax^{®} in presence of representative excipient

### 6.1 Methods:

### 6.1.1 Mixing of nanoparticle with excipient

SpikeVax^{®} (Moderna) was sourced commercially. LNPs were mixed with Kolliphor P188 (Poloxamer 188) to result in an excipient concentration of 1%(w/v) and an LNP concentration of 0.15mg mRNA/mL according to Table .

**Table 7: Sample preparation for Example 6**

| **Final sample** | **V (Spikevax^{®}) [µL]** | **V (H₂O) [µL]** | **V (20% P188 stock) [µL]** |
|---|---|---|---|
| Spikevax^{®} containing 1% (w/v) P188 | 112.5 | 30 | 7.5 |

### 6.1.2 Shaking of nanoparticle suspension

For stress testing, 100µL of the LNP/excipient mix was shaken at maximum speed on a vortex (Vortex Genie 2, Scientific Industries) for 1, 5, 30, 60 and 90 minutes.

### 6.1.3 Measurement of complex size and Pdl

See section 1.1.4

### 6.2 Results

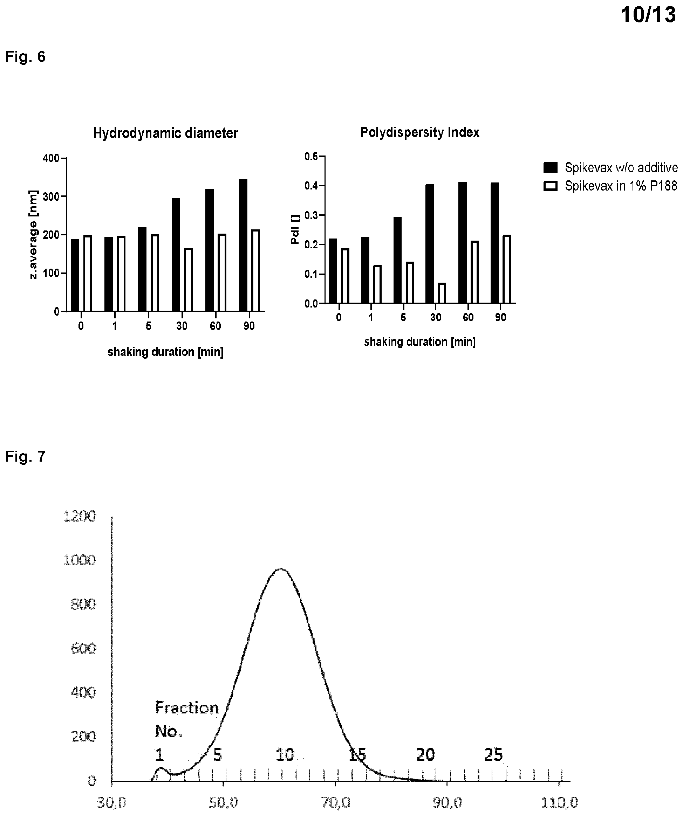

This experiment was aimed at determining if the observed protective effect of the present excipients is a unique property of the tested LNP (containing dL_05) or can be regarded as general property. For that purpose the LNP used in Example 1 was replaced by a commercially available mRNA/LNP formulation used for vaccination: Spikevax^{®} (Moderna). Data is summarized in Figure 6. The results show a high similarity with the data generated in Example 1. Shaking in absence of excipient results in reduction of particle quality (increased size and Pdl). The presence of Kolliphor P188 at a concentration 1%(w/v) results in stabilization of the suspension and prevents aggregation during physical stress.

### 7 Example 7 - Analysis of attachment of poloxamer to LiNP using preparative size exclusion chromatography

This experiment aimed at determining if a poloxamer such as p188, added to a LNP after nanoprecipitation is attached (in particular adhered to or integrated) to LNP or LiNP, or if it remains in solution.

A formulation containing LiNPs and P188 was separated on a size exclusion chromatography (SEC) column to determine whether the P188 is attached/associated to the particle under these conditions.

### Material and Methods

### 7.1.1 Materials

**Table 8 - Materials**

| **Material** | **Supplier** | **Lot no./ Buffer no.** | **Cat no.** |
|---|---|---|---|
| LiNP / P188 Formulation | selfmade | Test mRNA, conc. 1.3 mg/mL | na |
| HiPrep 16/60 Sephacryl S-500 HR (cv 120 mL) | Cytiva | | 28935606 |
| Running buffer | selfmade | 25 mM MOPS, 150 mM NaCl, pH 6.8 | na |
| Acetonitril, LiChrosolv^{®}, hypergrade, Supelco^{®}, 2.5 L | VWR | | 1.00029.2500 |
| DNAPac^{™} RP-Säule (4µm, 3x100 mm) | Thermo Fisher | | 88919 |
| Wasser für die HPLC super gradient grade 2,5 L | VWR | | 83650320 |

| **Device** | **Supplier** | **Cat no. or type** |
|---|---|---|
| FPLC | GE Healthcare | Äkta Purifier |
| 1260 Bio Quat Pump | Agilent | G5611A |
| 1260 HiP Bio Sampler | Agilent | G5667A |
| 1290 Thermostat | Agilent | G1330B |
| 1290 TCC | Agilent | G1316C |
| 1260 DAD | Agilent | G4212B |
| 1290 ELSD | Agilent | G7102A |

### 7.1.2 Methods

A LiNP formulation that contains the LiNP and P188 was separated via size exclusion chromatography. The single fractions were analyzed via HPLC fpr poloxamer contend, size distribution by DLS and integrity by Ribogreeen assay.

### 7.1.2.1 PrepSEC

The preparative size exclusion chromatography was performed on an Äkta Purifier System using a HiPrep 16/60 Sephacryl S-500 HR (cv 120 mL) column. Running buffer was 25 mM MOPS, 150 mM NaCl, pH 6.8. Flow rate for sample application and elution was set to 1 mL/min. For preparative runs fractions of 2.5 mL were collected during elution. UV signal at 260 nm as well as conductivity was monitored during the run.

### 7.1.2.2 HPLC analysis

The parameter set of the HPLC device is summarized in Table 1. The solvent gradient profile can be found in Table 9.

**Table 9: Device parameters for the HPLC method.**

| **Device** | **Parameter** | **Value and unit** |
|---|---|---|
| Pump | Flow | 0.4 ml/min |
| | Solvent A | Water |
| | Solvent B | Acetonitrile (MeCN) |
| | Pressure Limit | 250 bar |
| Column compartment | Column temperature | 65 °C |
| HiP Sampler | Injection volume | 20 µl |
| | Draw speed | 120 µl/min |
| | Eject speed | 120 µl/min |
| | Draw position | 2.5 mm |
| | Equilibration time | 5.0 s |
| | Sample flush out factor | 5 x injection volume |
| ELSD | Evaporator temperature | 70 °C |
| | Nebulizer temperature | 70 °C |
| | Gas flow rate | 0.90 SLM |
| | Signal Smoothing | 10 (1.0 s) |
| Valve | Initial valve position: | ELSD |
| | Change position: waste | 0.05 min |
| | Change position: ELSD | 6.00 min |

**Table 10: Gradient profile of the HPLC method for analysis of LNP and vehicle samples.**

| **Time (min)** | **A%** | **B%** |
|---|---|---|
| 0.00 | 100 | 0 |
| 7.75 | 100 | 0 |
| 8.00 | 42 | 58 |
| 16.00 | 36 | 64 |
| 17.25 | 10 | 90 |
| 17.70 | 0 | 100 |
| 23.00 | 0 | 100 |
| 23.25 | 100 | 0 |
| 28.00 | 100 | 0 |
| Posttime: 5 min | | |

### Results

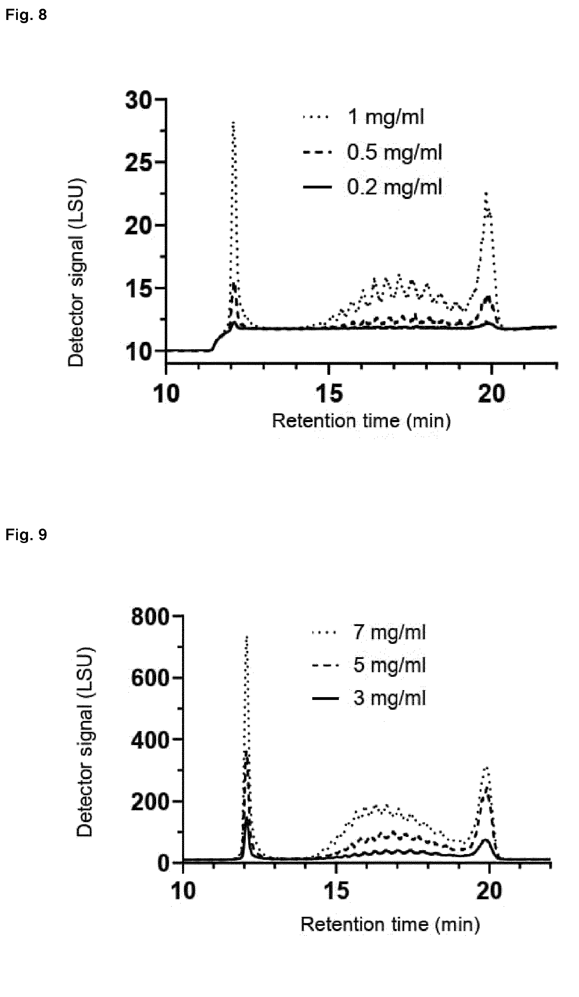

A HPLC calibration curve was prepared using P188 reference solutions at 0.2 to 7 mg/mL (Figure 8, 9 and 10).

Selected fractions after size exclusion chromatography were analyzed for presence of poloxamer with HPLC. Absorption at 260 nm in the elution profile shows, that the LiNP elutes within an elution volume of 50 -70 mL (Fraction 3-13, figure 7). Integrity of the LiNP was confirmed by DLS and ribogreen assay

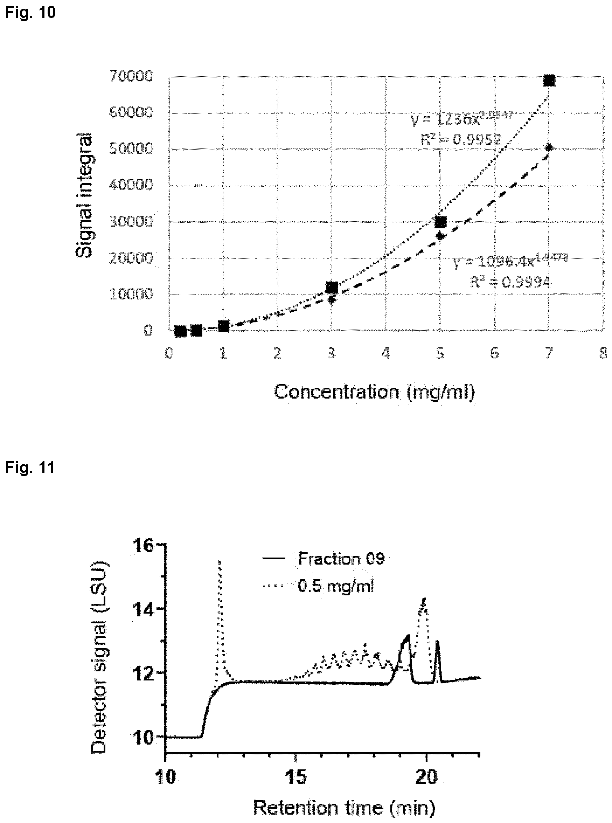

Within the fraction of the LiNP main peak we could not detect any P188 by HPLC (see figure 11, exemplary for fraction 9).

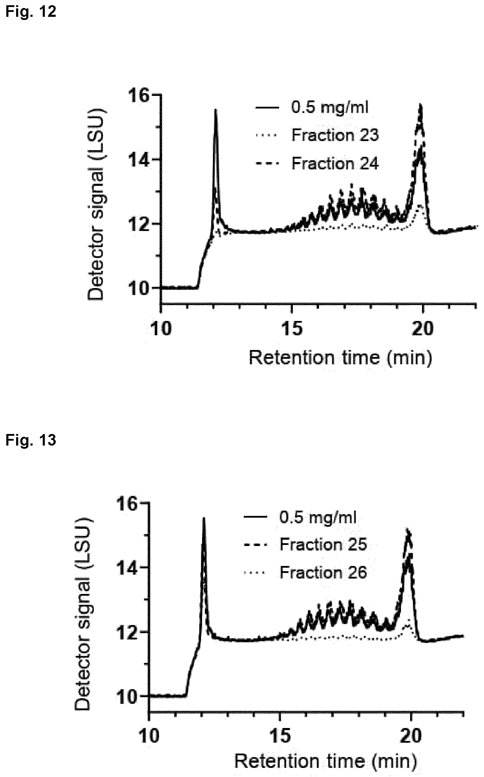

P188 in a concentration of 0.2 to 0.5 mg/mL was only detected in later eluting fractions 23, 24, 25 and 26 (figure 12 and 13).

### Summary

It was possible to separately detect poloxamer from intact LNPs according to their differences in molecular weight using a preparative size exclusion chromatography method. Intact LiNPs were detected in accordance with the elution profile measured at 260 nm wavelength for fractions 3-13. We could verify the integrity of the particles by DLS measurement as well as ribogreen assay. Poloxamer was detected in later eluting fractions 23-26 by HPLC. Based on this data, we conclude that poloxamer added to LNP is not attached to the particles.

**Table 11: Summary of analytical results of fraction from preparative size exclusion chromatography.**

| **Fraction No.** | **LiNP detection (UV260 nm, Ribogreen, DLS)** | **P188 detected by HPLC [mg/mL]** |
|---|---|---|
| 3-13 | Yes | not detected |
| 23-26 | Not detected | 0.2 - 0.5 |

### Brief description of the drawings:

Figure 1 shows the size distribution (A) and polydispersity index (B) of LNPs before (black bars) and after (white bars) shaking in presence of varying concentrations of different excipients.
Figure 2 shows the size distribution (A) and polydispersity index (B) of different concentrations of LNPs before (black bars) and after (white bars) shaking in presence of 1%(w/v) Poloxamer 188.
Figure 3 shows the size distribution (A) and polydispersity index (B) of MC3-LNPs before (black bars) and after (white bars) shaking in presence of varying concentrations of different excipients.
Figure 4 shows the size distribution (A) and polydispersity index (B) of ALC-0315 LNPs before (black bars) and after (white bars) shaking in the presence of varying concentrations of different excipients.
Figure 5 shows the size distribution of Comirnaty^{®} LNPs before (black bars) and after (white bars) shaking in the presence of varying concentrations of different excipients.
Figure 6 shows the size distribution and polydispersity index of Spikevax^{®} LNPs before (black bars) and after (white bars) shaking in presence of 1% (w/v) Kolliphor P188.
Figure 7 shows a size exclusion chromatogram of LNP (based on lipidoid of Scheme 1) formulated with poloxamer P188. The X axis corresponds to elution volume in mL and the Y-axis represent signal UV 260 nm.
Figure 8 shows a HPLC calibration curve for poloxamer P188 for 0.2, 0.5 and 1 mg/mL.
Figure 9 shows a HPLC calibration curve for poloxamer P188 for 3, 5 and 7 mg/mL.
Figure 10 shows a HPLC calibration curve plot for poloxamer P188.
Figure 11 shows a signal comparison between a reference sample containing 0.5 mg/mL poloxamer and fraction 9, containing LiNP.
Figure 12 shows a HPLC chromatogram showing fraction 23 and fraction 24 compared to a 0.5 mg/mL poloxamer P188 reference sample.
Figure 13 shows a HPLC chromatogram showing fraction 25 and fraction 26 compared to a 0.5 mg/mL poloxamer P188 reference sample.

## Claims

1. Use of a surfactant for stabilizing a suspension of lipid nanoparticles or of lipidoid nanoparticles in an aqueous vehicle solution against particle aggregation under a physical stress condition selected from shaking, stirring, vibrating, mixing, inverting, tapping, or dropping of the suspension, or a combination thereof, or wherein the physical stress condition is caused by pumping the suspension, by its withdrawal into a syringe, or by its transport, wherein the lipid nanoparticles or lipidoid nanoparticles comprise the following components (a) and (b):
(a) a nucleic acid and
(b) at least one selected from a permanently cationic lipid, an ionizable lipid and an ionizable lipidoid,
and wherein the surfactant is incorporated as an excipient into the aqueous vehicle solution.

2. The use of the surfactant in accordance with claim 1, wherein the physical stress condition is selected from inverting, tapping, or dropping of the suspension, or wherein the physical stress condition is caused by pumping the suspension or by its withdrawal into a syringe.

3. The use of the surfactant in accordance with claim 1 or 2, wherein the surfactant is essentially not attached with the nanoparticles.

4. The use of the surfactant in accordance with any one of claims 1 to 3, wherein the surfactant is not present in the vehicle solution during a lyophilization process.

5. The use of the surfactant in accordance with any of claims 1 to 4, wherein the surfactant is a nonionic surfactant.

6. The use of the surfactant in accordance with claim 5, wherein the surfactant is at least one nonionic surfactant selected from the group consisting of fatty alcohol ethoxylates, fatty acid ethoxylates, block copolymers of ethylene oxide and propylene oxide, alkylphenol ethoxylates or oligomers of alkylphenol ethoxylates, fatty acid esters of sorbitol, ethoxylated fatty acid esters of sorbitol, fatty acid esters of glycerol, ethoxylated castor oil and ethoxylated vitamin E.

7. The use of the surfactant in accordance with claim 6, wherein the surfactant is a block copolymer of ethylene oxide and propylene oxide, preferably a poloxamer selected from poloxamer 124, poloxamer 188, poloxamer 338 and poloxamer 407.

8. The use of the surfactant in accordance with claim 6, wherein the surfactant is at least one nonionic surfactant selected from the group consisting of oligomers of alkylphenol ethoxylates, fatty acid esters of sorbitol, fatty acid esters of glycerol, and ethoxylated vitamin E.

9. The use of the surfactant in accordance with any of claims 1 to 8, wherein the suspension of lipid nanoparticles or lipidoid nanoparticles in an aqueous vehicle solution comprises the surfactant at a concentration of 0.01 to 10 % (w/v).

10. The use of the surfactant in accordance with any of claims 1 to 8, wherein the nucleic acid is mRNA.

11. The use of the surfactant in accordance with any of claims 1 to 10 wherein the nanoparticles do not comprise Poloxamer 188 and/or do not comprise Poloxamer 407.

12. The use of the surfactant in accordance with any of claims 1 to 11, wherein the nanoparticles comprise an ionizable lipidoid (b) of the following formula (b-1), 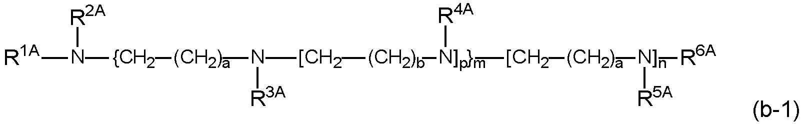 wherein:
a is 1 and b is an integer of 2 to 4; or a is an integer of 2 to 4 and b is 1,
p is 1 or 2,
m is 1 or 2; n is 0 or 1 and m+n is ≥ 2; and
R^{1A} to R^{6A} are independently of each other selected from: hydrogen; -CH₂-CH(OH)-R^{7A}, -CH(R^{7A})-CH₂-OH, -CH₂-CH₂-(C=O)-O-R^{7A,}
-CH₂-CH₂-(C=O)-NH-R^{7A}; -CH₂-R^{7A}; -C(NH)-NH₂; a poly(ethylene glycol) chain; and a receptor ligand; wherein R^{7A} is selected from C3-C18 alkyl and C3-C18 alkenyl having one C-C double bond;
provided that at least two residues among R^{1A} to R^{6A} are selected from -CH₂-CH(OH)-R^{7A}, -CH(R^{7A})-CH₂-OH, -CH₂-CH₂-(C=O)-O-R^{7A},
-CH₂-CH₂-(C=O)-NH-R^{7A} and -CH₂-R^{7A}, wherein R^{7A} is selected from C3-C18 alkyl or C3-C18 alkenyl having one C-C double bond;
or a protonated form thereof, wherein one or more of the nitrogen atoms contained in the compound of formula (b-1) are protonated to provide a compound carrying a positive charge.

13. Use of the surfactant in accordance with any of claims 1 to 12, wherein the nanoparticles comprise, as an ionizable lipid (b):
(6Z,9Z,28Z,31Z)-heptatriaconta-6,9,28,31-tetraene-19-yl 4-(dimethylamino)butanoate or a protonated form thereof wherein the nitrogen atom of the compound is protonated,
((4-hydroxybutyl)azanediyl)bis(hexan-6,1-diyl)bis(2-hexyldecanoate) or a protonated form thereof wherein the nitrogen atom of the compound is protonated, and/or
(heptadecan-9-yl 8-((2-hydroxyethyl)(6-oxo-6-(undecyloxy)hexyl)amino)octanoate( or a protonated form thereof wherein the nitrogen atom of the compound is protonated.

14. The use of the surfactant in accordance with any of claims 1 to 13, wherein the Z-average particle size of the suspended particles, as determined by dynamic light scattering at 25 °C, increases by less than 50% under the physical stress condition, preferably by less than 20%, still more preferably by less than 10%, and most preferably an increase is absent, and/or the polydispersity index of the suspended nanoparticles, as determined by dynamic light scattering at 25 °C, is maintained after exposure of the suspension to the physical stress condition relative to the polydispersity index of the suspension before exposure to the physical stress condition.

15. A suspension of lipid nanoparticles or of lipidoid nanoparticles in an aqueous vehicle solution, wherein the aqueous vehicle solution comprises a surfactant, and wherein the lipid nanoparticles or lipidoid nanoparticles comprise the following components (a) and (b):
(a) a nucleic acid, and
(b) at least one selected from a permanently cationic lipid, an ionizable lipid and an ionizable lipidoid,
and wherein the surfactant is essentially not attached to the nanoparticles.

16. A method for stabilizing a suspension of lipid nanoparticles or of lipidoid nanoparticles in an aqueous vehicle solution against particle aggregation under a physical stress condition selected from shaking, stirring, vibrating, mixing, inverting, tapping, or dropping of the suspension, or a combination thereof, or wherein the physical stress condition is caused by pumping the suspension, by its withdrawal into a syringe, or by its transport, wherein the lipid nanoparticles (LNP) or lipidoid nanoparticles (LiNP) comprise the following components (a) and (b):
(a) a nucleic acid and
(b) at least one selected from a permanently cationic lipid, an ionizable lipid and an ionizable lipidoid,
and wherein the method comprises incorporating a surfactant into the aqueous vehicle solution of the suspension of lipid nanoparticles or of lipidoid nanoparticles.

17. The method of claim 16, wherein the method comprises the following steps:
i) generating a preparation of lipid nanoparticles (LNP) or lipidoid nanoparticles (LiNP) by mixing of at least one selected from a permanently cationic lipid, an ionizable lipid and an ionizable lipidoid dissolved in an organic phase with a nucleic acid dissolved in an aqueous solution,
ii) diluting the preparation of lipid nanoparticles or lipidoid nanoparticles by dilution with a first solution,
iii) concentrating the diluted preparation of lipid nanoparticles or lipidoid nanoparticles by buffer exchange by ultra/diafiltration by TFF wherein a second solution is used for the ultra/diafiltration,
iv) obtaining a suspension of lipid nanoparticles or lipidoid nanoparticles;
wherein the first solution comprises between 0.01% w/v and 10% surfactant, preferably between 0.25% w/v surfactant and 5% w/v surfactant, more preferably between 0.33% w/v surfactant and 2.5% w/v surfactant, more preferably between 0.45% w/v and 1.5% w/v surfactant, most preferably between 0.5% w/v and 1.5% w/v surfactant, and/or wherein the second solution comprises between 0.01% w/v and 10% surfactant, preferably between 0.25% w/v surfactant and 5% w/v surfactant, more preferably between 0.33% w/v surfactant and 2.5% w/v surfactant, more preferably between 0.45% w/v and 1.5% w/v surfactant, most preferably between 0.5% w/v and 1.5% w/v surfactant,
and wherein the final concentration of surfactant from combined first and second solution is between 0.01% and 10% surfactant, preferably between 0.25% w/v surfactant and 5% w/v surfactant, more preferably between 0.33% w/v surfactant and 2.5% w/v surfactant, more preferably between 0.45% w/v and 1.5% w/v surfactant, most preferably between 0.5% w/v and 1.5% w/v surfactant with regard to the total volume of the suspension of the nanoparticles in the aqueous vehicle solution.

18. A method of reducing one or more side effects associated with a vaccine formulation or an anticancer formulation comprising LNPs or LiNPs carrying a nucleic acid, the method comprising modifying the vaccine formulation or anticancer formulation by adding a surfactant to a vaccine formulation comprising a LNP or LiNP suspension.

## Patentansprüche

1. Verwendung eines oberflächenaktiven Mittels zur Stabilisierung einer Suspension von Lipidnanopartikeln oder von Lipidoidnanopartikeln in einer wässrigen Trägerlösung gegen Partikelaggregation unter einer physikalischen Belastungsbedingung, ausgewählt aus Schütteln, Rühren, Vibrieren, Mischen, Invertieren, Klopfen oder Fallenlassen der Suspension oder einer Kombination davon, oder wobei die physikalische Belastungsbedingung durch das Pumpen der Suspension, durch ihr Aufziehen in eine Spritze oder durch ihren Transport verursacht wird, wobei die Lipidnanopartikel oder Lipidoidnanopartikel die folgenden Komponenten (a) und (b) umfassen :
(a) eine Nukleinsäure und
(b) mindestens eines, ausgewählt aus einem permanent kationischen Lipid, einem ionisierbaren Lipid und einem ionisierbaren Lipidoid,
und wobei das oberflächenaktive Mittel als ein Exzipient in die wässrige Trägerlösung eingebracht ist.

2. Die Verwendung des oberflächenaktiven Mittels gemäß Anspruch 1, wobei die physikalische Belastungsbedingung ausgewählt ist aus Invertieren, Klopfen oder Fallenlassen der Suspension, oder wobei die physikalische Belastungsbedingung durch das Pumpen der Suspension oder durch ihr Aufziehen in eine Spritze hervorgerufen wird.

3. Die Verwendung des oberflächenaktiven Mittels gemäß Anspruch 1 oder 2, wobei das oberflächenaktive Mittel im Wesentlichen nicht an die Nanopartikel gebunden ist.

4. Die Verwendung des oberflächenaktiven Mittels gemäß einem der Ansprüche 1 bis 3, wobei das oberflächenaktive Mittel in der Trägerlösung nicht während eines Lyophilisierungsverfahrens vorliegt.

5. Die Verwendung des oberflächenaktiven Mittels gemäß einem der Ansprüche 1 bis 4, wobei das oberflächenaktive Mittel ein nichtionisches oberflächenaktives Mittel ist.

6. Die Verwendung des oberflächenaktiven Mittels gemäß Anspruch 5, wobei das oberflächenaktive Mittel mindestens ein nichtionisches oberflächenaktives Mittel ausgewählt aus der Gruppe bestehend aus Fettalkoholethoxylaten, Fettsäureethoxylaten, Blockcopolymeren aus Ethylenoxid und Propylenoxid, Alkylphenolethoxylaten oder Oligomeren von Alkylphenolethoxylaten, Fettsäureestern von Sorbitol, ethoxylierten Fettsäureestern von Sorbitol, Fettsäureestern von Glycerol, ethoxyliertem Rizinusöl und ethoxyliertem Vitamin E, ist.

7. Die Verwendung des oberflächenaktiven Mittels gemäß Anspruch 6, wobei das oberflächenaktive Mittel ein Blockcopolymer aus Ethylenoxid und Propylenoxid ist, vorzugsweise ein Poloxamer ausgewählt aus Poloxamer 124, Poloxamer 188, Poloxamer 338 und Poloxamer 407.

8. Die Verwendung des oberflächenaktiven Mittels gemäß Anspruch 6, wobei das oberflächenaktive Mittel mindestens ein nichtionisches oberflächenaktives Mittel ist, ausgewählt aus der Gruppe bestehend aus Oligomeren von Alkylphenolethoxylaten, Fettsäureestern von Sorbitol, Fettsäureestern von Glycerol und ethoxyliertem Vitamin E.

9. Die Verwendung des oberflächenaktiven Mittels gemäß einem der Ansprüche 1 bis 8, wobei die Suspension von Lipidnanopartikeln oder Lipidoidnanopartikeln in einer wässrigen Trägerlösung das oberflächenaktive Mittel in einer Konzentration von 0,01 bis 10 % (w/v) umfasst.

10. Die Verwendung des oberflächenaktiven Mittels gemäß einem der Ansprüche 1 bis 8, wobei die Nukleinsäure mRNA ist.

11. Die Verwendung des oberflächenaktiven Mittels gemäß einem der Ansprüche 1 bis 10, wobei die Nanopartikel kein Poloxamer 188 und/oder kein Poloxamer 407 umfassen.

12. Die Verwendung des oberflächenaktiven Mittels gemäß einem der Ansprüche 1 bis 11, wobei die Nanopartikel ein ionisierbares Lipidoid (b) der folgenden Formel (b-1) umfassen: 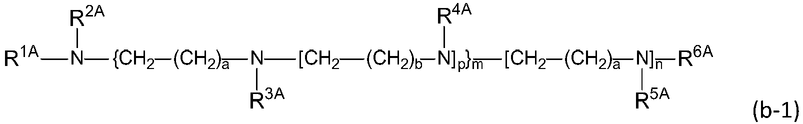 wobei:
a 1 ist und b eine ganze Zahl von 2 bis 4 ist; oder a eine ganze Zahl von 2 bis 4 ist und b 1 ist,
p 1 oder 2 ist,
m 1 oder 2 ist; n 0 oder 1 ist und m + n ≥ 2 ist; und
R^{1A} bis R^{6A} unabhängig voneinander ausgewählt sind aus: Wasserstoff; -CH₂-CH(OH)-R^{7A}, -CH(R^{7A})-CH₂-OH, -CH₂-CH₂-(C=O)-O-R^{7A},
-CH₂-CH₂-(C=O)-NH-R^{7A}; -CH₂-R^{7A}; -C(NH)-NH₂; einer Poly(ethylenglykol)-Kette;
und einem Rezeptorliganden; wobei R^{7A} ausgewählt ist aus C3-C18-Alkyl und C3-C18-Alkenyl mit einer C-C-Doppelbindung;
mit der Maßgabe, dass mindestens zwei Reste R^{1A} bis R^{6A} ausgewählt sind aus -CH₂-CH(OH)-R^{7A}, -CH(R^{7A})-CH₂-OH, -CH₂-CH₂ -(C=O)-O-R^{7A},
-CH₂-CH₂-(C=O)-NH-R^{7A} und -CH₂-R^{7A}, wobei R^{7A} ausgewählt ist aus C3-C18-Alkyl oder C3-C18-Alkenyl mit einer C-C-Doppelbindung;
oder eine protonierte Form davon, wobei eines oder mehrere der in der Verbindung der Formel (b-1) enthaltenen Stickstoffatome protoniert ist (sind), um eine positiv geladene Verbindung bereitzustellen.

13. Verwendung des oberflächenaktiven Mittels gemäß einem der Ansprüche 1 bis 12, wobei die Nanopartikel als ionisierbares Lipid (b) umfassen:
(6Z,9Z,28Z,31Z)-Heptatriaconta-6,9,28,31-tetraen-19-yl-4-(dimethylamino)butanoat oder eine protonierte Form davon, wobei das Stickstoffatom der Verbindung protoniert ist,
((4-Hydroxybutyl)azandiyl)bis(hexan-6,1-diyl)bis(2-hexyldecanoat) oder eine protonierte Form davon, wobei das Stickstoffatom der Verbindung protoniert ist, und/oder
(Heptadecan-9-yl-8-((2-hydroxyethyl)(6-oxo-6-(undecyloxy)hexyl)amino)octanoat( oder eine protonierte Form davon, wobei das Stickstoffatom der Verbindung protoniert ist.

14. Die Verwendung des oberflächenaktiven Mittels gemäß einem der Ansprüche 1 bis 13, wobei die Z-mittlere Partikelgröße der suspendierten Partikel, bestimmt durch dynamische Lichtstreuung bei 25 °C, unter der physikalischen Belastungsbedingung um weniger als 50 % zunimmt, vorzugsweise um weniger als 20 %, noch stärker bevorzugt um weniger als 10 %, und am stärksten bevorzugt keine Zunahme auftritt, und/oder der Polydispersitätsindex der suspendierten Nanopartikel, bestimmt durch dynamische Lichtstreuung bei 25 °C, nach Einwirkung der physikalischen Belastungsbedingung auf die Suspension im Vergleich zum Polydispersitätsindex der Suspension vor Einwirken der physikalischen Belastungsbedingung erhalten bleibt.

15. Eine Suspension von Lipidnanopartikeln oder von Lipidoidnanopartikeln in einer wässrigen Trägerlösung, wobei die wässrige Trägerlösung ein oberflächenaktives Mittel umfasst und wobei die Lipidnanopartikel oder Lipidoidnanopartikel die folgenden Komponenten (a) und (b) umfassen:
(a) eine Nukleinsäure und
(b) mindestens eines, ausgewählt aus einem permanent kationischen Lipid, einem ionisierbaren Lipid und einem ionisierbaren Lipidoid,
und wobei das oberflächenaktive Mittel im Wesentlichen nicht an die Nanopartikel gebunden ist.

16. Ein Verfahren zur Stabilisierung einer Suspension von Lipidnanopartikeln oder Lipidoidnanopartikeln in einer wässrigen Trägerlösung gegen Partikelaggregation unter einer physikalischen Belastungsbedingung, ausgewählt aus Schütteln, Rühren, Vibrieren, Mischen, Invertieren, Klopfen oder Fallenlassen der Suspension oder einer Kombination davon, oder wobei die physikalische Belastungsbedingung durch Pumpen der Suspension, durch ihr Aufziehen in eine Spritze oder durch ihren Transport verursacht wird, wobei die Lipidnanopartikel (LNP) oder Lipidoidnanopartikel (LiNP) die folgenden Komponenten (a) und (b) umfassen:
(a) eine Nukleinsäure und
(b) mindestens eines, ausgewählt aus einem permanent kationischen Lipid, einem ionisierbaren Lipid und einem ionisierbaren Lipidoid,
und wobei das Verfahren das Einbringen eines oberflächenaktiven Mittels in die wässrige Trägerlösung der Suspension von Lipidnanopartikeln oder Lipidoidnanopartikeln umfasst.

17. Das Verfahren gemäß Anspruch 16, wobei das Verfahren die folgenden Schritte umfasst:
i) Herstellen einer Zubereitung von Lipidnanopartikeln (LNP) oder Lipidoidnanopartikeln (LiNP) durch Mischen mindestens eines, ausgewählt aus einem permanent kationischen Lipid, einem ionisierbaren Lipid und einem ionisierbaren Lipidoid, gelöst in einer organischen Phase, mit einer in einer wässrigen Lösung gelösten Nukleinsäure,
ii) Verdünnen der Zubereitung aus Lipidnanopartikeln oder Lipidoidnanopartikeln durch Verdünnen mit einer ersten Lösung,
iii) Konzentrieren der verdünnten Zubereitung aus Lipid-Nanopartikeln oder LipidoidNanopartikeln durch Pufferaustausch mittels Ultra-/Diafiltration durch TFF, wobei eine zweite Lösung für die Ultra-/Diafiltration verwendet wird,
iv) Erhalten einer Suspension von Lipidnanopartikeln oder Lipidoidnanopartikeln;
wobei die erste Lösung zwischen 0,01 % w/v und 10 % oberflächenaktives Mittel, vorzugsweise zwischen 0,25 % w/v oberflächenaktives Mittel und 5 % w/v oberflächenaktives Mittel, stärker bevorzugt zwischen 0,33 % w/v oberflächenaktives Mittel und 2,5 % w/v oberflächenaktives Mittel, stärker bevorzugt zwischen 0,45 % w/v und 1,5 % w/v oberflächenaktives Mittel, am stärksten bevorzugt zwischen 0,5 % w/v und 1,5 % w/v oberflächenaktives Mittel, umfasst, und/oder
wobei die zweite Lösung zwischen 0,01 % w/v und 10 % oberflächenaktives Mittel, vorzugsweise zwischen 0,25 % w/v oberflächenaktives Mittel und 5 % w/v oberflächenaktives Mittel, stärker bevorzugt zwischen 0,33 % w/v oberflächenaktives Mittel und 2,5 % w/v oberflächenaktives Mittel, stärker bevorzugt zwischen 0,45 % w/v und 1,5 % w/v oberflächenaktives Mittel, am stärksten bevorzugt zwischen 0,5 % w/v und 1,5 % w/v oberflächenaktives Mittel, umfasst,
und wobei die Endkonzentration an oberflächenaktivem Mittel aus der kombinierten ersten und zweiten Lösung zwischen 0,01 % und 10 % oberflächenaktivem Mittel, vorzugsweise zwischen 0,25 % w/v oberflächenaktivem Mittel und 5 % w/v oberflächenaktivem Mittel, stärker bevorzugt zwischen 0,33 % w/v oberflächenaktivem Mittel und 2,5 % w/v oberflächenaktivem Mittel, stärker bevorzugt zwischen 0,45 % w/v und 1,5 % w/v oberflächenaktivem Mittel, am stärksten bevorzugt zwischen 0,5 % w/v und 1,5 % w/v oberflächenaktivem Mittel, bezogen auf das Gesamtvolumen der Suspension der Nanopartikel in der wässrigen Trägerlösung beträgt.

18. Ein Verfahren zur Verringerung einer oder mehrerer Nebenwirkungen, die mit einer Impfstoffformulierung oder einer Antikrebsformulierung umfassend LNPs oder LiNPs, welche eine Nukleinsäure tragen, in Zusammenhang stehen, wobei das Verfahren die Modifizierung der Impfstoffformulierung oder der Antikrebsformulierung durch Zugabe eines oberflächenaktiven Mittels zu einer Impfstoffformulierung umfassend eine LNP- oder LiNP-Suspension, umfasst.

## Revendications

1. Utilisation d'un tensioactif pour stabiliser une suspension de nanoparticules de lipide ou de nanoparticules de lipoïde dans une solution aqueuse de véhicule vis-à-vis d'une agrégation de particules dans une condition de contrainte physique choisie parmi un secouage, une agitation, une vibration, un mélange, un retournement, un tapotement, et une chute de la suspension, ou une combinaison de ceux-ci, ou dans laquelle la condition de contrainte physique est provoquée par un pompage de la suspension, par son aspiration dans une seringue, ou par son transport, dans laquelle les nanoparticules de lipide ou les nanoparticules de lipoïde comprennent les composants (a) et (b) suivants :
(a) un acide nucléique et
(b) au moins l'un choisi parmi un lipide cationique à charge permanente, un lipide ionisable et un lipoïde ionisable,
et dans laquelle le tensioactif est incorporé à titre d'excipient dans la solution aqueuse de véhicule.

2. Utilisation d'un tensioactif selon la revendication 1, dans laquelle la condition de contrainte physique est choisie parmi un retournement, un tapotement, et une chute de la suspension ou dans laquelle la condition de contrainte physique est provoquée par un pompage de la suspension ou par son aspiration dans une seringue.

3. Utilisation d'un tensioactif selon la revendication 1 ou 2, dans laquelle le tensioactif n'est pratiquement pas attaché aux nanoparticules.

4. Utilisation d'un tensioactif selon l'une quelconque des revendications 1 à 3, dans laquelle le tensioactif n'est pas présent dans la solution de véhicule pendant un procédé de lyophilisation.

5. Utilisation d'un tensioactif selon l'une quelconque des revendications 1 à 4, dans laquelle le tensioactif est un tensioactif non-ionique.

6. Utilisation d'un tensioactif selon la revendication 5, dans laquelle le tensioactif est au moins un tensioactif non-ionique choisi dans le groupe constitué par les alcools gras éthoxylés, les acides gras éthoxylés, les copolymères séquencés d'oxyde d'éthylène et d'oxyde de propylène, les alkylphénols éthoxylés ou les oligomères d'alkylphénols éthoxylés, les esters d'acide gras et de sorbitol, les esters d'acide gras et de sorbitol éthoxylés, les esters d'acide gras et de glycérol, l'huile de ricin éthoxylée et la vitamine E éthoxylée.

7. Utilisation d'un tensioactif selon la revendication 6, dans laquelle le tensioactif est un copolymère séquencé d'oxyde d'éthylène et d'oxyde de propylène, de préférence un poloxamère choisi parmi le poloxamère 124, le poloxamère 188, le poloxamère 338 et le poloxamère 407.

8. Utilisation d'un tensioactif selon la revendication 6, dans laquelle le tensioactif est au moins un tensioactif non-ionique choisi dans le groupe constitué par les oligomères d'alkylphénols éthoxylés, les esters d'acide gras et de sorbitol, les esters d'acide gras et de glycérol, et la vitamine E éthoxylée.

9. Utilisation d'un tensioactif selon l'une quelconque des revendications 1 à 8, dans laquelle la suspension de nanoparticules de lipide ou de nanoparticules de lipoïde dans une solution aqueuse de véhicule comprend le tensioactif à une concentration de 0,01 à 10 % (en poids/volume).

10. Utilisation d'un tensioactif selon l'une quelconque des revendications 1 à 8, dans laquelle l'acide nucléique est un ARNm.

11. Utilisation d'un tensioactif selon l'une quelconque des revendications 1 à 10, dans laquelle les nanoparticules ne comprennent pas de poloxamère 188 et/ou ne comprennent pas de poloxamère 407.

12. Utilisation d'un tensioactif selon l'une quelconque des revendications 1 à 11, dans laquelle les nanoparticules comprennent un lipoïde ionisable (b) de formule (b-1) suivante, 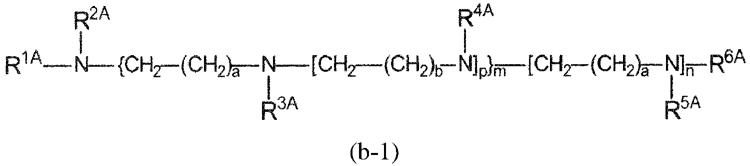 dans laquelle :
a vaut 1 et b est un entier de 2 à 4 ; ou bien a est un entier de 2 à 4 et b vaut 1,
p vaut 1 ou 2,
m vaut 1 ou 2 ; n vaut 0 ou 1 et m+n est ≥ 2 ; et
R^{1A} à R^{6A}, indépendamment les uns des autres, sont choisis parmi : l'hydrogène ; -CH₂-CH(OH)-R^{7A}, -CH(R^{7A})-CH₂-OH, -CH₂-CH₂-(C=O)-O-R^{7A}, -CH₂-CH₂-(C=O)-NH-R^{7A} ; -CH₂-R^{7A} ; -C(NH)-NH₂ ; une chaîne poly(éthylèneglycol) ; et un ligand de récepteur ; où R^{7A} est choisi parmi alkyle en C3 à C18 et alcényle en C3 à C18 ayant une double liaison C-C ;
sous réserve qu'au moins deux résidus parmi R^{1A} à R^{6A} soient choisis parmi -CH₂-CH(OH)-R^{7A}, -CH(R^{7A})-CH₂-OH, -CH₂-CH₂-(C=O)-O-R^{7A}, -CH₂-CH₂-(C=O)-NH-R^{7A} et -CH₂-R^{7A}, où R^{7A} est choisi parmi alkyle en C3 à C18 et alcényle en C3 à C18 ayant une double liaison C-C ;
ou une forme protonée de celui-ci, dans laquelle un ou plusieurs des atomes d'azote contenus dans le composé de formule (b-1) sont protonés pour donner un composé portant une charge positive.

13. Utilisation d'un tensioactif selon l'une quelconque des revendications 1 à 12, dans laquelle les nanoparticules comprennent, à titre de lipide ionisable (b) :
du 4-(diméthylamino)butanoate de (6Z,9Z,28Z,31Z)-heptatriaconta-6,9,28,31-tétraén-19-yle ou une forme protonée de celui-ci dans laquelle l'atome d'azote du composé est protoné,
du bis(2-hexyldécanoate) de ((4-hydroxybutyl)azanediyl)bis(hexane-6,1-diyle) ou une forme protonée de celui-ci dans laquelle l'atome d'azote du composé est protoné, et/ou
du 8-((2-hydroxyéthyl)(6-oxo-6-(undécyloxy)hexyl)amino)octanoate d'heptadécan-9-yle ou une forme protonée de celui-ci dans laquelle l'atome d'azote du composé est protoné.

14. Utilisation d'un tensioactif selon l'une quelconque des revendications 1 à 13, dans laquelle la granulométrie moyenne Z des particules en suspension, telle que déterminée par diffusion dynamique de la lumière à 25 °C, augmente de moins de 50 % dans la condition de contrainte physique, de préférence de moins de 20 %, mieux encore de moins de 10 %, et de manière plus préférée il n'y a pas d'augmentation, et/ou l'indice de polydispersité des nanoparticules en suspension, tel que déterminé par diffusion dynamique de la lumière à 25 °C, est maintenu après exposition de la suspension à la condition de contrainte physique, en comparaison avec l'indice de polydispersité de la suspension avant exposition à la condition de contrainte physique.

15. Suspension de nanoparticules de lipide ou de nanoparticules de lipoïde dans une solution aqueuse de véhicule, dans laquelle la solution aqueuse de véhicule comprend un tensioactif, et dans laquelle les nanoparticules de lipide ou les nanoparticules de lipoïde comprennent les composants (a) et (b) suivants :
(a) un acide nucléique et
(b) au moins l'un choisi parmi un lipide cationique à charge permanente, un lipide ionisable et un lipoïde ionisable,
et dans laquelle le tensioactif n'est pratiquement pas attaché aux nanoparticules.

16. Méthode pour stabiliser une suspension de nanoparticules de lipide ou de nanoparticules de lipoïde dans une solution aqueuse de véhicule vis-à-vis d'une agrégation de particules dans une condition de contrainte physique choisie parmi un secouage, une agitation, une vibration, un mélange, un retournement, un tapotement, et une chute de la suspension, ou une combinaison de ceux-ci, ou dans laquelle la condition de contrainte physique est provoquée par un pompage de la suspension, par son aspiration dans une seringue, ou par son transport, dans laquelle les nanoparticules de lipide (LNP) ou les nanoparticules de lipoïde (LiNP) comprennent les composants (a) et (b) suivants :
(a) un acide nucléique et
(b) au moins l'un choisi parmi un lipide cationique à charge permanente, un lipide ionisable et un lipoïde ionisable,
et laquelle méthode comprend l'incorporation d'un tensioactif dans la solution aqueuse de véhicule de la suspension de nanoparticules de lipide ou de nanoparticules de lipoïde.

17. Méthode selon la revendication 16, laquelle méthode comprend les étapes suivantes :
i) génération d'une préparation de nanoparticules de lipide (LNP) ou de nanoparticules de lipoïde (LiNP) par mélange d'au moins l'un choisi parmi un lipide cationique à charge permanente, un lipide ionisable et un lipoïde ionisable, dissous dans une phase organique avec un acide nucléique dissous dans une solution aqueuse,
ii) dilution de la préparation de nanoparticules de lipide ou de nanoparticules de lipoïde par dilution avec une première solution,
iii) concentration de la préparation diluée de nanoparticules de lipide ou de nanoparticules de lipoïde par échange de tampon au moyen d'une ultra/dia-filtration par TFF, dans laquelle une deuxième solution est utilisée pour l'ultra/la dia-filtration,
iv) obtention d'une suspension de nanoparticules de lipide ou de nanoparticules de lipoïde ;
dans laquelle la première solution comprend entre 0,01 % et 10 % en poids/volume de tensioactif, de préférence entre 0,25 % en poids/volume de tensioactif et 5 % en poids/volume de tensioactif, mieux encore entre 0,33 % en poids/volume de tensioactif et 2,5 % en poids/volume de tensioactif, plus particulièrement entre 0,45 % en poids/volume et 1,5 % en poids/volume de tensioactif, tout spécialement entre 0,5 % en poids/volume et 1,5 % en poids/volume de tensioactif, et/ou
dans laquelle la deuxième solution comprend entre 0,01 % et 10 % en poids/volume de tensioactif, de préférence entre 0,25 % en poids/volume de tensioactif et 5 % en poids/volume de tensioactif, mieux encore entre 0,33 % en poids/volume de tensioactif et 2,5 % en poids/volume de tensioactif, plus particulièrement entre 0,45 % en poids/volume et 1,5 % en poids/volume de tensioactif, tout spécialement entre 0,5 % en poids/volume et 1,5 % en poids/volume de tensioactif,
et dans laquelle la concentration finale de tensioactif provenant des première et deuxième solutions combinées est comprise entre 0,01 % et 10 % de tensioactif, de préférence entre 0,25 % en poids/volume de tensioactif et 5 % en poids/volume de tensioactif, mieux encore entre 0,33 % en poids/volume de tensioactif et 2,5 % en poids/volume de tensioactif, plus particulièrement entre 0,45 % en poids/volume et 1,5 % en poids/volume de tensioactif, tout spécialement entre 0,5 % en poids/volume et 1,5 % en poids/volume de tensioactif, par rapport au volume total de la suspension des nanoparticules dans la solution aqueuse de véhicule.

18. Méthode de réduction d'un ou plusieurs effets secondaires associés à une formulation de vaccin ou à une formulation anticancéreuse comprenant des LNP ou des LiNP portant un acide nucléique, la méthode comprenant la modification de la formulation de vaccin ou de la formulation anticancéreuse par addition d'un tensioactif à une formulation de vaccin comprenant une suspension de LNP ou de LiNP.
